# EUROPEAN PATENT SPECIFICATION

(11) **EP 2 164 965 B1**
(45) Date of publication and mention of the grant of the patent: **11.09.2013**
(21) Application number: 08763141.2
(22) Date of filing: 29.05.2008
(51) Int. Cl.: C12N 15/113, A61K 31/713

(54) **A PRIMARY MICRO RNA EXPRESSION CASSETTE**
PRIMÄRE miRNA-EXPRESSIONSKASSETTE
CASSETTE D'EXPRESSION DE MICRO ARN PRIMAIRE

(30) Priority: 29.05.2007 ZA 200704435
(43) Date of publication of application: 24.03.2010
(73) Proprietor: University Of The Witwatersrand, Johannesburg, 2050 Johannesburg (ZA)
(72) Inventor: PATRICK, Arbuthnot, 2094 Kensington (ZA); ELY, Abdullah, 2094 Bezuidenhouts Valley (ZA); NAIDOO, Tanusha, Westgate 1734 (ZA); WEINBERG, Marc Saul, 1685 Midrand (ZA); LONGSHAW, Victoria Mary, 7441 Cape Town (ZA)
(74) Representative: Read, David Graham
(86) International application number: PCT/IB2008/052103
(87) International publication number: WO 2008/146251

(56) References cited:
- WO-A-2007/053184
- CARMONA SERGIO ET AL: "Effective inhibition of HBV replication in vivo by anti-HBx short hairpin RNAs." MOLECULAR THERAPY : THE JOURNAL OF THE AMERICAN SOCIETY OF GENE THERAPY FEB 2006, vol. 13, no. 2, February 2006 (2006-02), pages 411-421, XP002515738 ISSN: 1525-0016
- KANDA TATSUO ET AL: "Small interfering RNA targeted to hepatitis C virus 5' nontranslated region exerts potent antiviral effect." JOURNAL OF VIROLOGY JAN 2007, vol. 81, no. 2, January 2007 (2007-01), pages 669-676, XP002515739 ISSN: 0022-538X
- SILVA J M ET AL: "Second-generation shRNA libraries covering the mouse and human genomes" NATURE GENETICS, NATURE PUBLISHING GROUP, NEW YORK, US, vol. 37, no. 11, 2 October 2005 (2005-10-02), pages 1281-1288, XP002399751 ISSN: 1061-4036
- ZENG Y ET AL: "USE OF RNA POLYMERASE II TO TRANSCRIBE ARTIFICIAL MICRORNAS" METHODS IN ENZYMOLOGY, ACADEMIC PRESS INC, SAN DIEGO, CA, US, vol. 392, 1 January 2005 (2005-01-01), pages 371-380, XP009082667 ISSN: 0076-6879
- CHEN ET AL: "Inhibition of avian leukosis virus replication by vector-based RNA interference" VIROLOGY, ACADEMIC PRESS,ORLANDO, US, vol. 365, no. 2, 9 May 2007 (2007-05-09), pages 464-472, XP022196436 ISSN: 0042-6822
- CHANG KENNETH ET AL: "LESSONS FROM NATURE: MICRORNA-BASED SHRNA LIBRARIES" NATURE METHODS, NATURE PUBLISHING GROUP, GB, vol. 3, no. 9, 1 September 2006 (2006-09-01), pages 707-714, XP009082232 ISSN: 1548-7091
- STEGMEIER FRANK ET AL: "A lentiviral microRNA-based system for single-copy polymerase II-regulated RNA interference in mammalian cells" PROCEEDINGS OF THE NATIONAL ACADEMY OF SCIENCES OF USA, NATIONAL ACADEMY OF SCIENCE, WASHINGTON, DC.; US, vol. 102, no. 37, 1 September 2005 (2005-09-01), pages 13212-13217, XP002482035 ISSN: 0027-8424
- CHUNG KWAN-HO ET AL: "Polycistronic RNA polymerase II expression vectors for RNA interference based on BIC/miR-155" NUCLEIC ACIDS RESEARCH, OXFORD UNIVERSITY PRESS, SURREY, GB, vol. 34, no. 7, 13 April 2006 (2006-04-13), pages e53-1, XP002456694 ISSN: 0305-1048
- ZENG Y ET AL: "Sequence requirements for micro RNA processing and function in human cells" RNA, COLD SPRING HARBOR LABORATORY PRESS, WOODBURY, NY, US, vol. 9, no. 1, 1 January 2003 (2003-01-01), pages 112-123, XP003014844 ISSN: 1355-8382

## Description

### BACKGROUND OF THE INVENTION

THIS INVENTION relates to inhibition of viral gene expression. More specifically, this invention relates to a method of using RNA sequences to inhibit Hepatitis B Virus replication. Expression constructs containing sequences derived from endogenous micro RNAs (miRs) are used in the method to generate silencing sequences that are specific to HBV sequences.

RNA interference (RNAi) is an evolutionary conserved biological response to double-stranded RNA that has been described in plants [1], invertebrates [2-4] and in mammalian cells [5]. RNAi functions by directing the suppression of genes expressing homologous sequences to either endogenous or introduced RNAi effectors [6] with no effect on genes with unrelated sequences [7, 8]. RNAi is thought to be an ancient response pathway that plays a role in regulating the expression of protein-coding genes [8] and mediates resistance to both endogenous parasitic and exogenous pathogenic nucleic acids. Naturally occurring small RNAs that activate RNAi are part of a complex network of micro RNAs (miRs), which are processed by Drosha and Dicer before effecting silencing of gene expression. These miRs have their effect by regulating translation of specific cellular mRNAs [9]. Processing of 'rogue' double stranded viral and cellular RNA by Dicer may also lead to formation of short interfering RNAs (siRNAs) [10-12]. These siRNAs are typically 21-23 bp with 2 nucleotide 3' overhangs [13] and activate RNAi to effect silencing of the potentially harmful RNA. m iR sequences are initially transcribed as primary miRs (pri-miRs) from cellular DNA sequences using Pol II transcription regulatory sequences and RNA Polymerase II. Processing of pri-miRs by Drosha results in the formation of precursor miRs (pre-miRs) which in turn are cleaved by intracellular Dicer before the mature miRs (approximately 22 nt in length) induce silencing of gene expression. Drosha and Dicer are both RNase III enzymes that are essential for normal functioning of the RNAi pathway [14]. The post-transcriptional silencing action of RNAi has been reported to be more efficient than either ribozyme or antisense RNA action [15]. To cause gene silencing, miRs or one strand of an siRNA is incorporated into an RNA induced silencing complex (RISC). RISC includes Ago2 (an RNA endonuclease) and a helicase [16] amongst other subunits [17, 18]. Using the miR or antisense strand of siRNA as a hybridising guide sequence, RISC identifies the target mRNA [10, 19]. Generally, if the guide sequence is perfectly complementary to its cognate, then cleavage of the target occurs. If however there are mismatches in the hybrid within RISC, then translational suppression occurs. Gene silencing by siRNA-mediated methylation of promoter DNA sequences has also been shown to reduce gene transcription in mammalian cells [20].

Effecting RNAi in mammalian cells has, until recently, been a difficult undertaking. Double-stranded RNAs which are longer than 30 base-pairs trigger the non-specific interferon response pathway, which is mediated by the activation of dsRNA-dependent protein kinase (PKR) [21] and 2',5'-oligoadenylate synthetase (2'5'OAS) [22]. This response pathway results in global repression of translation and leads ultimately to apoptosis [23]. To induce specific and significant gene silencing, intracellular delivery or production of siRNA or short hairpin RNA (shRNA) fragments of exact size is important. By introducing SiRNAs as short synthetic annealed oligonucleotides (<30 bp) directly into mammalian cells, Tuschl and colleagues were successfully able to bypass the interferon pathway and effect RNAi in mammalian cell cultures [15].

Many of the studies undertaken to achieve gene silencing have used presynthesized RNAs. Typically, complementary RNA oligonucleotides are annealed *in vitro* to generate an exogenous source of siRNA for delivery into cells. Since synthetic oligoribonucleotides are not replenished naturally within a cell, to maintain an adequate intracellular concentration for sustained activity, these molecules need to be administered regularly. Synthetic oligoribonucleotides may be chemically altered to preserve their longevity in physiological fluids. However, these modifications may have adverse toxic effects in vivo [24]. Results from a number of studies suggest that siRNAs can be expressed endogenously as independent sense and antisense RNA strands [25, 26], as shRNAs [27- 31] or as derivatives of naturally-occurring miRs [32, 33]. Transcription of miR genes naturally produces pri-miR sequences, which are processed in the nucleus by the enzyme Drosha to form pre- miR. Pr-miR is then transported to the cytoplasm via the exportin 5 pathway, where it is processed by Dicer to form mature miR. Since little is known about the promoters involved in miR expression, most studies have used the U6 small nuclear RNA [34] promoter [27] or more compact H1 promoter [8] or tRNA^{Val} promoter [35]. These promoters are recognised by RNA Polymerase III, and are capable of constitutively producing effecters of RNAi. Pol III promoters have the advantage of containing all of their control elements upstream of the transcription initiation site, and this enables the generation of expression cassettes that produce transcripts of defined length. However, the constitutively active nature of Pol III promoter transcription may lead to saturation of the normal cellular RNAi pathway and resultant toxicity [36]. Pol II promoters can induce tissue- or cell-type- specific RNA expression but have the disadvantage of requiring control elements downstream of the transcription initiation site. Thus in addition to potentially therapeutic RNA, additional sequences derived from regulatory elements are included in the transcript. Previous studies have shown that these additional sequences inhibit the function of traditional shRNA molecules [37]. In fact, the silencing effect of transcribed shRNAs, or individual sense and antisense siRNA strands, is compromised by the presence of as few as 9 extra bases at the 5' end, between the transcription start site and the 21 base pair hairpin [37].

Carmona Sergio et al: Molecular Therapy: The Journal of the American Society of Gene Therapy Feb 2006, vol. 13, no. 2, pages 411-421, reported that anti-subgenotype A1 short hairpins RNAs targeted to HBx are powerful and specific inhibitors of HBV replication.

Kanda Tatsuo et al: Journal of Virology Jan 2007, vol. 81, no. 2, pages 669-676, reported on vector encoding short hairpin RNA targeting 5' nontranslated conserved region of HCV genome for inhibition of virus replication.

There is at present no means of conveniently generating functional RNAi effectors from Pol II transcripts. Chemical RNA synthesis, *in vitro* transcription and use of Pol III-based cassettes are currently the preferred methods of generating short RNA sequences of precise length.

Terms used herein have their art-recognised meaning unless otherwise indicated. According to their use here, the following terms have meanings defined below.

### Transcription

The process of producing RNA from a DNA template.

### Nucleic acid

The term "nucleic acid" refers to deoxyribonucleotide or ribonucleotide polymer in either single- or double-stranded form, and unless otherwise limited, encompasses analogues of natural nucleotides that hybridise to nucleic acids in a manner similar to naturally occurring nucleotides. Unless otherwise indicated, a particular nucleic acid sequence includes the complementary sequence thereof.

### Expression cassette

A "recombinant expression cassette" or simply "expression cassette" is a nucleic acid construct, generated recombinantly or synthetically, with nucleic acid elements which permit transcription of a particular nucleic acid in the cell. The recombinant expression cassette can be part of a plasmid, virus or nucleic acid fragment. Typically, the recombinant expression cassette includes a nucleic acid to be transcribed, and an operably linked promoter. In some embodiments, the expression cassette may also include an origin of replication and/or chromosome integration elements (e. g. a retroviral LTR).

### Operably linked

The term "operably linked" refers to a functional linkage between a nucleic acid expression control sequence (such as a promoter, enhancer or array of transcription factor binding sites) and a second nucleic acid sequence, wherein the expression control sequence directs transcription of the nucleic acid corresponding to the second sequence.

### Promoter

A promoter is an array of DNA control sequences which is involved in binding of an RNA polymerase to initiate transcription of a nucleic acid. As used herein, a promoter includes necessary nucleic acid sequences near the start site of transcription. A promoter also optionally includes distal enhancer or repressor elements which can be located as much as several thousand base pairs away from the start site of transcription.

### Pol II promoter

A Pol II promoter is a DNA sequence that includes elements that are recognised by RNA Polymerase II to enable initiation of transcription by this enzyme. A Pol II promoter typically includes characteristic elements such as a TATA box.

### Pol III promoter

A Pol III promoter is a DNA sequence that includes elements that are recognised by RNA Polymerase III to enable initiation of transcription by this enzyme.

### Transcription termination signal

A transcription termination signal is a DNA sequence that terminates transcription. These elements are different for RNA Polymerase II and RNA Polymerase III enzymes.

### RNA interference

The process by which the expression of a double stranded nucleic acid (including miR, siRNA, shRNA) causes sequence-specific degradation of complementary RNA, sequence-specific translational suppression or transcriptional gene silencing.

### Target recognition sequence

As used herein, the term 'target recognition sequence' refers to a sequence derived from a gene, in respect of which gene the invention is designed to inhibit, block or prevent gene expression, enzymatic activity or interaction with other cellular or viral factors.

### Guide Sequence

A short single stranded RNA fragment derived from an RNAi effecter, for example miR, siRNA, shRNA that is incorporated into RISC, and which is responsible for sequence-specific degradation or translation suppression of target RNA at a target recognition sequence.

### RNAi effecter

Any RNA sequence (e. g. shRNA, miR and siRNA) including its precursors, which can cause RNAi.

### RNAi effecter processing unit

RNA that includes sequences of an RNA effecter together with processing units (e. g. hammerhead ribozyme).

### RNAi effecter processing cassette

An RNAi effecter processing unit with operably linked promoter.

### RNAi precursor

Any RNA species that is processed to form a guide sequence, which may then be incorporated into RISC and effect RNAi.

### Dicer

An RNAse III enzyme, which digests double stranded RNA and is responsible for maturation of RNAi precursors. For example, Dicer is responsible for acting on pre-miRs to form mature miR.

### Drosha

Drosha is an RNase III enzyme that forms part of the nuclear microprocessor complex that recognises specific pri-miR secondary structures to cleave and release pre-miR sequences of approximately 60-80 nt.

### RNAi-encoding sequence

A nucleic acid sequence which, when expressed, causes RNA interference.

### Pri-miR

Pri-miR is a primary miR transcript that is typically derived from Pol II transcription. These sequences may originate from an independent miR transcription unit, a transcript that includes more than one miR precursor or an intronic miR precursor. These sequences are usually processed by Drosha within the cell nucleus to generate pre-miR.

### Pre-miR

Pre-miR is the product of pri-miR processing by Drosha. Generally pre-miRs are 60-80 nt in length. Pre-miRs are exported from the cell nucleus by exportin-5. In the cytoplasm, pre-miRs are processed by Dicer to form mature miR.

### miR

miRs are small RNA molecules of approximately 22 nt in length that are derived from processing of pri-miR and then pre-miR sequences.

### miR expression cassette

A miR expression cassette refers to a DNA sequence which encodes RNA that simulates endogenous miR. A pri-miR transcript is generated from the cassette and is processed by cellular mechanisms to generate pre-miR and mature miR.

### Pri-miR expression cassette

A nucleic acid construct that encodes a pri-miR sequence.

### shRNA

Short hairpin RNA (shRNA) is a short sequence of single stranded RNA which folds back on itself such that nucleotides from the two separate segments have base paired, and the resulting structure appears as the name describes. shRNA is a substrate for Dicer and effects RNAi (the double stranded region of the hairpin may include base mismatches i. e. non AU or GC pairs).

### siRNA

Small interfering RNA (siRNA) consists of a short double-stranded RNA molecule. Typically a siRNA molecule comprises a 19 bp duplex region with 3' overhangs of 2 nt. One strand is incorporated into a cytoplasmic RNA-induced silencing complex (RISC). This directs the sequence specific RNA cleavage that is effected by RISC. Mismatches between the siRNA guide and its target may cause translational suppression instead of RNA cleavage. siRNA may be synthetic or derived from processing of a precursor by Dicer.

### shRNA precursor

A shRNA precursor is a hairpin RNA sequence that is processed intracellularly by Dicer to generate a shRNA molecule.

### Multimeric cassette

A tandem arrangement of monomeric units, which may include miR-encoding sequences.

### In silico

*In silico* refers to the laboratory conditions under which a reaction is carried out in a test tube (or equivalent vessel) and when no living cells are present.

### Monomeric unit

A nucleic-acid sequence that encodes components of one RNAi effecter sequence.

### Subsequence

The term "subsequence" in the context of a particular nucleic acid sequence refers to a region of the nucleic acid equal to or smaller than the specified nucleic acid, or a part thereof.

### In vitro transcription

The transcription of a DNA molecule into RNA molecules using a laboratory medium which contains an RNA polymerase and RNA precursors.

### Intracellular transcription

The transcription of a DNA molecule into RNA molecules, within a living cell.

### In vivo transcription

The transcription of a DNA molecule into RNA molecules, within a living organism.

### Hybridisation

Nucleic acids are claimed that specifically hybridize to the nucleic acids herein disclosed under sufficient stringency conditions. Specific or selective hybridization is that hybridization wherein the nucleic acid binds the target nucleic acid with minimal background, nonspecific hybridization to non-target nucleic acids. Typically, the stringency of hybridization to achieve selective hybridization is about 5°C to 20°C below the Tm (the melting temperature at which half of the molecules dissociate from its partner), but it is further defined by the salt concentration and the permitivity of the solution. Hybridization temperatures are typically higher for DNA-RNA and RNA-RNA hybridizations. The washing temperatures can similarly be used to achieve selective stringency, as is known in the art (Sambrook et al., 1987).

### SUMMARY OF THE INVENTION

This invention describes a universally applicable method, which incorporates features of naturally occurring miRs into expression cassettes, to allow generation of an RNAi effecter from a Pol II promoter.

According to the invention there is provided an anti-hepatitis virus primary micro RNA (pri-miR) expression cassette, including:
(i) a DNA sequence encoding an artificial pri-miR-31 or pri-miR-122 sequence which mimics a naturally occurring pri-miR-31 or pri-miR-122 sequence, wherein the artificial pri-miR-31 or pri-miR-122 sequence differs from the naturally occurring pri-miR-31 or pri-miR-122 sequence in that the guide sequence of the naturally occurring pri-miR-31 or pri-miR-122 has been replaced with a guide sequence that targets hepatitis virus, and wherein the sequence of the artificial pri-miR-31 or pri-miR-122 has been designed such that the secondary structure of the artificial pri-miR-31 or pri-miR-122 mimics the secondary structure of the naturally occurring pri-miR-31 or pri-miR-122; and
(ii) a Pol II promoter.

### BRIEF DESCRIPTION OF THE FIGURES

The invention will now be described, by way of non-limiting example, with reference to the accompanying drawings. In the drawings.

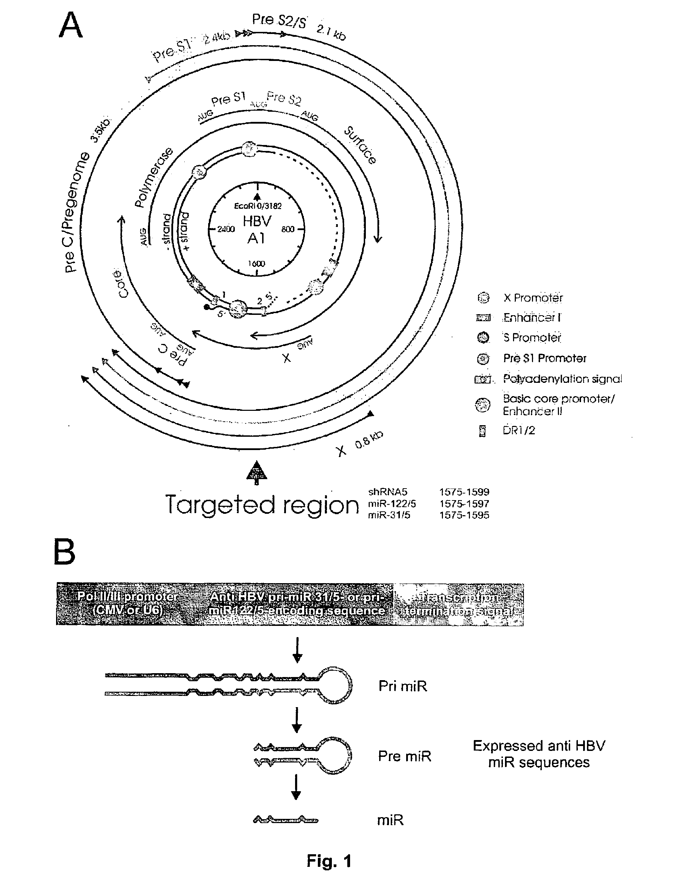

**Figure 1** **A** shows organization of the hepatitis B virus (HBV) genome showing sites targeted by a representative anti HBV miRs. Nucleotide co-ordinates of the genome are given relative to the single *Eco*RI restriction site. Partially double-stranded HBV DNA comprises + and - strands with cohesive complementary 5' ends. The *cis*-elements that regulate HBV transcription are indicated by the circular and rectangular symbols. Immediately surrounding arrows show the viral open reading frames (with initiation codons) that encompass the entire genome. Four outer arrows indicate the HBV transcripts, which have common 3' ends that include *HBx*.

**Figure 1** **B** shows a schematic illustration (not to scale) of anti HBV miR DNA expression cassettes. The arrangement of the PoIII (CMV) or Pol III (U6) promoter, miR-31/5- or miR-122/5-encoding sequences together with transcription termination sequences are indicated. Generation and processing of the primary miR (pri-miR), precursor miR (pre-miR) and miR transcripts are indicated schematically.

**Figure 2** shows a schematic illustration of the structure and sequences of pri-miR-31 and pri-miR-122 with representative anti HBV derivatives pri-miR-31/5 and pri-miR-122/5. The sequence of the putative pre-miRs generated after Drosha processing is indicated in colour (purple and red) and the mature processed guide sequence generated after Dicer processing and strand selection by RISC is indicated in red only. The anti HBV guide of pri-miR-31/5 targets HBV coordinates 1575-1595 and pri-miR-122/5 targets HBV coordinates 1575-1597.

**Figure 3** shows northern blot hybridization analysis of expressed miR shuttle sequences that were extracted from HEK293 cells after transfection with plasmids encoding the indicated miR or shRNA cassettes. Hybridization was to a radiolabelled probe complementary to the putative mature anti HBV guide 5 strand. Representative hybridization signals to detect precursors of mature miRs. Blots were stripped and rehybridized to a probe complementary to endogenous U6 snRNA to confirm equal loading of cellular RNA (lower panels of a, b and c).

**Figure 4** shows detection using northern blot analysis of processed siRNA sequences produced from expressed anti HBV miRs. Hybridisation was to a radiolabeled probe complementary to mature miR-31/5 sequences (upper panel). RNA was extracted from transfected HEK293 cells that had been transfected with plasmid vectors that included the indicated CMV (Pol II) or U6 (Pol III) pri-miR expression cassettes. Blots were stripped and rehybridised to a probe complementary to endogenous U6 snRNA to confirm equal loading of cellular RNA (lower panel).

**Figure 5** shows detection using northern blot analysis of processed siRNA sequences produced from expressed anti HBV miRs. Hybridisation was to a radiolabeled probe complementary to mature miR-122/5 sequences (upper panel). RNA was extracted from transfected HEK293 cells that had been transfected with plasmid vectors that included the indicated CMV (Pol II) or U6 (Pol III) expression cassettes. Blots were stripped and rehybridised to a probe complementary to endogenous U6 snRNA to confirm equal loading of cellular RNA (lower panel).

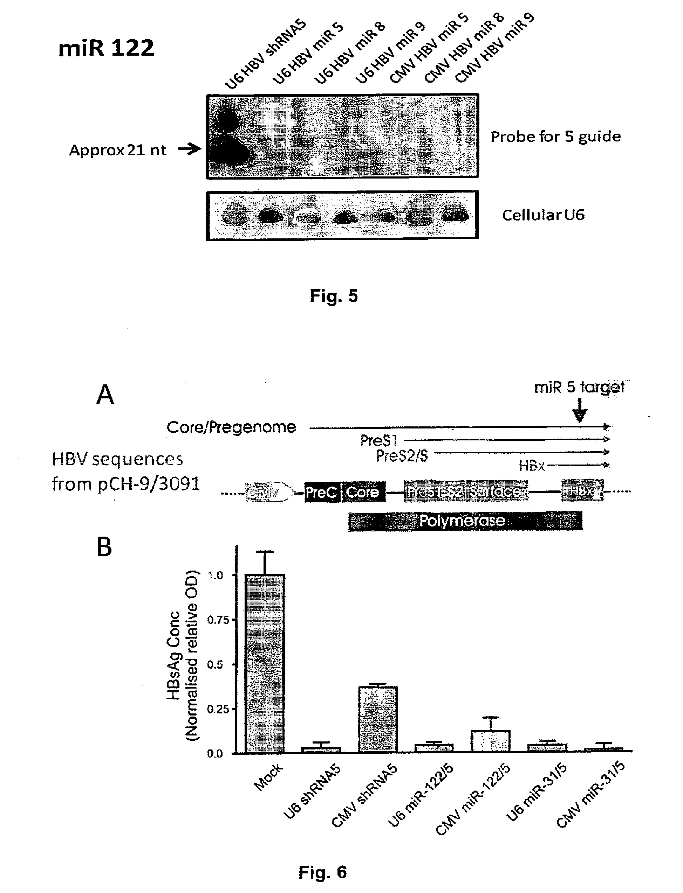

**Figure 6** shows HBsAg secretion from transfected Huh7 liver cells. **Figure 6** **A** illustrates the organization of the HBV genome with open reading frames and sites within the pCH-9/3091 target vector that are viral sequence targets complementary to processed products of miR-31/5 and miR-122/5 expressing vectors. Four parallel arrows indicate the HBV transcripts, which have common 3' ends, and include the miR-31/5 and miR-122/5 targets. **Figure 6** **B** shows data on the HBsAg secretion from Huh7 cells co-transfected with indicated miR- or shRNA-encoding plasmids together with HBV target plasmid. HBsAg measurements from quantitative ELISA are given as a normalized mean relative to the mock treated cells. Results are from 4 independent transfections and the bars indicate the standard error of the mean (SEM).

**Figure 7** **A** illustrates the structure of the pCH Firefly Luc target vector with HBV sequences and also Firefly luciferase open reading frame. The site targeted by miR-31/5 and miR-122/5 is indicated by an arrow. **Figure 7** **B** shows firefly luciferase reporter gene activity in Huh7 cells co-transfected with indicated miR-encoding plasm ids together with constitutively active *Renilla* luciferase-expressing plasmid. Measurements are given as a normalized ratio (±SEM) of firefly to *Renilla* luciferase activity and were determined from 3 independent experiments.

**Figure 8** shows the effects of miR sequences on markers of HBV replication in the hydrodynamic injection model of HBV replication. Serum HBsAg concentrations were determined at days 3 and 5 after hydrodynamic injection of mice with pCH-9/3091 HBV target and CMV miR-31/5, CMV miR-122/5 or U6 shRNA 5 plasmids. Results were normalised relative to the mock treated mice and are expressed as the mean (±SEM) from at least 5 mice.

**Figure 9** shows the effects of miR sequences on markers of HBV replication in the hydrodynamic injection model of HBV replication. Viral particle equivalents (VPEs) were determined at days 3 and 5 after hydrodynamic injection of mice with pCH-9/3091 HBV target and CMV miR-31/5, CMV miR-122/5 or U6 shRNA 5 plasmids. The number of circulating genome equivalents was determined using real time PCR with Eurohep calibration standards. Results were normalised relative to the mock treated mice and are expressed as the mean (±SEM) from at least 5 mice.

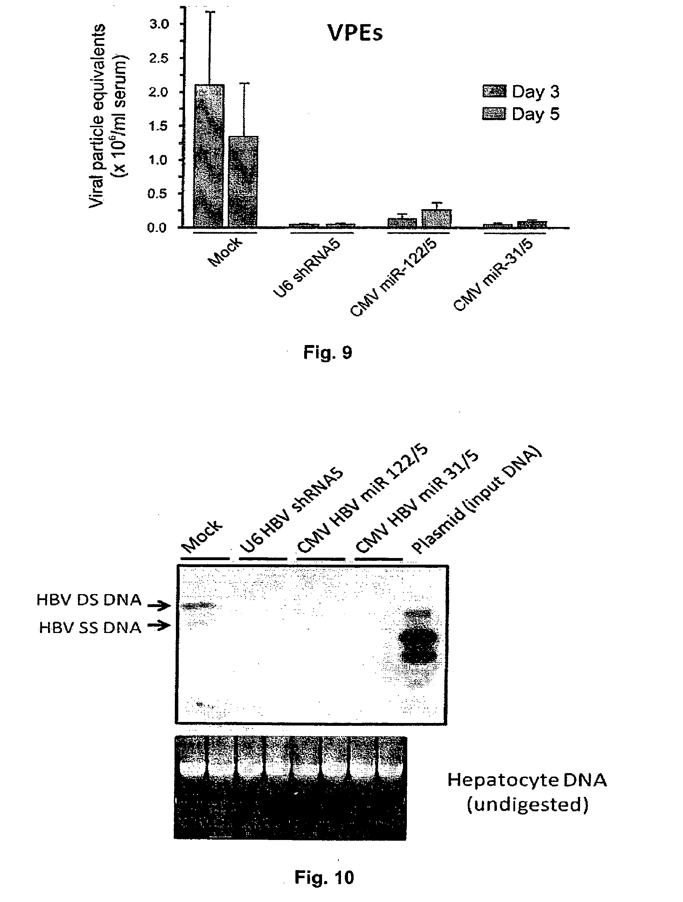

**Figure 10** shows Southern blot analysis of HBV DNA replication intermediates extracted from 2 representative animals from each of the groups of mice that had been subjected to the HDI procedure (upper panel). Mice had been injected with the indicated plasmids together with pCH 9/3091 HBV replication competent target. HBV double stranded (DS) and single stranded (SS) replication intermediates were only detectable in the mock treated animals, but not in any of the mice that had been coinjected with CMV miR-31/5, CMV miR-122/5 or U6 shRNA 5 plasmids. The HBV DNA bands detected in the mock treated mice did not correspond in size to any of the pCH 9/3091 bands. The lower panel, which is a representation of the separated DNA after ethidium bromide staining and prior to Southern transfer and hybridisation.

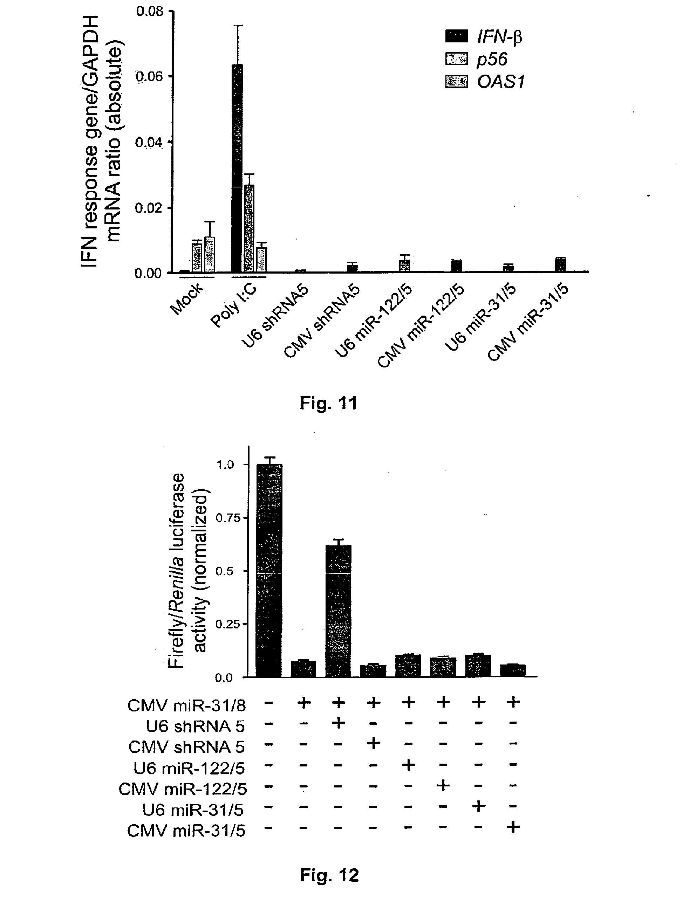

**Figure 11** shows assessment of interferon response in transfected HEK293 cells. Cells were transfected with the indicated miR-encoding cassettes, or with poly (I:C). RNA was extracted from the cells 24 hours later and then subjected to quantitative real time PCR to determine concentrations of *OAS1, IFN-ß,* p56 and *GAPDH* mRNA. Means (±SEM) of the normalized ratios of *IFN-ß, p56* or *OAS1* to *GAPDH* mRNA concentrations are indicated from 3 independent experiments. The poly (I:C) positive control verified that an interferon response was induced in the cells under the conditions used here.

**Figure 12** shows analysis of attenuation of independent RNAi-mediated silencing, which was carried out by cotransfection of liver-derived Huh7 cells with plasmids expressing the indicated shRNA or miR cassettes together with pCMV miR-31/8 and a psi-CHECK-8T dual luciferase vector. The reporter plasmid contained the independent HBV miR-31/8 cognate sequence downstream of the *Renilla* luciferase ORF. Measurement of *Renilla*:Firefly luciferase activity was used to assess effects of shRNA 5, miR-31/5 or miR-122/5 expressing plasmids on miR-31/8 silencing of its own independent target.

**Figure 13** shows effect of the amounts of transfected pU6 HBV shRNA 5 on attenuation of CMV miR-31/8 silencing. The indicated ratios of pCMV miR-31/8 to pU6 HBV shRNA 5 vectors were co transfected with the psi-CHECK-8T vector. Again measurements of *Renilla*:Firefly luciferase activities (±SEM) were used to assess effects of decreasing amounts of pU6 HBV shRNA 5 on miR-31/8 silencing of its own independent target.

**Figure 14** shows titration of inhibition of HBsAg secretion from Huh7 cells after transfection with decreasing amounts of pU6 HBV shRNA 5. The indicated ratios of pU6 HBV shRNA 5 to HBV replication competent pCH-9/3091 vectors were transfected and the HBsAg concentrations in the culture supernatants were determined 48 hours thereafter. Normalized mean relative OD readings (±SEM) from ELISA assays are represented.

**Figure 15** shows assessment of effects of miR shuttles on independent silencing *in vivo.* Mice were subjected to HDI with the psi-CHECK-8T vector together with the indicated RNAi expression cassettes. Where relevant, the ratios of the CMV miR-31/5, CMV miR-122/5 and U6 shRNA 5 to CMV miR-31/8 vectors are indicated in parentheses. Normalized mean *Renilla:*Firefly luciferase activities (±SEM) were determined in liver homogenates 3 days after plasmid injection.

**Figure 16** shows a schematic representation of cloning strategy for the generation of liver-specific expression vectors. The liver-specific promoters that were propagated in the pTZ-derived vectors are illustrated as green arrows and show 5' to 3' polarity.

**Figure 17** shows expression of Firefly luciferase in transfected cells that were transfected with indicated expression vectors. Means of relative Firefly luciferase activity in liver-derived cells (black bars) and kidney-derived cells (gray bars) are shown. Error bars indicate Standard Error of the Mean (SEM).

**Figure 18** shows expression of pri-miR-122 shuttles from various expression vectors. Means of Firefly luciferase activity relative to *Renilla* luciferase activity are shown. Black bars indicate expression in Huh 7 cells and grey bars indicate expression in 116 cells. Error bars indicate SEM.

**Figure 19** shows a schematic illustration of the trimeric anti HBV pri-miR 31 expression cassette. The transcript generated from the CMV Pol II promoter comprises 3 pri-miR sequences that are processed to form independent pre-miRs, which in turn re the precursors of mature miR guide sequences that target independent sites of HBV.

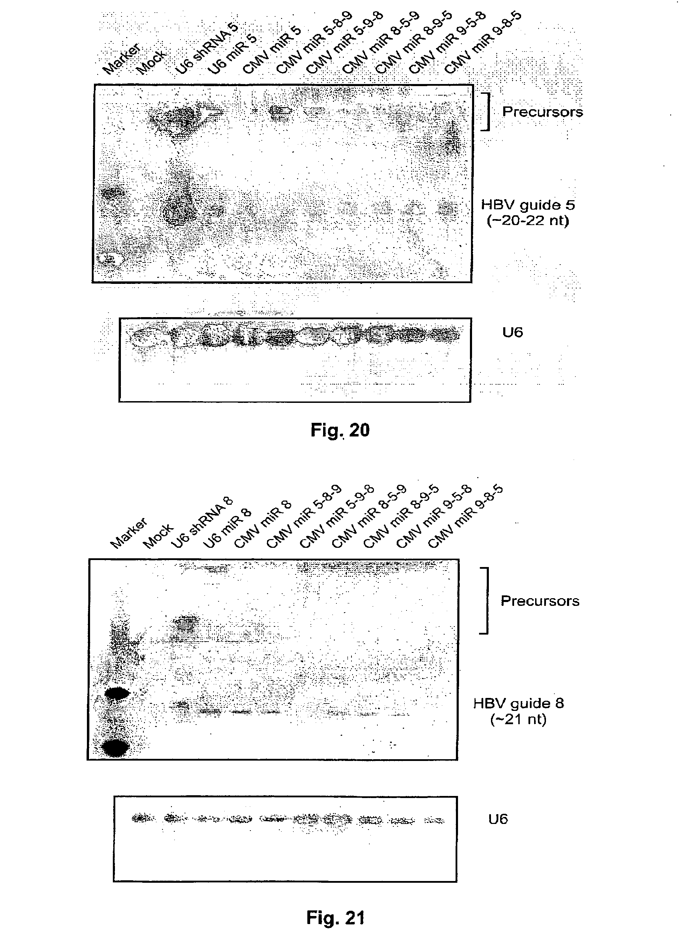

**Figure 20** shows northern blot analysis of RNA extracted from cultured liver-derived cells that had been transfected with the indicated anti HBV RNAi-activating expression cassettes or with backbone pCl neo plasmid that did not contain miR sequences (mock). Electrophoretically resolved RNA was probed with an oligonucleotides complementary to the putative guide 5 sequence (upper panel). U6 plasmids contain the Pol III U6 promoter that drives expression of shRNA 5 or miR 31/5. Vectors with each possible ordering combination of the individual pri miRs within the trimeric cassettes were also used to transfect cultured cells. These multimeric pri-miR shuttles were expressed from the CMV promoter derived from pClneo plasmid. To control for equal loading and transfer of RNA the blots were stripped and reprobed with an oligonucleotide that was complementary to U6 snRNA (lower panel).

**Figure 21** shows northern blot analysis of RNA extracted from cultured liver-derived cells that had been transfected with the indicated anti HBV RNAi-activating expression cassettes or with backbone pCl neo plasmid that did not contain miR sequences (mock). Electrophoretically resolved RNA was probed with an oligonucleotide complementary to the putative guide 8 sequence (upper panel). U6 plasmids contain the Pol III U6 promoter that drives expression of shRNA 8 or miR 31/8. Vectors with each possible ordering combination of the individual pri miRs within the trimeric cassettes were also used to transfect cultured cells. These multimeric pri-miR shuttles were expressed from the CMV promoter derived from pClneo plasmid. To control for equal loading and transfer of RNA the blots were stripped and reprobed with an oligonucleotide that was complementary to U6 snRNA (lower panel).

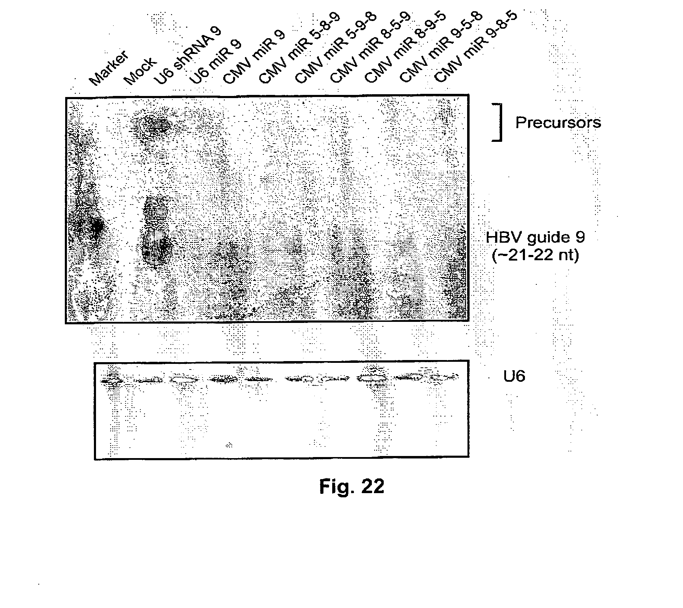

**Figure 22** shows northern blot analysis of RNA extracted from cultured liver-derived cells that had been transfected with the indicated anti HBV RNAi-activating expression cassettes or with backbone pCl neo plasmid that did not contain miR sequences (mock). Electrophoretically resolved RNA was probed with an oligonucleotide complementary to the putative guide 9 sequence (upper panel). U6 plasmids contain the Pol III U6 promoter that drives expression of shRNA 9 or miR 31/9. Vectors with each possible ordering combination of the individual pri miRs within the trimeric cassettes were also used to transfect cultured cells. These multimeric pri-miR shuttles were expressed from the CMV promoter derived from pClneo plasmid. To control for equal loading and transfer of RNA the blots were stripped and reprobed with an oligonucleotide that was complementary to U6 snRNA (lower panel).

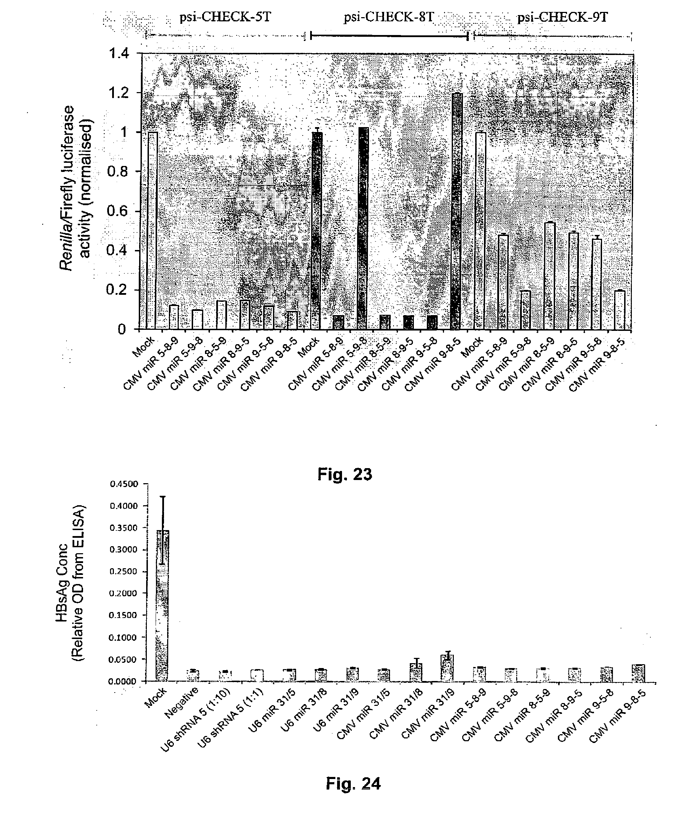

**Figure 23** shows assessment of knockdown of reporter gene expression using the dual luciferase assay. PsiCHECK-derived plasmids containing indicated target sequences complementary to guide sequences 5, 8 and 9 were transfected into cultured cells together with the trimeric pri-miR shuttle-expressing plasmids. Plasmids with each possible ordering combination of the individual pri miRs within the trimeric cassettes were used to transfect cultured cells. Mock treated cells received the psiCHECK vectors together with pClneo backbone plasmid that did not contain anti HBV sequences. In all transfections, cells received the plasmids at a mass ratio of 10:1 for RNAi effecter to psiCHECK-derived plasmids. The ratio of *Renilla* to Firefly luciferase activity was measured and used to determine knockdown efficiency.

**Figure 24** shows assessment of knockdown of HBsAg secretion from transfected cells. pCH9/3091 HBV replication competent target plasmid was transfected into cultured cells together with the monomeric or trimeric pri-miR shuttle-expressing plasmids. Mock treated cells received the pCH9/3091 vector together with pClneo backbone plasmid that did not contain anti HBV sequences. The negative cells did not receive pCH9/3091 and were only transfected with pClneo backbone plasmid. U6 plasmids contain the Pol III U6 promoter that drives expression of monomeric shRNA 5, pri miR 31/5, pri miR 31/8 or pri miR 31/9-expressing sequences. Ratios of 10:1 and 1:1 of U6 shRNA 5 vector to pCH9/3091 were tested, but for all other combinations, the ratio of RNAi expressing vector to pCH9/3091 was 10:1. Pol II monomeric plasmids generated pri miR 31/5, pri miR 31/8 or pri miR 31/9 from the CMV promoter. Again, plasmids with each possible ordering combination of the individual pri miRs within the trimeric cassettes expressed from the CMV were also transfected into cultured cells. To assess effects of these RNAi effecters on HBsAg secretion, the concentration of this viral antigen was determined in the culture supernatant 48 hours after transfection.

**Figure 25** shows a secondary structure prediction of miR-30 as predicted by the online software mFOLD.

**Figure 26** shows a secondary structure prediction of miR31 as predicted by the online software mFOLD.

**Figure 27** shows a secondary structure prediction of sh260 targeting HCV 5' UTR as predicted by the online software mFOLD.

**Figure 28** shows a secondary structure prediction of miR260 targeting HCV 5' UTR as predicted by the online software mFOLD.

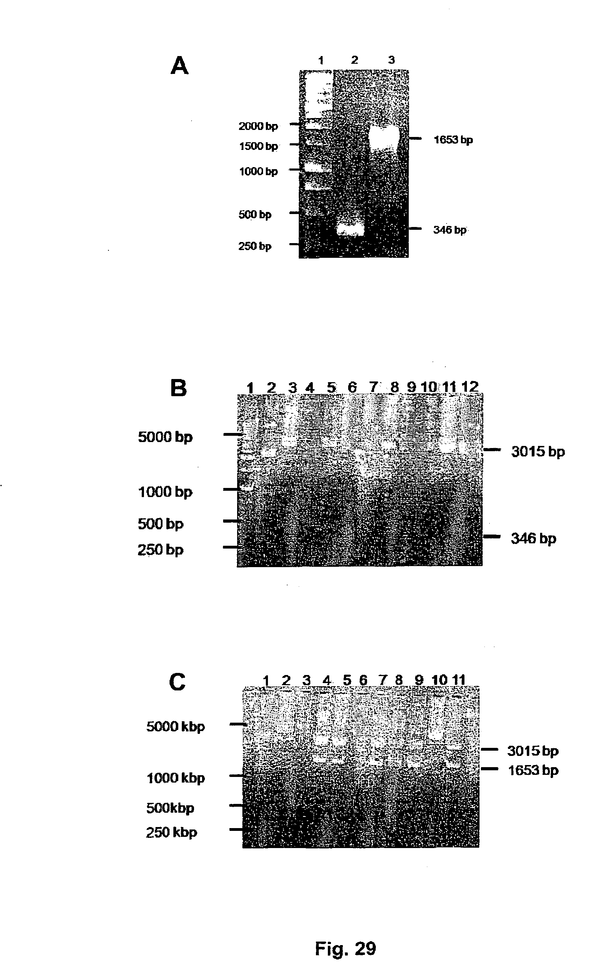

**Figure 29** Cloning of pGEM-T-UTR and pGEM-T-LUC showing (A) an agarose electrophoretogram showing Benchtop 1kb marker (lane 1) and the products of the PCR amplification of HCV 5'UTR (lane 2) and firefly luciferase (lane 3), (B) an agarose electropherogram of EcoRI-restricted putative pGEM-T-UTR showing Benchtop 1kb marker (lane 1), unrestricted plasmid DNA from clone 1 (lane 2), and *Eco*RI-restricted plasmid DNA from clones 1-10 (lanes 3-12), and (C) an agarose electropherogram of *Eco*RI-restricted putative pGEM-T-LUC showing unrestricted plasmid DNA from clone 1 (lane 1), and *Eco*RI-restricted plasmid DNA from clones 1-10 (lanes 2-11).

**Figure 30** The cloning of pCineoUTRLUC showing Benchtop 1 kb marker (lane 1), unrestricted plasmid DNA from clone 1 (lane 2), and *Sph*I-restricted plasmid DNA from clones 1-22 (lanes 3-24).

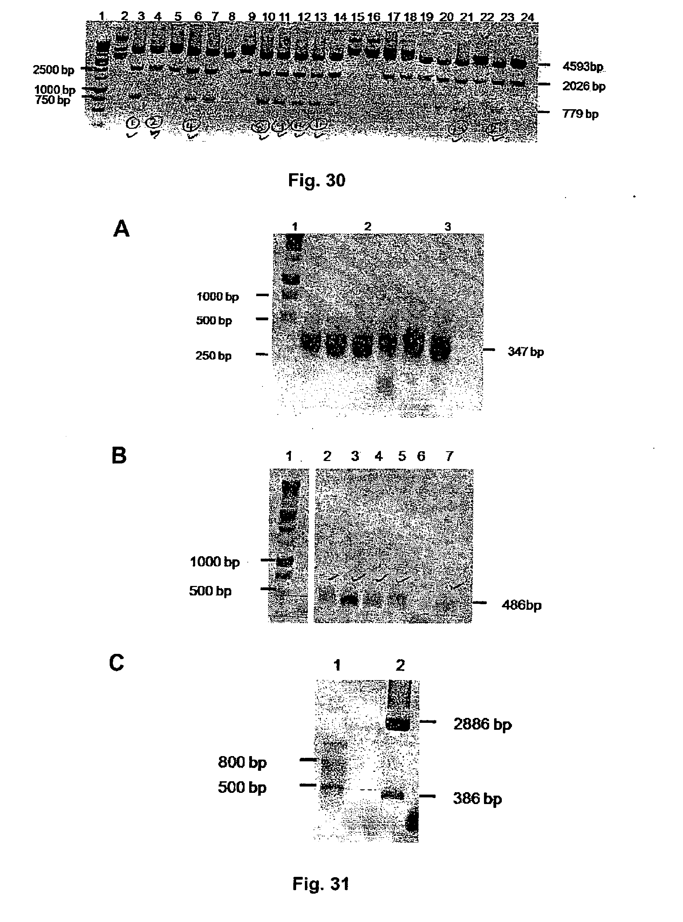

**Figure 31** The cloning of pTz57R-sh260 showing (A) an agarose electropherogram of the two-step PCR reaction products showing Benchtop 1 kb marker (lane 1), the PCR products of the first sh260 PCR reaction (lane 2), and the PCR products of the second sh260 PCR reaction (lane 3), (B) an agarose electropherogram of the PCR screening of putative pTz57R-sh260 plasmid DNA showing Benchtop 1 kb marker (lane 1), the PCR products of the PCR screening of clones 1-6 (lanes 2-7), (C) an agarose electropherogram of *Sal*I-restricted putative pTz57R-sh260 plasmid DNA showing Benchtop 100 bp marker (lane 1) and *Sal*I-restricted plasmid DNA from clone 3.

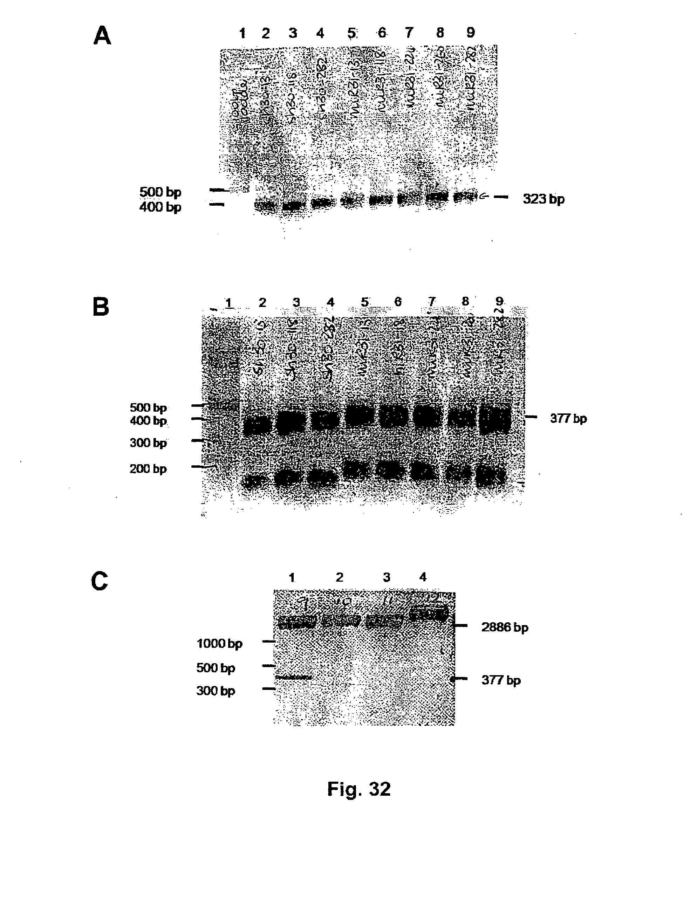

**Figure 32** The cloning of pTz57R-miR260 showing (A) an agarose electropherogram of the first step of the two-step PCR reaction products showing Benchtop 100 bp marker (lane 1) and the PCR products of the first miR260 PCR reaction (lane 8), (B) an agarose electropherogram of the second step of the two-step PCR reaction products showing Benchtop 100 bp marker (lane 1) and the PCR products of the second miR260 PCR reaction (lane 8), (C) an agarose electropherogram of *Sal*I-restricted putative pTz57R-miR260 plasmid DNA showing Benchtop 1 kb marker (lane 1) and *Sal*I-restricted plasmid DNA from clones 1-4 (lanes 1-4).

**Figure 33** A bar graph showing the relative luciferase activity (Firefly/Renilla) of HuH-7 cells transfected with target construct pCineoUTRLUC and pCMV-Ren, and co-transfected with pTz57R-miR118, pTz57R-sh260, or pTz57R-miR260, where the results are from one experiment representive of two experiments performed in triplicate (bars indicate the mean ± SD).

**Figure 34** *Homo sapiens* PRI MIR 31

**Figure 35** *Homo sapiens* PRI MIR 122

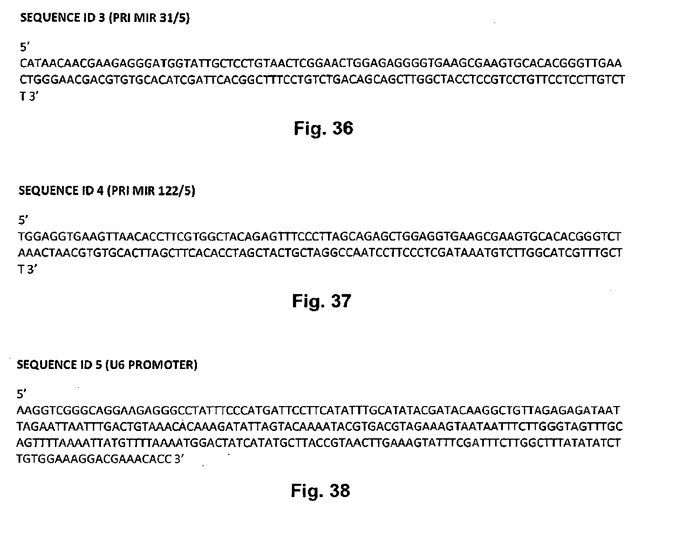

**Figure 36** PRI MIR 31/5

**Figure 37** PRI MIR 122/5

**Figure 38** U6 Promoter

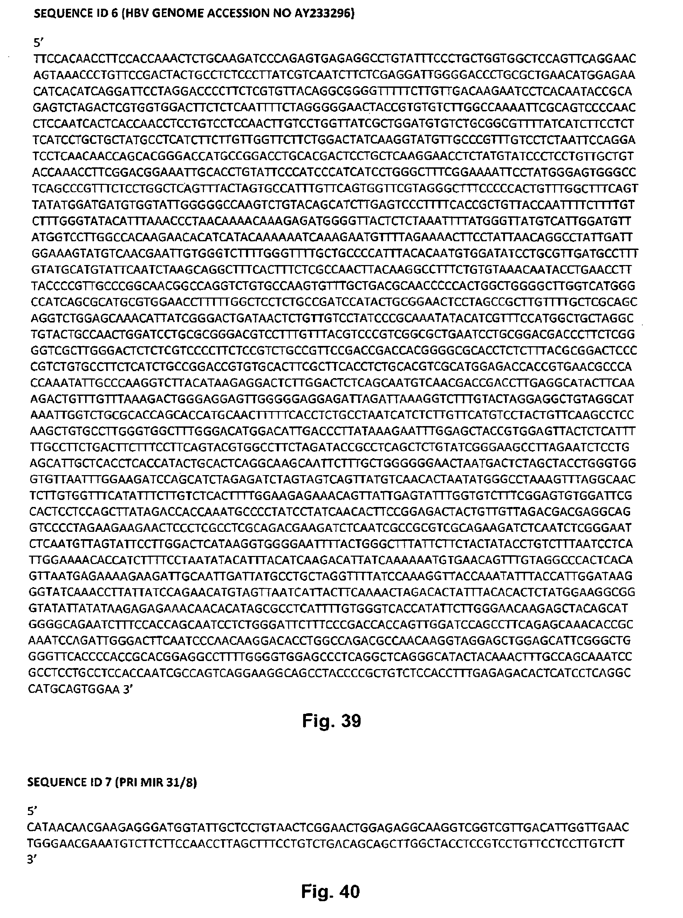

**Figure 39** HBV Genome Accession No AY233296

**Figure 40** PRI MIR 31/8

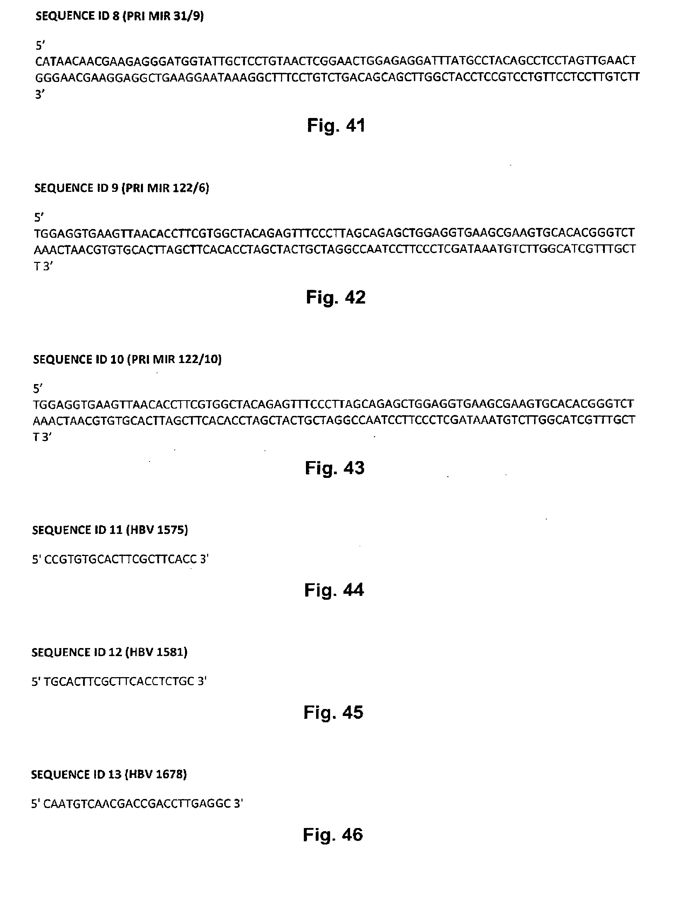

**Figure 41** PRI MIR 31/9

**Figure 42** PRI MIR 122/6

**Figure 43** PRI MIR 122/10

**Figure 44** HBV 1575

**Figure 45** HBV 1581

**Figure 46** HBV 1678

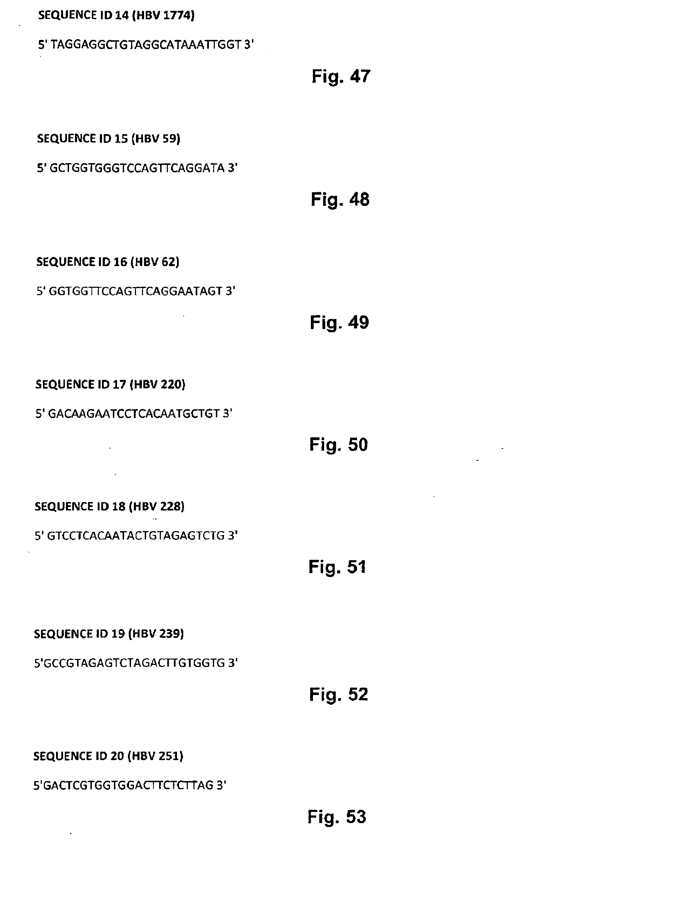

**Figure 47** HBV 1774

**Figure 48** HBV 59

**Figure 49** HBV 62

**Figure 50** HBV 220

**Figure 51** HBV 228

**Figure 52** HBV 239 **Figure 53** HBV 251

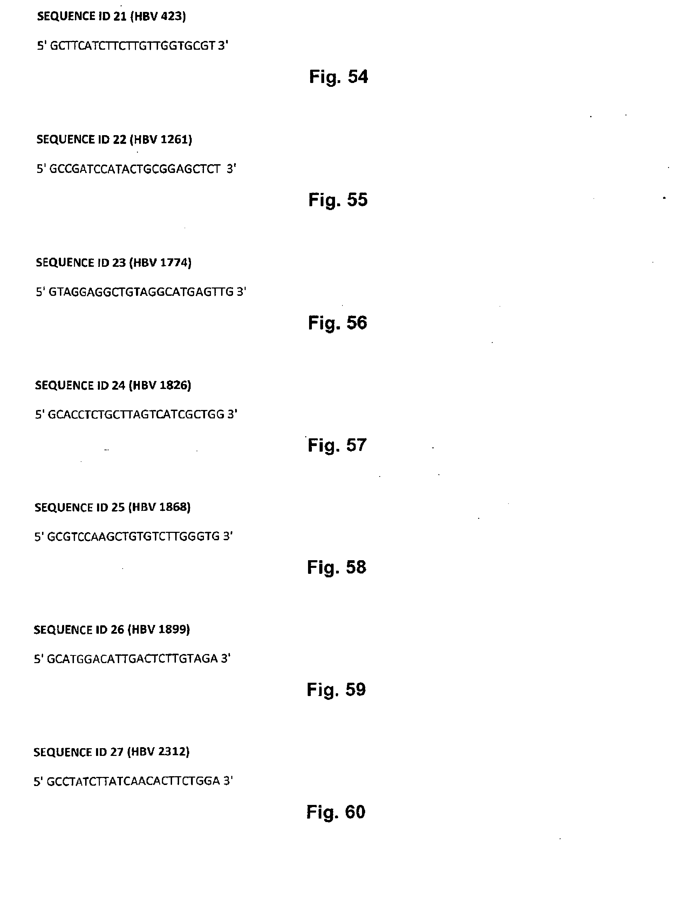

**Figure 54** HBV 423

**Figure 55** HBV 1261

**Figure 56** HBV 1774

**Figure 57** HBV 1826

**Figure 58** HBV 1868

**Figure 59** HBV 1899

**Figure 60** HBV 2312

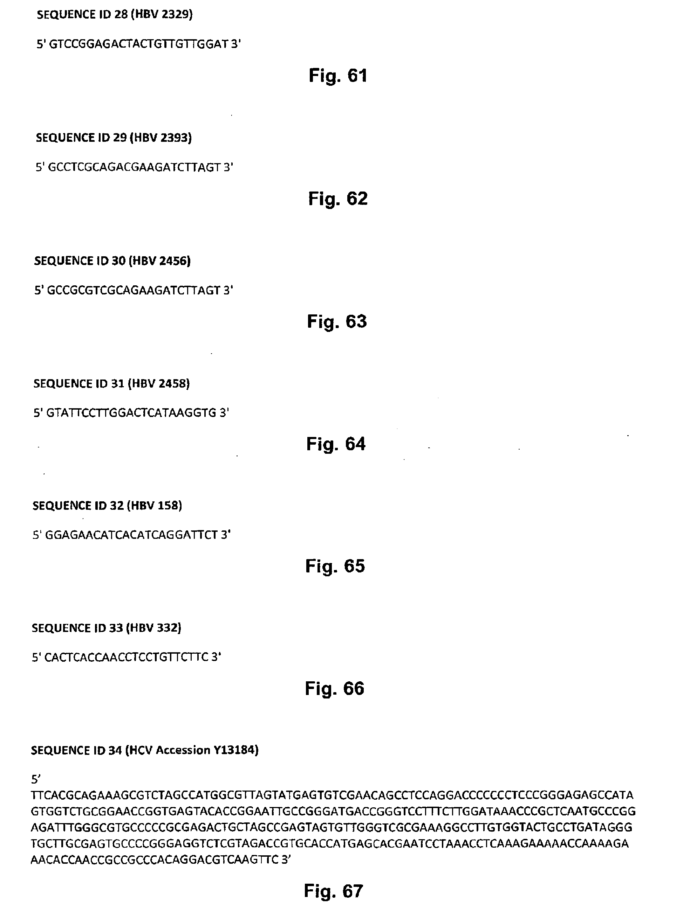

**Figure 61** HBV 2329

**Figure 62** HBV 2393

**Figure 63** HBV 2456

**Figure 64** HBV 2458

**Figure 65** HBV 158

**Figure 66** HBV 332

**Figure 67** HBV Accession Y13184

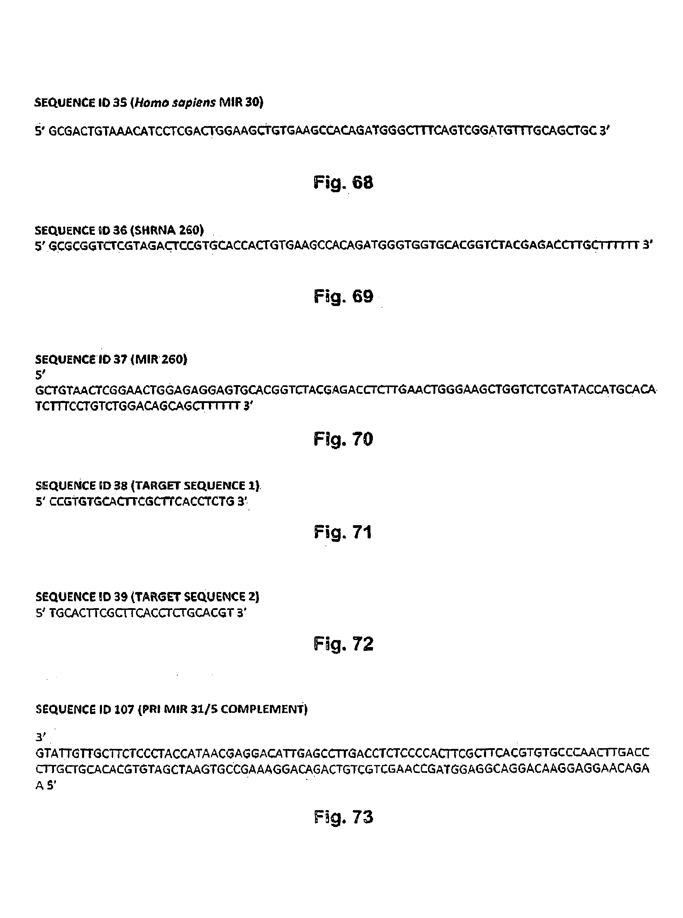

**Figure 68** *Homo sapiens* MIR 30

**Figure 69** shRNA260

**Figure 70** MIR 260

**Figure 71** Target sequence

**Figure 72** Target sequence

**Figure 73** PRI MIR 31/5 Complement

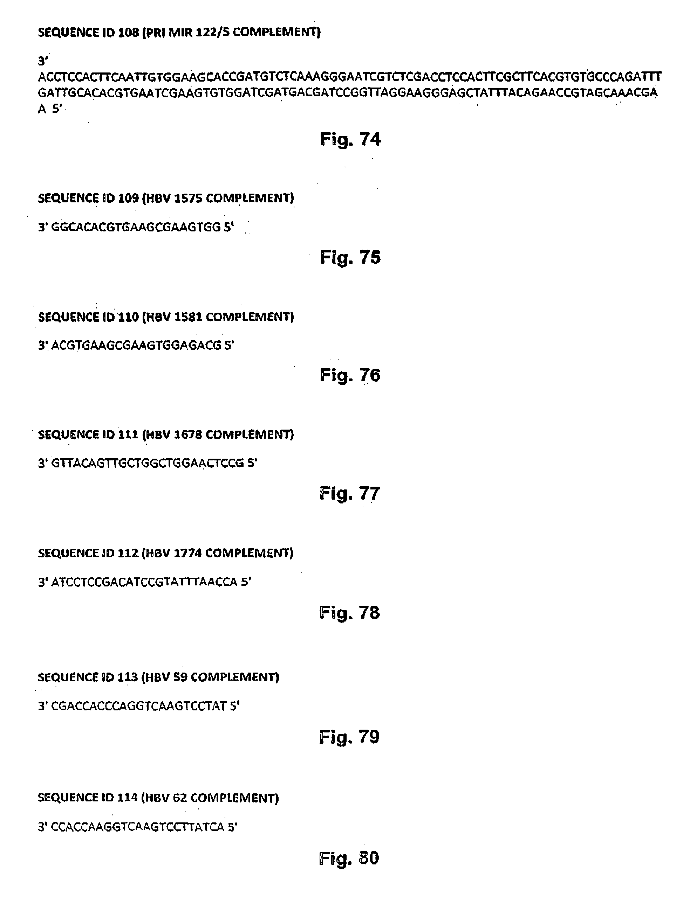

**Figure 74** PRI MIR 122/5 Complement

**Figure 75** HBV 1575 Complement

**Figure 76** HBV 1581 Complement

**Figure 77** HBV 1678 Complement

**Figure 78** HBV 1774 Complement

**Figure 79** HBV 59 Complement

**Figure 80** HBV 62 Complement

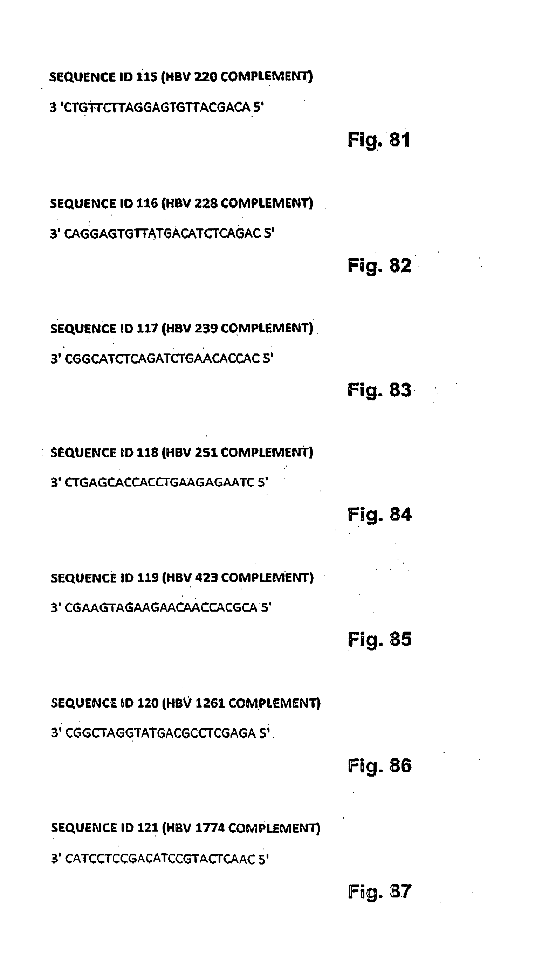

**Figure 81** HBV 220 Complement

**Figure 82** HBV 228 Complement

**Figure 83** HBV 239 Complement

**Figure 84** HBV 251 Complement

**Figure 85** HBV 423 Complement

**Figure 86** HBV 1261 Complement

**Figure 87** HBV 1774 Complement

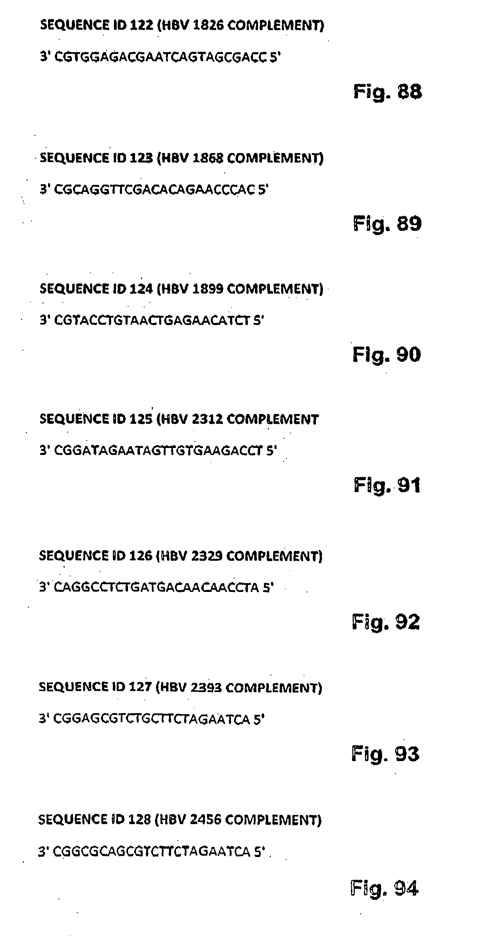

**Figure 88** HBV 1826 Complement

**Figure 89** HBV 1868 Complement

**Figure 90** HBV 1899 Complement

**Figure 91** HBV 2312 Complement

**Figure 92** HBV 2329 Complement

**Figure 93** HBV 2393 Complement

**Figure 94** HBV 2456 Complement

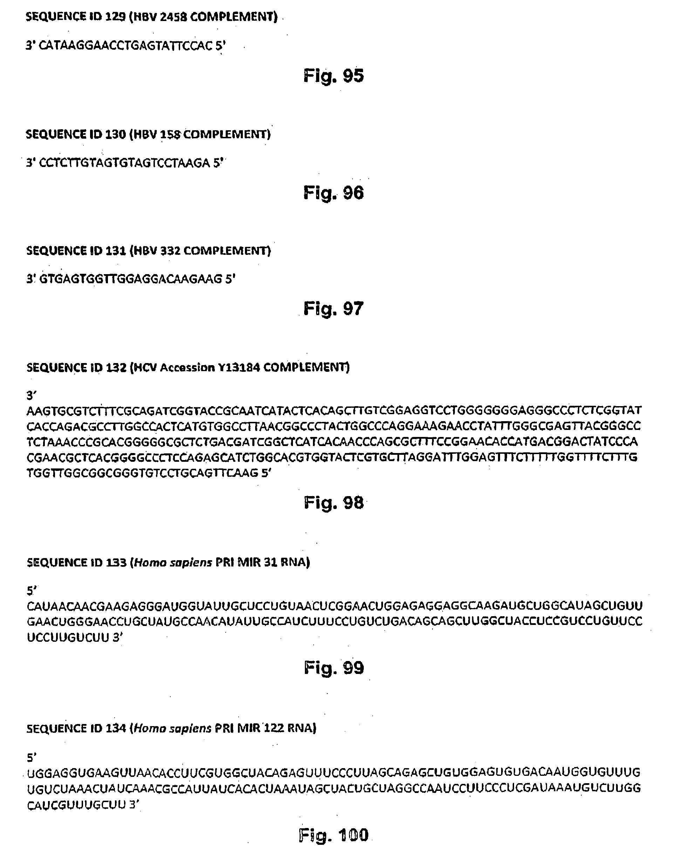

**Figure 95** HBV 2458 Complement

**Figure 96** HBV 158 Complement

**Figure 97** HBV 332 Complement

**Figure 98** HBV Accession Y3184 Complement

**Figure 99** *Homo sapiens* PRI MIR 31 RNA

**Figure 100** *Homo sapiens* PRI MIR 122 RNA

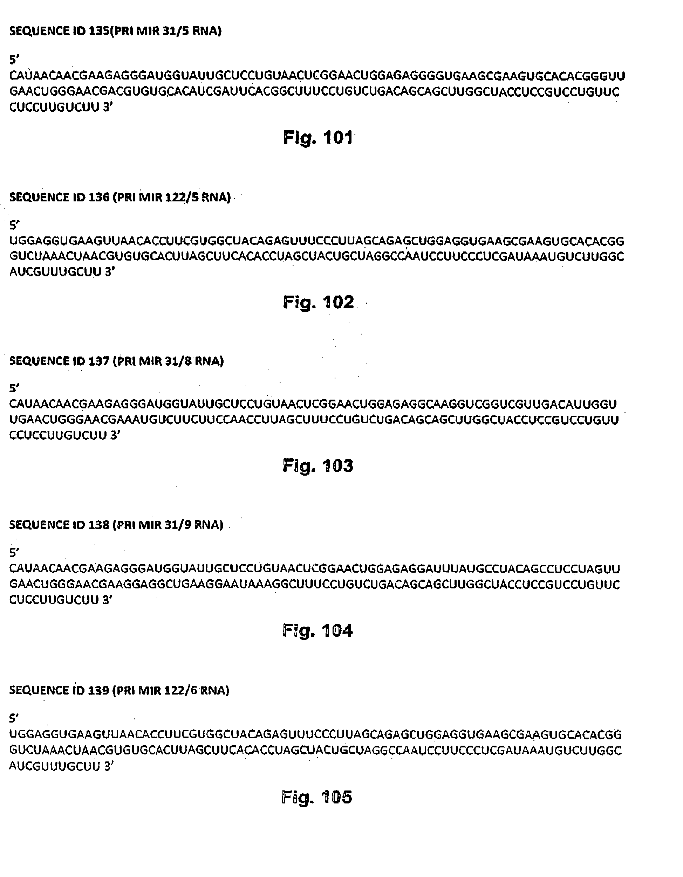

**Figure 101** PRI MIR 31/5 RNA

**Figure 102** PRI MIR 122/5 RNA

**Figure 103** PRI MIR 31/8 RNA

**Figure 104** PRI MIR 31/9 RNA

**Figure 105** PRI MIR 122/6 RNA

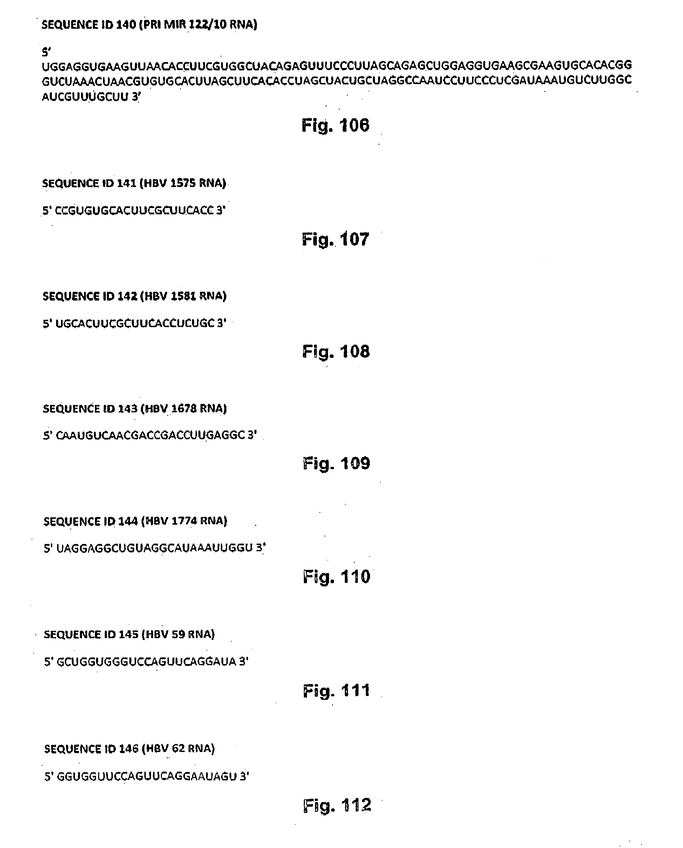

**Figure 106** PRI MIR 122/10 RNA

**Figure 107** HBV 1575 RNA

**Figure 108** HBV 1581 RNA

**Figure 109** HBV 1678 RNA

**Figure 110** HBV 1774 RNA

**Figure 111** HBV 59 RNA

**Figure 112** HBV 62 RNA

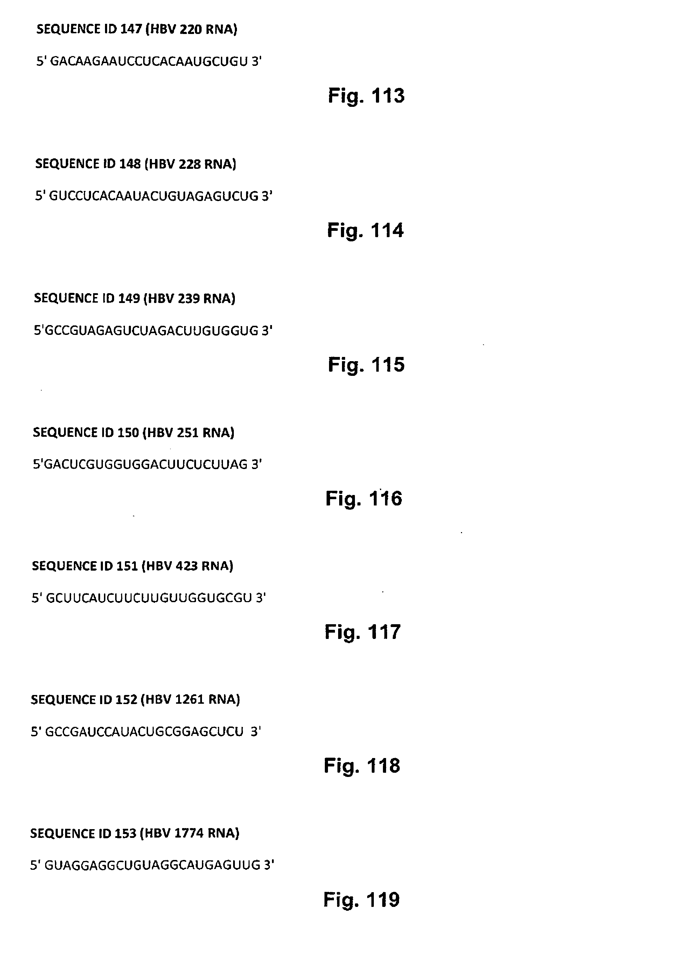

**Figure 113** HBV 220 RNA

**Figure 114** HBV 228 RNA

**Figure 115** HBV 239 RNA

**Figure 116** HBV 251 RNA

**Figure 117** HBV 423 RNA

**Figure 118** HBV 1261 RNA

**Figure 119** HBV 1774 RNA

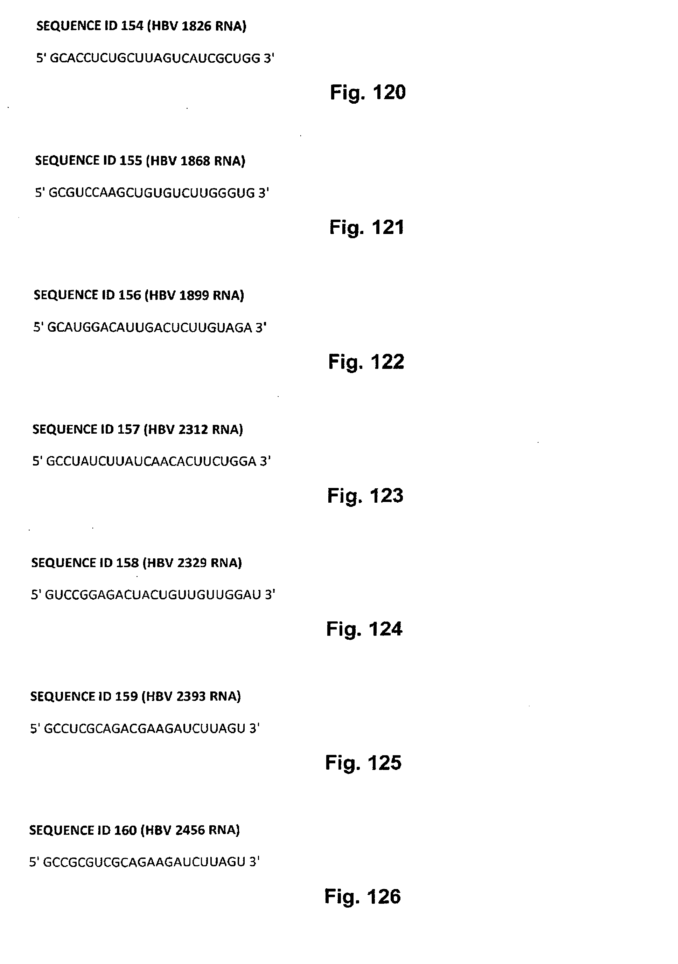

**Figure 120** HBV 1826 RNA

**Figure 121** HBV 1868 RNA

**Figure 122** HBV 1899 RNA

**Figure 123** HBV 2312 RNA

**Figure 124** HBV 2329 RNA

**Figure 125** HBV 2393 RNA

**Figure 126** HBV 2456 RNA

**Figure 127** HBV 2458 RNA

**Figure 128** HBV 158 RNA

**Figure 129** HBV 332 RNA

**Figure 134** *Homo sapiens* MIR 30 RNA

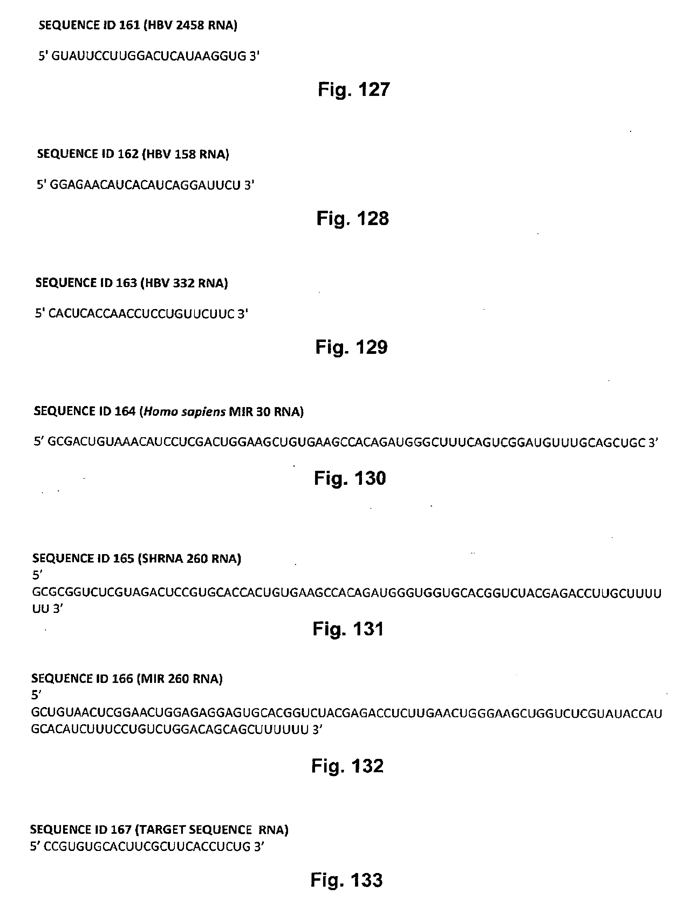

**Figure 131** shRNA260 RNA

**Figure 132** MIR 260 RNA

**Figure 133** Target sequence RNA

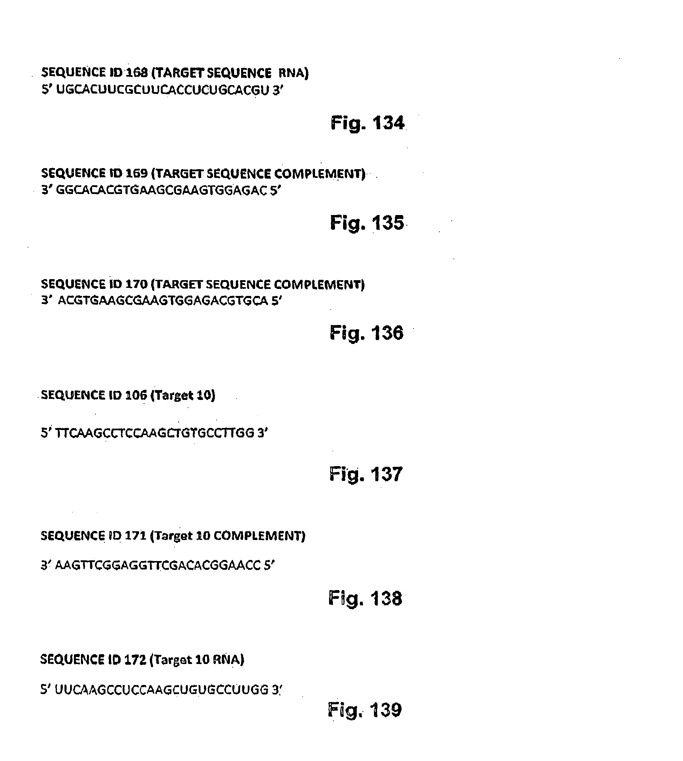

**Figure 134** Target sequence RNA

**Figure 135** Target sequence Complement

**Figure 136** Target sequence Complement

**Figure 137** Target 10

**Figure 138** Target 10 Complement

**Figure 139** Target 10 RNA

### DETAILED DESCRIPTION OF THE INVENTION

Activation of the RNAi pathway to effect specific gene silencing has prompted enthusiasm for the potential of nucleic acid-based HBV treatment. RNAi involves specific and powerful gene silencing through predictable complementary interaction between RNAi effecters and their targets. Naturally, RNAi plays an important role in regulation of gene expression through processing of endogenous miRs, which control several cellular processes that include organogenesis, apoptosis, cell proliferation and tumorigenesis. miRs are transcribed by Pol II as pri-miR hairpin-like structures, which are then processed to form precursor miRs (pre-miRs) within the nucleus. This step is catalyzed by Drosha (an RNAse III enzyme) together with Di George critical region 8 protein (DGCR8), which is its double stranded RNA binding (dsRBD) partner. Some endogenous miRs are polycistronic and more than one mature miR, with different may be generated from a single transcript. After export from the nucleus, pre-miRs are processed by Dicer with associated dsRBD TAR RNA-binding protein. The resulting 19-24 bp duplex is handed on to the RNA induced silencing complex (RISC) before selection of one strand as the mature miR guide. miRs are usually not entirely complementary to their targets and bind to the 3' untranslated regions of cognate mRNA to induce translational suppression. When base pairing between guide and target is perfectly matched, the Ago2 component of RISC exerts silencing through site-specific cleavage of the guide complement.

The specific and powerful gene silencing that may be induced by RNAi has prompted investigation of RNAi-based therapeutic modalities to inhibit expression of pathology-causing genes, which include those of viruses such as HBV and hepatitis C virus (HCV). Typically, exogenous RNAi-inducing sequences have been either synthetic short interfering RNA (siRNA) duplexes or expressed shRNA sequences. Synthetic siRNAs are similar to Dicer cleavage products and cause gene silencing by direct activation of RISC. shRNAs enter the RNAi pathway at an earlier stage and act as pre-miR mimics. Constitutively active Pol III promoters have been favored to transcribe shRNAs because of their ability to generate short, defined transcripts with a minimal requirement for regulatory elements within the transcript-encoding sequences. Several sites of the HBV genome have been targeted with synthetic and expressed RNA sequences and impressive knockdown of markers of viral replication has been shown. However, recent demonstration that U6 Pol III-expressed anti HBV shRNAs cause serious toxicity *in vivo* as a result of saturating the endogenous miR pathway is an important concern for therapeutic application of expressed RNAi sequences. Tissue-specific and inducible Pol II promoters may therefore be preferable to Pol III regulatory elements as they provide a better means of transcription control and dose regulation of expressed RNAi effecters. Some reports have demonstrated efficient silencing by Pol II RNAi expression cassettes, but this approach has been hampered by unpredictable and variable silencing efficacy of conventional hairpin sequences. Sequences upstream and downstream of the hairpins, which are incorporated into Pol II-derived transcripts, may interfere with processing of the silencers. To improve transcription control of potentially therapeutic sequences, we have taken advantage of the natural Pol II-mediated transcriptional control of cellular miRs. Anti HBV sequences were incorporated into expression cassettes that encode mimics of pri-miR-31 or pri-miR-122. Potent silencing of markers of viral replication was achieved *in vitro* and *in vivo* when anti HBV pri-miR shuttle expression cassettes were placed under control of Pol II liver specific promoters. Also, these shuttle sequences enable production of multimeric silencing sequences that target different sequences simulataneously.

The invention describes a method of inhibiting or silencing hepatitis virus (in particular hepatitis B or C virus) expression using expression cassettes that encode mimics of primary micro RNAs incorporating one or more target sequences of the hepatitis virus.

The anti-hepatitis primary micro RNA (pri-miR) expression cassette generally includes a DNA sequence encoding an artificial (i.e. engineered) pri-miR sequence which mimics a naturally occurring miR sequence (such as miR-30, miR-31 and mi-R-122), the guide sequence of the naturally occurring pri-miR sequence (and the complementary sequence of the guide sequence) having been replaced with a sequence which targets hepatitis virus.

The expression cassette includes a Pol II promoter, which may be a constitutive promoter, such as CMV, or a tissue specific promoter, such as the liver-specific tissue promoters alpha-1-antitrypsin (A1AT) promoter, Factor VIII (FVIII) promoter, HBV basic core promoter (BCP) and PreS2 promoter. The expression cassette may also include a promoter/transcription regulatory sequence and/or a termination signal.

The selection of target sites that were used against the hepatitis B virus (HBV) were based on the conservation of the sequences amongst all the HBV genotypes, and also on the predicted susceptibility of the targets to short hairpin RNAs (shRNAs) silencing. This was analysed using an algorithm that is available online through the City of Hope Hospital (Duarte, CA) (Heale et al [45]). Although the hepatitis B virus is relatively well-conserved, there are many different known genotypes, and hence sequences of 90% and 95% to the target sequences described in the examples are claimed so as to also cover the targets in these other genotypes.

The expression cassette may be incorporated into a viral or non-viral vector, which may be used to introduce the expression cassette into a host cell or organism.

The expression cassette is intended to be used to treat or prevent hepatitis infection, in particular in a human, and may be incorporated into a pharmaceutical composition, such as an anti hepatitis medicament which also includes a pharmaceutically acceptable adjuvant and/or carrier.

The present invention is further described by the following examples. Hepatitis B targets 5, 6, 8, 9 and 10 were tested for silencing with miRs, but the other targets described herein have been subjected to less complicated shRNA silencing tests. A person skilled in the art will understand that if the shRNAs work then the miRs will also do so.

### Example 1: Design and propagation of anti HBVpri-miR-expressing plasmids

### 1. Design

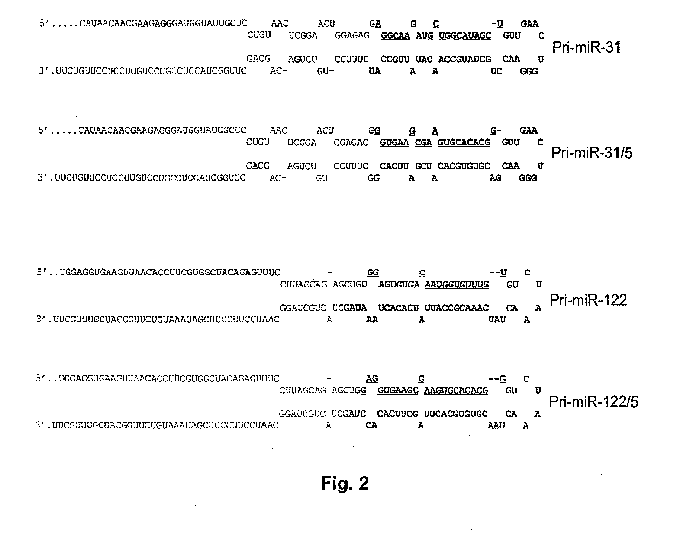

The pri-miR expression cassettes were designed by replacing the guide sequences of naturally occurring miR-31 (SEQ ID NOs: 1/ 107/ 133) and miR-122 (SEQ ID NOs: 2/ 108/ 134) with the guide sequence targeting an HBV sequence (SEQ ID NO: 11/ 109/ 141). The entire sequence of the encoded anti HBV pri miR sequences are given in SEQ ID NOs: 3 (135) and 4 (136). The wild-type sequences of the miR were maintained as far as possible and computer-aided prediction [38] of secondary structure of the transcripts did not differ significantly from that of their respective wild-type miRs. The final cassettes contained 51 nucleotides of wild-type sequences flanking either end the pre-miR (Zeng and Cullen, 2005). To facilitate cloning of the miR cassettes restriction sites for *Nhe* I and *Spe* I were included at their 5' and 3' ends, respectively. A schematic illustration of the targeted genomic site of HBV and also the pri-miR expression cassettes are indicated in Figure 1. Figure 2 shows the structure of the wildtype pri-miR-31 and miR122 sequences together with their anti HBV derivatives (pri-miR-31/5 and miR122/5).

### 1. Generation of cassettes encoding miR sequences that target HBV

*miR-derived anti HBV sequences.* DNA encoding pre-miR-31 and pre-miR-122 containing the guide sequence targeting Hepatitis B Virus (HBV coordinates 1781 to 1801) (SEQ ID NOs: 3 and 4) (Figure 1) were generated by primer extension of paired pre-miR31/5 and pre-miR-12215 forward and reverse oligonucleotides. Oligodeoxynucleotides encoding the miR sequences were synthesised using phosphoramadite chemistry (Inqaba Biotech, South Africa). Primer extensions were performed as PCR using Promega's PCR Master Mix (Promega, WI, USA). The thermal cycling conditions were as follows: Initial denaturation at 94°C for 5 minutes, followed by 30 cycles of denaturation at 94°C for 10 seconds, annealing at 50°C for 10 seconds and extension at 72°C for 10 seconds and a final extension step at 72°C for 10 minutes. The sequences of the oligonucleotides encoding the miR cassettes that target the HBV coordinates 1781-1801 were:
**Pre-miR-31/5 Forward**
   5'- GTAACTCGGAACTGGAGAGGGGTGAAGCGAAGTGCACACGGGTTGAACTGGGAACGACG -3' (SEQ ID NO: 40)
**Pre-miR-31/5 Reverse**
   5'-CTGCTGTCAGACAGGAAAGCCGTGAATCGATGTGCACACGTCGTTCCCAGTTCAACCCTG -3' (SEQ ID NO: 41)
**Pre-miR-122/5 Forward**
   5'- GAGTTTCCTTAGCAGAGCTGGAGGTGAAGCGAAGTGCACACGGGTCTAAACTAACGTGTGCA -3' (SEQ ID NO: 42)
**Pre-miR-122/5 Reverse**
   5'- GGATTGCCTAGCAGTAGCTAGGTGTGAAGCTAAGTGCACACGTTAGTTTAGACCCGTGTGCA-3' (SEQ ID NO: 43)

The primer extended products were subjected to agarose gel electrophoresis (1% gel), excised and extracted from the gel slice using Qiagen's MinElute™ Gel Extraction Kit (Qiagen, Germany). Approximately 100 ng of purified pre-miR-31/5 and pre-miR-122/5 was used as template and amplified with forward and reverse pri-miR-31 and pri-miR-122 primers.
**Pri-miR-31 Forward**
   5'- GCTAGCCATAACAACGAAGAGGGATGGTATTGCTCCTGTAACTCGGAACTGGAGAGG -3' (SEQ ID NO: 44)
**Pri-miR-31 Reverse**
   5'- AAAAAAACTAGTAAGACAAGGAGGAACAGGACGGAGGTAGCCAAGCTGCTGTCAGACAGGAAGC -3' (SEQ ID NO: 45)
**Pri-miR-122 Forward**
   5'- GACTGCTAGCTGGAGGTGAAGTTAACAGCTTCGTGGCTACAGAGTTTCCTTAGCAGAGCTG -3' (SEQ ID NO: 46)
**Pri-miR-122 Reverse**
   5'- GATCACTAGTAAAAAAGCAAACGATGCCAAGACATTTATCGAGGGAAGGATTGCCTAGCAGTAGCTA -3' (SEQ ID NO: 47)

*Amplification of U6 Pol III promoter sequences.* The U6 promoter was also amplified with the following primers: U6 F 5'- GAT CAG ATC TGG TCG GGC AGG AAG AGG GCC -3' (SEQ ID NO: 48) and U6 R 5'- GCT AGC GGT GTT TCG TCC TTT CCA CA -3' (SEQ ID NO: 49). Thermal cycling parameters were as before. Amplicons were subjected to agarose gel electrophoresis (1% gel), excised and purified from the gel slice using the MinElute™ Gel Extraction kit from Qiagen.

*Propagation of miR and U6 sequences.* The DNA fragments encoding pri-miR-31/5, pri-miR-122/5 (SEQ ID NOS: 3 and 4) and the U6 promoter (SEQ ID NO: 5) were ligated into pTZ-57R/T (InsTAclone™ PCR Cloning Kit, Fermentas, Hanover, MD, USA) to generate pTZ-57R/T pri-miR-31/5 and pTZ-57R/T pri-miR-122/5 respectively. pTZ-U6 was generated similarly by insertion of the amplified U6 Pol III promoter sequence into pTZ. Ligation and selection were carried out according to the manufacturer's instructions. Briefly, the ligation reactions were incubated at 22°C for 2-3 hours. Chemically competent *E*. *coli* were transformed with the ligation mix, plated on ampicillin, IPTG, X-gal positive LB agar plates and the plates incubated at 37°C overnight. Clones positive for insert and in the reverse orientation (relative to the *ß-galactosidase* gene) were sequenced according to standard dideoxy chain termination protocols (Inqaba Biotechnology, South Africa).

*Anti HBV miR expression cassettes.* To generate Pol III-driven pri-miR vectors the sequences encoding pri-miR-31/5 and pri-miR-122/5 were cloned downstream of the U6 promoter. To generate U6 pri-miR-31/5, pri-miR-31/5 was excised from pTZ-57R/T pri-miR-31/5 by digesting with *Nhe* I and *Sca* I and inserted into pTZ-U6 which had been digested with *Spe* I and *Sca* I. U6 pri-miR-122/5 was generated by excising pri-miR-122/5 from pTZ-57R/T pri-miR-122/5 with *Nhe* I and *Eco*RI and inserting it into pTZ-U6 which had been digested with *Spe* I and *Sea* I. The generation of pHBx shRNA 5 has been previously described [39] and involved a two step PCR reaction in which the U6 Pol III promoter was amplified together with downstream hairpin-encoding sequence and then inserted into pTZ-57R/T (InsTAclone™ PCR Cloning Kit, Fermentas, WI, USA).

Pol II-driven pri-miR vectors were generated by cloning pri-miR-31/5 and pri-miR-122/5 sequences downstream of the CMV promoter of pCl-neo (Promega, WI, USA). CMV pri-miR-31/5 was generated by excising pri-miR-31/5 from pTZ-57R/T pri-miR-31/5 with *Sal* I and *Nhe* I and ligating it into pCl-neo which had been digested with *Xho* I and *Xba* I. CMV pri-miR-122/5 was generated by excising pri-miR-122/5 from pTZ-57R/T pri-miR-122/5 with *Nhe* I and *Xba* I digestion followed by ligation into pCl-neo which had been digested with the same restriction enzymes.

Expression cassettes targeting different sites within the *HBx* open reading frame (sites 8 and 9) were generated according to similar procedures. The HBV sequence (SEQ ID NO: 6, ACCESSION AY233296) co-ordinates targeted by each of these cassettes were as follows: pTZ-57R/T pri-miR-31/8 targeting HBV 1678-1698 (SEQ ID NO: 7 (137)), pTZ-57R/T pri-miR-31/9 targeting HBV 1774-1794 (SEQ ID NO: 8 (138), pTZ-57R/T pri-miR-122/6 targeting HBV 1678-1700 (SEQ ID NO: 9 (139)) and pTZ-57R/T pri-miR-122/10 targeting HBV 1774-1796 (SEQ ID NO: 10 (140)).

### Example 2: Intracellular expression of anti HBV sequences derived from pTZ-57R/T pri-miR-31/5 and pTZ-57R/T pri-miR-122/5 in transfected cultured cells.

*Transfection of cultured cells with plasmids encoding miR-31*/*5 and miR122*/*5.* HEK293 cells were propagated in DMEM supplemented with 10% FCS, penicillin (50 IU/ml) and streptomycin (50 µg/ml) (Gibco BRL, UK). On the day prior to transfection, 1 500 000 HEK293 cells were seeded in dishes of 10 cm diameter. Transfection was carried out with 10 µg of shRNA- or miR-expressing plasmid using Lipofectamine (Invitrogen, CA, USA) according to the manufacturer's instructions.

*Northern blot analysis.* HEK293 cells were harvested 4 days after transfection and total RNA was extracted using Tri Reagent (Sigma, MI, USA) according to the manufacturer's instructions. Twenty µg of RNA was resolved on urea denaturing 12. 5% polyacrylamide gels and blotted onto nylon membranes. Radioactively labelled DNA oligonucleotides were run alongside the cellular RNA and used as size indicators. Blots were hybridised to a probe that was designed to be specific to the putative mature miR-31/5 and miR-122/5 products. The sequence of this probe oligonucleotide was: 5' GACTCCCCGTCTGTGCCTTCTCA 3' (SEQ ID NO: 50). To verify equal loading of the lanes with cellular RNA, the blots were stripped and reprobed with an oligonucleotide complementary to endogenous U6 snRNA. The sequence of the U6 snRNA probe was: 5' TAGTATATGTGCTGCCGAAGCGAGCA 3' (SEQ ID NO: 51). All probes were radioactively labelled according to standard procedures using polynucleotide kinase and γ-³²P ATP.

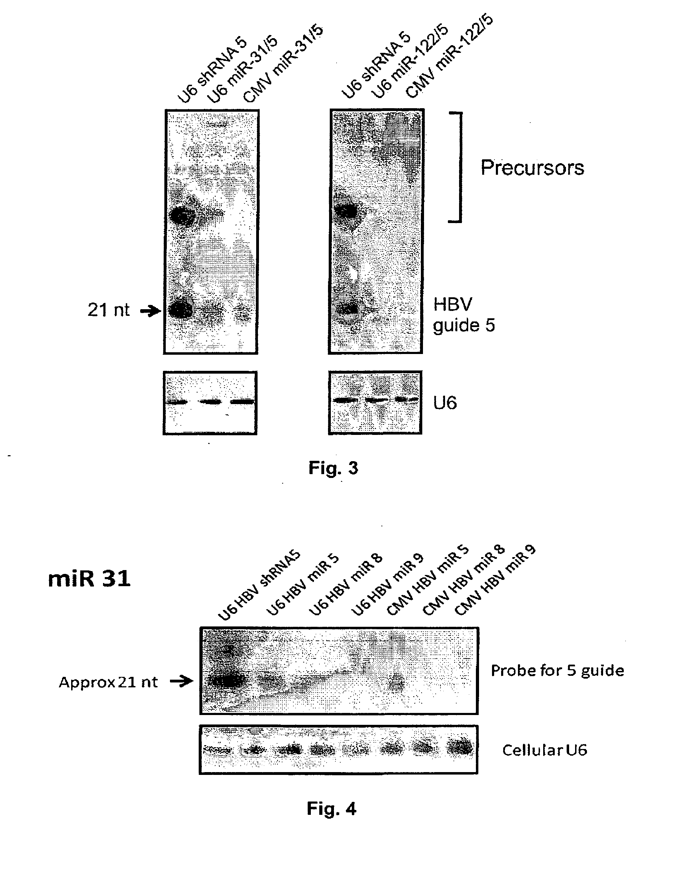

*Detection of processed sequences.* Figures 3, 4 and 5 show the hybridisation signals obtained after northern blot analysis of RNA extracted from cells that had been transfected with miR-31 (Figs. 3 and 4) and miR-122 (Figs. 3 and 5) derived anti HBV expression cassettes. The dominant processed product was detectable as a band of approximately 21 nt in size, which is a similar length to naturally occurring mature miR-31 and miR-122 products [40, 41]. The U6 shRNA HBV shRNA5 expression cassette was included as a positive control of high level hairpin expression. Compared to the shRNA5 expression vector, the amount of the processed guide miR is present in considerably lower concentration (up to 85-fold lower concentration) when expressed in the contexts of the miR-31 and miR-122 vectors. Interestingly, bands corresponding to RNA of 20 and 22 nt in length were also detected in cells transfected with CMV miR-31/5 and U6 miR-31/5 (Fig. 3), which implies that processing of anti HBV guide strands in the context of the miR-31 shuttle may be heterogenous. Larger molecular weight miR/shRNA intermediates were detected in RNA extracted from cells transfected with U6 promoter-containing vectors but not from cells expressing the CMV miR-31/5 or CMV miR-122/5 cassettes. This suggests that complete processing of the CMV Pol II transcripts occurs more efficiently than that of the Pol III-expressed RNA. Interestingly, intracellular concentrations of miR-derived guides from U6 cassettes were lower than for U6 shRNA 5 and may be a result of lower Pol III transcription efficiency of the longer miR-122/5 and miR-31/5 sequences. The detected guide strand signal was specific as no bands were detectable when the probe was hybridized to RNA that had been extracted from cells transfected with similar pri-miR expression vectors that target different HBV sites (Figs. 4 and 5). Equal loading of the cellular RNA onto each of the lanes was confirmed by similar signal intensity of the U6 snRNA probe.

### Example 3: Testing of anti HBV efficacy of miR sequences in cell culture models of HBV replication

*Target plasmids*. pCH-9/3091 has been described previously [42]. It contains a greater than genome length HBV sequence, which is similar to the HBV A1 subgenotype consensus, and generates a 3.5 kb HBV pregenomic transcript from its CMV promoter. By replacing the preS2/S ORF of pCH-9/3091 with Firefly luciferase-encoding DNA, the pCH Firefly Luc vector was prepared similarly to the previously described procedure employed to generate pCH GFP [43]. Briefly, a *Firefly luciferase* sequence was amplified from psiCheck2 (Promega, WI, USA) using PCR. The primer combination to amplify the region encoding *Firefly luciferase* was:
5'ACTGCTCGAGGATTGGGGACCCTGCGCTGAACATGGTGAGCAAGGGCG3' (forward; SEQ ID NO: 52); and 5'ACGTTCTAGAGTATACGGACCGTTACTTGTACAGCTC3' (reverse; SEQ ID NO: 53).

The forward primer comprised sequences complementary to HBV sequences from coordinates 129-159 (including a naturally occurring *Xho*I restriction site) and 5' *Firefly luciferase* sequences. In this primer, the position of the Firefly luciferase initiation codon is equivalent to that of the translation initiation codon of the middle HBs protein. The reverse primer included sequences complementary to the 3' end of the *Firefly luciferase* ORF as well as a *Spe*I restriction site. The PCR primer sequences were as follows:
Luciferase Forward: 5'- ACTG**CTCGAG**GATTGGGGACCCTGCGCTGAACATGGAAG -3' (SEQ ID NO: 54); and
Luciferase Reverse: 5'- ACTG**ACTAGT**TTACACGGCGATCTTTCC -3'(SEQ ID NO: 55)

*XhoI* and *SpeI* sites incorporated by the primers are indicated in bold. The PCR product was cloned into pTZ-57R/T (InsTAclone™ PCR Cloning Kit, Fermentas, WI, USA). The *Firefly luciferase* sequence was then excised from pCI-EGFP with *Xho*I and *Spe*I and inserted into the *XhoI* and *SpeI* sites of pCH-9/3091 to generate pCH-Firefly Luc. The psiCheck-*HBx* target plasmid was prepared by directed insertion of the *Xho*I-*Not* I digested *HBx* fragment from pCI-neo *HBx* [44] into the plasmid psiCheck2 (Promega, WI, USA) such that the *HBx* ORF is within the 3' untranslated region (UTR) of the *Renilla* Luciferase cassette.

*Cell culture.* Huh7 cells were maintained in RPMI medium supplemented with 2. 5% fetal calf serum (FCS), penicillin (50 IU/ml) and streptomycin (50 µg/ml) (Gibco BRL, UK). HEK293 cells were propagated in DMEM supplemented with 10% FCS, penicillin (50 IU/ml) and streptomycin (50 µg/ml) (Gibco BRL, UK). On the day prior to transfection, 250 000 HEK293 cells or 150 000 Huh7 cells were seeded in wells of 2 cm diameter. Transfection was carried out using Lipofectamine (Invitrogen, CA, USA) according to the manufacturer's instructions. To determine effects of miR-31/5 and miR122/5-encoding plasmids, Huh7 cells were transfected with a combination of 6 µg of pCH-9/3091 [42] or pCH Firefly Luc target vector and 2 µg of miR-31/5 and miR122/5 pTZ-derived plasmid or plasmid lacking the miR cassettes. In the case of transfections with the pCH Firefly Luc target vector, a plasmid that constitutively produces *Renilla* luciferase under control of the CMV promoter was included to control for transfection efficiency (pCMV-Ren vector, which was a gift from Dr John Rossi, City of Hope Hospital, Duarte, CA USA). HBV surface antigen (HBsAg) secretion into the culture supernatants was measured using the Monolisa (ELISA) immunoassay kit (BioRad, CA, USA). A plasmid vector that constitutively produces EGFP [43] was also included in each cotransfection and equivalent transfection efficiencies were verified by fluorescence microscopy. The activities of *Renilla* and firefly luciferase were measured with the dual luciferase assay kit (Promega, WI, USA) and using the Veritas dual injection luminometer (Turner BioSystems, CA, USA).

*miR-mediated inhibition of HBV s antigen (HBsAg) secretion from transfected cells.* Initially, to assess efficacy against HBV *in vitro,* Huh7 cells were cotransfected with miR-31/5- and miR-122/5-expressing vectors together with the pCH-9/3091 HBV target plasmid [42]. The *HBx* sequence is common to all HBV transcripts (Fig. 6A) and inhibition of HBsAg secretion correlates with RNAi-mediated silencing of HBV replication [39, 43, 44]. Controls included a U6 shRNA-encoding plasmid (U6 shRNA 5), which was previously shown to be effective against HBV [39] and also a vector in which the CMV promoter controlled expression of the shRNA5 sequence. Compared to mock treated cells, knockdown of 95-98% of viral antigen secretion was achieved by U6 shRNA 5, miR-31/5- and miR-122/5-expressing vectors (Fig. 6B). This effect was observed in both U6 Pol III and also CMV Pol II miR-31/5- and miR-122/5-expressing vectors. CMV miR-122/5 was slightly less effective than the other miR vectors (85-90% knockdown). The vector encoding shRNA 5 derived from the CMV promoter effected least efficient silencing of approximately 60%.

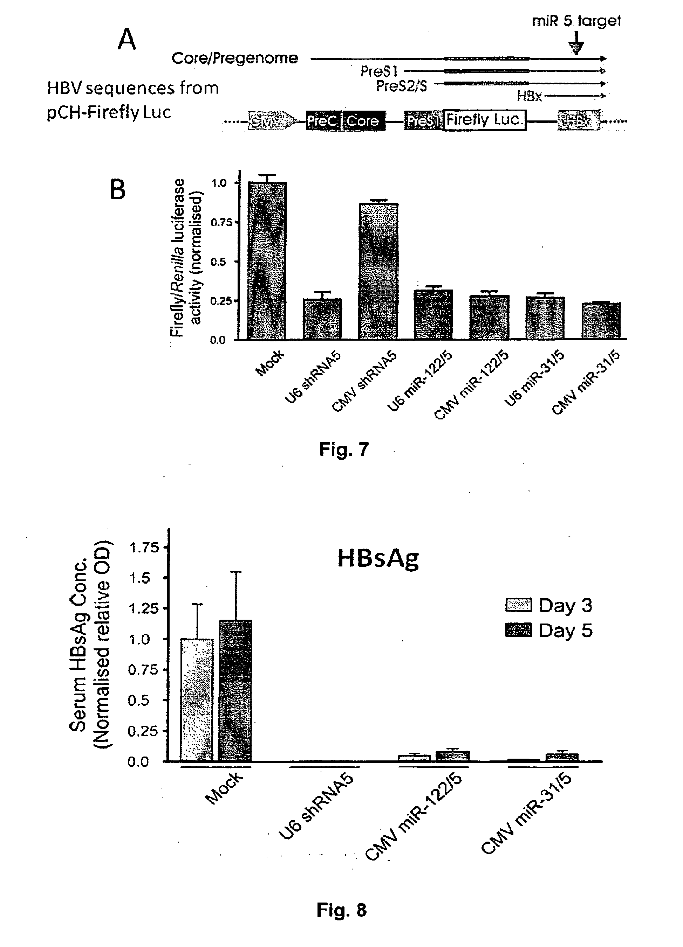

*miR-mediated inhibition of Firefly luciferase activity in transfected cells.* The data derived from analysis of HBsAg secretion of transfected cells (Fig. 6) were corroborated using a reporter gene plasmid (pCH Firefly Luc) to measure knockdown efficiency *in situ* (Fig. 7). In pCH Firefly Luc, the preS2/S sequence of pCH-9/3091 was replaced with the *Firefly Luciferase* ORF, with the targeted *HBx* ORF remaining intact. Cotransfection of pCH Firefly Luc with miR-encoding vectors allows for the convenient quantitative measurement of anti HBV sequences *in situ* by determination of luciferase reported gene activity. Analysis showed that the Firefly luciferase activity was diminished significantly by U6 shRNA 5, U6 miR-31/5, U6 miR-122/5, CMV miR-31/5 and CMV miR-122/5-containing vectors. Each of these vectors effected knockdown of approximately 75% compared to controls (Fig. 7B). The CMV shRNA 5 vector did not inhibit Firefly luciferase activity significantly. Taken together with the results shown in Figure 6, these data indicate that the incorporation of miR-like structure of miR-31 or miR-122 enables expression of the silencing sequence from a Pol II or Pol III promoter without compromising silencing efficacy. Moreover, the data presented in Figures 3, 4 and 5 show that the silencing efficacy is caused by a much reduced concentration of the RNAi effecter. A total of 23 target sites (SEQ ID NOs: 11, 12, 13, 14, 15, 16, 17, 18, 19, 20, 21, 22, 23, 24, 25, 26, 27, 28, 29, 30, 31, 32 and 33 (RNA SEQ ID NOs: 141-163)) were assessed. These were sequences of 21 to 25 nt in length that started from HBV nucleotide coordinates 1575, 1581, 1678, 1774, 59, 62, 220, 228, 239, 251, 423, 1261, 1774, 1826, 1868, 1899, 2312, 2329, 2393, 2456, 2458, 158 and 332. The identification of HBV target sites was determined by assessing conservation across genotypes and also by assessing suitability for RNAi-mediating silencing according to the algorithm described by Heale et al [45].

### Example 4: Testing of anti HBV efficacy of miR sequences in vivo using the hydrodynamic injection model of HBV replication

*Hydrodynamic injection of mice.* The murine hydrodynamic tail vein injection (HDI) method was employed to determine the effects of miR plasmid vectors on the expression of HBV genes *in vivo*. Experiments on animals were carried out in accordance with protocols approved by the University of the Witwatersrand Animal Ethics Screening Committee. A saline solution comprising 10% of the mouse's body mass was injected via the tail vein over 5-10 seconds. This saline solution included a combination of three plasmid vectors: 5 µg target DNA (pH-9/3091); 5 µg pCl-neo plasmid DNA (Promega WI, USA) that lacks HBV sequences or 5 µg anti HBV sequence (p U6 shRNA 5, pCMV miR-31/5 or pCMV miR-122/5 plasmid); and 5 µg pCI neo EGFP (a control for hepatic DNA delivery, which constitutively expresses the enhanced Green Fluorescent Protein (eGFP) marker gene [43]). After aborting investigations on animals where injections were suboptimal, each experimental group comprised 5-8 mice. Blood was collected under anaesthesia by retroorbital puncture on days 3 and 5 after HDI. Serum HBsAg concentration was measured using the Monolisa (ELISA) immunoassay kit (BioRad, CA, USA) according to the manufacturer's instructions. To measure effects of miR shuttle sequences on circulating viral particle equivalents (VPEs), total DNA was isolated from 50 µl of the serum of mice on days 3 and 5 after HDI and viral DNA determined using quantitative PCR according to previously described methods [39]. Briefly, total DNA was isolated from 50 µl of mouse serum using the Total Nucleic Acid Isolation Kit and MagNApure instrument from Roche Diagnostics. Controls included water blanks and HBV negative serum. DNA extracted from the equivalent of 8 µl of mouse serum was amplified using SYBR green Taq readymix (Sigma, MO, USA). Crossing point analysis was used to measure virion DNA concentrations and standard curves were generated using EuroHep calibrators [45]. The HBV surface primer set was: HBV surface forward: 5'-TGCACCTGTATTCC ATC-3' (SEQ ID NO: 56) and HBV surface reverse: 5'-CTGAAAGCCAAACAGTGG-3' (SEQ ID NO: 57). PCR was carried out using the Roche Lightcycler V. 2. Capillary reaction volume was 20 µl and thermal cycling parameters consisted of a hot start for 30 sec at 95°C followed by 50 cycles of 57°C for 10 sec, 72°C for 7 sec and then 95°C for 5 sec. Specificity of the PCR products was verified by melting curve analysis and agarose gel electrophoresis.

The livers were harvested after sacrificing mice at 5 days after HDI. Total DNA was extracted from the liver according to standard procedures [46]. DNA was subjected to standard agarose gel electrophoresis without restriction digestion before processing for Southern blot analysis using Rapidhyb buffer (Amersham, UK). To generate a probe, HBV X open reading frame DNA was amplified with the following probes: HBx Forward: 5'- GATCAAGCTTTCGCCAACTTACAAGGCCTTT -3' (SEQ ID NO: 58) and HBx Reverse: 5'- GATCTCTAGAACAGTAGCTCCAAATTCTTTA -3' (SEQ ID NO: 59). PCR products were purified and used as template for random-primed labelling with the HexaLabel™ DNA Labelling kit (Fermentas, MD, USA) according to the manufacturer's instructions. Fixed frozen liver sections were processed for detection of eGFP according to standard procedures.

Figure 8 shows the concentrations of HBsAg detected in the serum of mice that had been subjected to the HDI procedure with the indicated plasmids. p U6 shRNA 5, pCMV miR-31/5 and pCMV miR-122/5 plasmids each effected knockdown of the viral antigen by at least 95%. This was observed when measurements were taken at both 3 days and 5 days after HDI. Of the 3 plasmid vectors, that containing U6 shRNA 5 was the most efficient and HBsAg concentration in the serum of mice injected with this plasmid was not detectable. The number of circulating VPEs in the same mice were also measured using quantitative real time PCR at days 3 and 5. These data are shown in Figure 9. The results corroborate HBsAg determinations (Fig. 8) in that p U6 shRNA 5, pCMV miR-31/5 and pCMV miR-122/5 plasmids each effected efficient knockdown of the number of circulating VPEs by at least 95%. At days 3 and 5, the number of VPEs was approximately 1.3 x 10⁶ and 2.1 x 10⁶ per ml of serum respectively in the mock treated animals. The circulating VPEs in p U6 shRNA 5, pCMV miR-31/5 and pCMV miR-122/5 treated animals was approximately 10-fold lower and ranged from 0. 5-2. 5 x 10⁵ per ml of serum p U6 shRNA 5 and pCMV miR-31/5 had approximately equal efficacy in knocking down this marker of replication and both these vectors were slightly more efficient than pCMV miR-122/5.

The HBV DNA replication intermediates were measured in representative liver tissue of 2 animals from each group that had been subjected to HDI experimentation. The results of this analysis are shown in Figure 10. HBV duplex linear (DL) and relaxed circular (RC) replication intermediates were only detectable in the mock treated animals, but not in any of the mice that had been coinjected with CMV miR-31/5, CMV miR-122/5 or U6 shRNA 5 plasmids. The HBV DNA bands detected in the mock treated mice did not correspond in size to any of the pCH 9/3091 bands, which indicates that the DL and RC HBV DNA that were detected were not the same as input plasmid DNA. Staining of the separated DNA with ethidium bromide prior to Southern transfer and hybridisation verified that the amount of cellular DNA that was loaded onto each of the lanes was equivalent. Unequal loading of the lanes thus did not account for the differences in the concentrations of HBV DNA replication intermediates that were observed. Collectively, the data from Figs 8-10 show that CMV miR-31/5 and CMV miR-122/5 expression cassettes are highly efficient silencers of HBV gene expression, and their efficacy is approximately as good as that of the U6 shRNA 5-containing vector.

### Example 5: Assessing induction of non specific induction of interferon response genes by anti HBV miR shuttles

*Cell culture, transfection and RNA extraction.* HEK293 cells were cultured and transfected as described above. Briefly, cells were maintained in DMEM supplemented with 10% FCS, penicillin (50 IU/ml) and streptomycin (50 µg/ml) (Gibco BRL, UK). On the day prior to transfection, 250 000 HEK293 cells were seeded in dishes of 2 cm diameter. Transfection was carried out with 800 ng of shRNA- or miR-expressing plasmid using Lipofectamine (Invitrogen, CA, USA) according to the manufacturer's instructions. As a positive control for the induction of the IFN response, cells were also transfected with 800 ng poly (I:C) (Sigma, MI, USA). Two days after transfection, RNA was extracted with Tri Reagent (Sigma, MI, USA) according to the manufacturer's instructions.

*Real time quantitative PCR of interferon response genes.* To amplify *oligoadenylate synthase-1 (OAS-1), interferon-β(IFN-β), p56* and *glyceraldehydes-3 phosphate dehydrogenase (GAPDH)* cDNA, the procedures described by Song et al [47] were used. All qPCRs were carried out using the Roche Lightcycler V. 2. Controls included water blanks and RNA extracts that were not subjected to reverse transcription. Taq readymix with SYBR green (Sigma, MO, USA) was used to amplify and detect DNA during the reaction. Thermal cycling parameters consisted of a hotstart for 30 sec at 95°C followed by 50 cycles of 58°C for 10 sec, 72°C for 7 sec and then 95°C for 5 sec. Specificity of the PCR products was verified by melting curve analysis and agarose gel electrophoresis. The primer combinations used to amplify IFN response-related mRNA of human HEK293 cells were as follows:
IFN-β Forward: 5' TCCAAATTGCTCTCCTGTTGTGCT 3',
IFN-β Reverse: 5' CCACAGGAGCTTCTGACACTGAAAA 3',
GAPDH Forward: 5' AGGGGTCATTGATGGCAACAATATCCA 3',
GAPDH Reverse: 5' TTTACCAGAGTTAAAAGCAGCCCTGGTG 3',
OAS1 Forward: 5' CGAGGGAGCATGAAAACACATTT 3',
OAS1 Reverse: 5' GCAGAGTTGCTGGTAGTTTATGAC 3',
p56 Forward: 5'- CCCTGAAGCTTCAGGATGAAGG -3' and
p56 Reverse: 5'- AGAAGTGGGTGTTTCCTGCAAG -3'.

Figure 11 shows a comparison of the concentration ratio of *OAS-1*, *p56* and *IFN*-β genes to GAPDH, which is a housekeeping gene. Expression of IFN genes was increased at 24 hours after treatment of cells with poly (I:C), which confirms activation of the IFN response under the experimental conditions used. Induction of *IFN-β* mRNA was not observed with RNA extracted from cells that had been transfected with p U6 shRNA 5, p CMV miR-31/5, p CMV miR-122/5, p U6 miR-31/5 or p U6 miR-122/5 vectors. These data indicate that the silencing effect of miR expression cassettes on HBV markers of replication is unlikely to be caused by non specific induction of the interferon response and resultant programmed cell death (apoptosis).

### Example 6: Assessing attenuation of independent RNAi-mediated silencing by anti HBV miR shuttles

*Cell culture, transfection and RNA extraction.* HEK293 and Huh7 cells were cultured and transfected as described above. On the day prior to transfection, 250 000 HEK293 cells were seeded in dishes of 2 cm diameter. To assess effects of miR-31/5- and miR-122/5-expressing plasmids on independent RNAi-mediated silencing, cells were seeded into 24-well dishes at a density of 35-40% then transfected with 80 ng of psi-CHECK-8T, 40 ng of pCMV miR-31/8 and 780 ng of shRNA 5 or miR 5 expression plasmids. Plasmid dose effects of pU6 shRNA 5 on independent silencing by pCMV miR-31/8 was determined by transfecting pU6 shRNA 5 in a range from 0 to 10 µg. Similarly, to determine silencing potency of pU6 shRNA 5 against pCH-9/3091, pU6 shRNA 5 was transfected in a range from 0 to 10 µg. A constant amount of 1 µg of pCMV miR-31/8 and pCH-9/3091 was transfected in each well. Backbone plasmid was included in each case to ensure that equal amounts of total plasmid DNA was transfected. A plasmid vector that constitutively produces eGFP [43] was also included in each cotransfection to *verify equivalent* transfection efficiencies using fluorescence microscopy. Transfection was carried out with 800 ng of shRNA- or miR-expressing plasmid using Lipofectamine (Invitrogen, CA, USA) according to the manufacturer's instructions. To generate psi-CHECK-8T, which contained the miR 8 target, primer 8T forward 5'- CAA TGT CAA CGA CCG ACC TT -3' and primer 8T reverse 5'- ACT AGT GCC TCA AGG TCG GT -3' were used to amplify nucleotides 1678 to 1702 of the HBV genome and introduce a *Spe* I site at the 3' end of the amplicon. Purified fragment was ligated into the pTZ-57R/T PCR cloning vector and the insert was removed with *Sal* I and Spe I and ligated into the *Xho* I and *Spe* I sites of psi-CHECK 2. 2 (Promega, WI, USA) to generate psi-CHECK-8T with the HBV target site downstream of the Renilla *luciferase* ORF. All plasmid sequences were verified according to standard dideoxy chain termination protocols (Inqaba Biotechnology, South Africa).

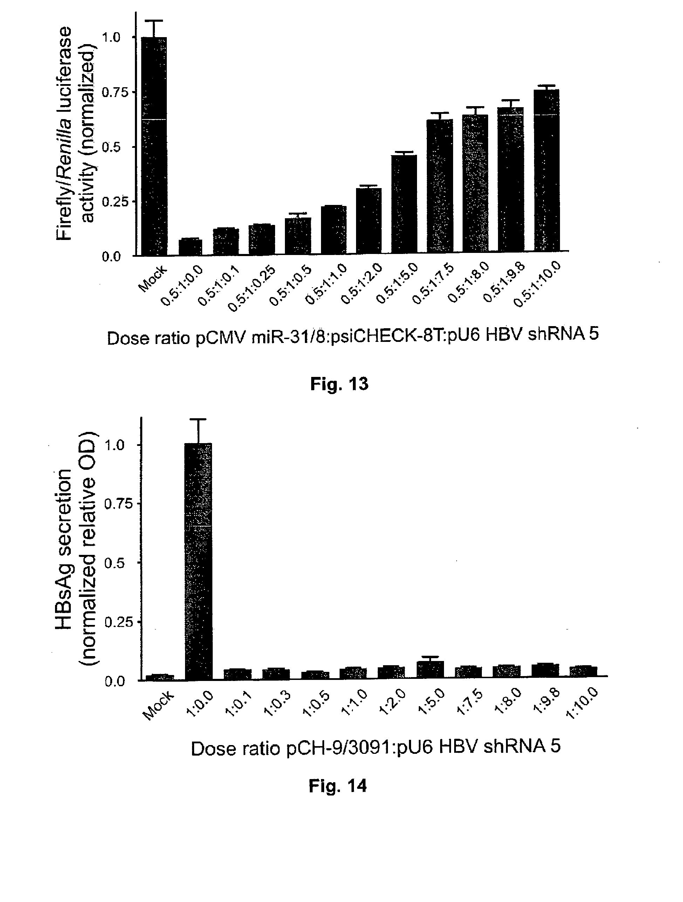

*Assessment of miR shuttle effects on independent RNAi-mediated silencing.* To determine the effect of miR-expressing vectors on independent RNAi-mediated gene silencing, a dual luciferase reporter plasmid (psi-CHECK-8T) containing an independent HBV miR-31/8 target sequence downstream of the *Renilla* luciferase ORF was transfected together with pCMV miR-31/8 and each of the shRNA 5-, miR-31/5- or miR-122/5-expressing vectors (Fig. 12). In accordance with previous observations that overexpression of shRNA from U6 Pol III promoter causes disruption of the endogenous miR pathway [36], the silencing of psi-CHECK-8T target by pCMV miR-31/8 was diminished in the presence of pU6 HBV shRNA 5. This effect was however not observed when miR-122/5- or miR-31/5-expressing plasmids were cotransfected. These consequences are likely to be dependent on RNAi effecter concentration, which is in keeping with our finding that the intracellular pri miR-derived guide sequences are present at lower concentrations than U6 shRNA 5 guides (Figs. 3, 4 and 5). To corroborate this hypothesis, decreasing concentrations of pU6 HBV shRNA5 plasmid were cotransfected with constant amounts of CMV miR-31/8 and psi-CHECK-8T target (Fig. 13). Efficient miR-31/8-mediated knockdown was achieved at low concentrations of pU6 HBV shRNA5. However, when the amount of pU6 HBVshRNA5 was increased, the efficacy against HBV target 8 was diminished. Cotransfecting a similar range of pU6 HBV shRNA5 concentrations with pCH-9/3091 HBV replication competent plasmid confirmed that potent silencing of HBsAg secretion is achieved by the HBV target 5 (Fig. 14). These data further support the notion that disruption by pU6 HBV shRNA5 of independent pCMV miR-31/8 silencing is influenced by the concentration of expressed shRNA5. Importantly, no disruption if independent silencing was observed when cotransfecting cells with the anti HBV miR shuttle expression cassettes.

### Example 7: Assessment of in vivo toxicity and disruption of independent RNAi-mediated silencing by anti HBV miR shuttles

*Hydrodynamic injection of mice and measurement of hepatic luciferase activity.* To determine effects of miR on reporter gene activity *in vivo*, BALB/c mice received 0. 5 µg reporter target DNA (psi-CHECK-8T), 5 µg pCH-9/3091, combinations of anti HBV plasmids (pCMV miR-31/8, pU6 shRNA 5, pCMV miR-31/5 or pCMV miR-122/5) or mock (pCI-neo backbone). Mice were sacrificed 3 days after HDI, their livers harvested, homogenized in phosphate buffered saline and activities of *Renilla* and Firefly luciferase were determined as described above.

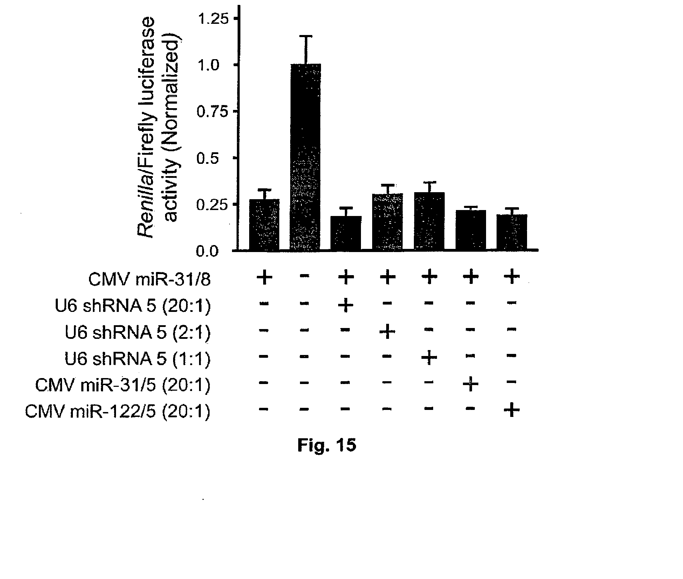

*Assessment of toxicity and disruption of independent RNAi-mediated silencing.* To assess possible disruption of independent RNAi-mediated silencing, as well as toxicity *in vivo* caused to hepatocytes by pri-miR shuttles, mice were also injected with the psi-CHECK-8T dual luciferase reporter plasmid and various anti HBV expression cassettes (Fig. 15). Firefly and *Renilla* luciferase activities in liver homogenates were measured 3 days after HDI. Selective and efficient silencing of *Renilla* luciferase activity was achieved with pCMV miR-31/8. This knockdown was not attenuated by coinjection of 20-fold excess of U6 shRNA 5, CMV miR-122/5 or CMV miR-31/5, which indicates that under the experimental conditions described here, independent silencing was unaffected by miR shuttle expression. Using the HDI model, direct assessment of hepatotoxicity caused by miR mimics is complicated by damage to liver cells with release of enzyme markers that is inherent to the injection procedure itself. As a surrogate indicator of damage to liver cells caused by pri miR shuttles, untargeted and constitutively active psi-CHECK-8T-derived Firefly luciferase activity was independently evaluated in the groups of mice. Compared to the animals receiving no RNAi effecter, no diminished Firefly luciferase activity in liver homogenates was observed in those mice receiving the miR shuttles. Collectively, these data show that miR mimics generated from CMV miR-31/5 and CMV miR-122/5 are specific silencers of HBV replication *in vivo* with negligible effects on independent RNAi-mediated silencing. Moreover, efficacy of the miR expression cassettes is approximately as good as that of the U6 shRNA 5 sequences.

### Example 8: Characterisation of cassettes expressing miRNA shuttles from liver-specific promoters

The human Alpha-1-antitrypsin (A1AT) and Factor VIII (FVIII) promoters were amplified from total genomic DNA extracted from Huh7 cells using the primer sets in Table 1. The HBV Basic Core Promoter (BCP) and PreS2 promoter were amplified from the plasmid pCH-9/3091 using the primer sets in Table 1. All oligonucleotides were synthesised by standard phosphoramidite chemistry (Inqaba Biotechnology, South Africa). Primers were designed such that amplification introduced a *Bgl*II (*Bcl*I in the case of A1AT) site at the 5' end and a *Hind*III site at the 3' end of the amplicons. Promoter sequences were amplified using the Expand High Fidelity PCR^{PLUS} System (Roche, Germany) according to manufacturer's instructions. PCRs were set up in 50 µl reaction mixtures and contained 5× Expand HiFi^{PLUS} Buffer (with MgCl₂), 0. 2 mM dNTPs (dGTP, dATP, dTTP and dCTP), 0. 5 µM of respective forward and reverse primers, 2. 5 U Expand HiFi^{PLUS} Enzyme Blend and 50 ng of genomic DNA of 100 pg plasmid DNA. The PCR conditions were as follows: Initial denaturation at 94°C for 2 minutes; 30 cycles of denaturation at 94°C for 30 seconds, annealing at 55°C for 30 seconds and extension at 72°C for 3 minutes (during cycles 20-30 extension time increased by 10 seconds every cycle); and a final extension at 72°C for 7 minutes.

**Table 1: Oligonucleotide sequences for amplification of liver-specific promoters**

| | |
|---|---|
| A1AT F | 5'- GATCT**GATCA**TTCCCTGGTCTGAATGTGTG -3' (SEQ ID NO: 70) |
| A1AT R | 5'- GATC**AAGCTT**ACTGTCCCAGGTCAGTGGTG -3' (SEQ ID NO: 71) |
| FVIII F | 5'- GATC**AGATCT**GAGCTCACCATGGCTACATT -3' (SEQ ID NO: 72) |
| FVIII R | 5'- GATC**AAGCTT**GACTTATTGCTACAAATGTTCAAC -3' (SEQ ID NO: 73) |
| BCP F | 5'- GATC**AGATCT**GCATGGAGACCACCGTGAAC -3' (SEQ ID NO: 74) |
| BCP R | 5'- GATC**AAGCTT**CACCCAAGGCACAGCTTGGA -3' (SEQ ID NO: 75) |
| PreS2 F | 5'- GATC**AGATCT**GCCTTCAGAGCAAACACCGC -3' (SEQ ID NO: 76) |
| PreS2 R | 5'- GATC**AAGCTT**ACAGGCCTCTCACTCTGGGA -3' (SEQ ID NO: 77) |

### Restriction sites are indicated in bold.

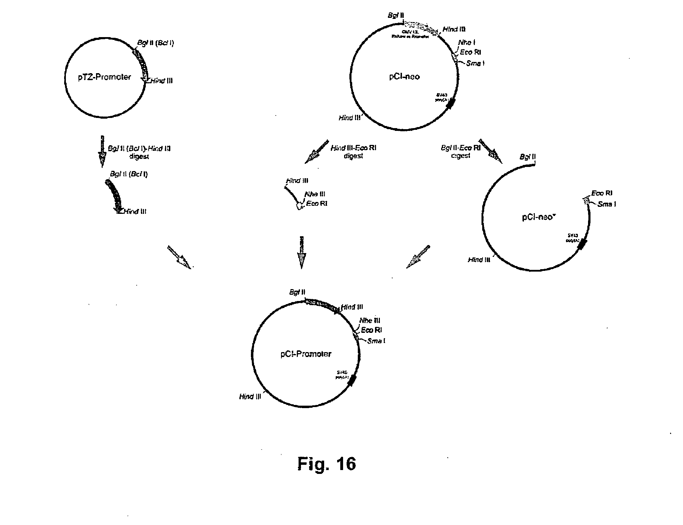

The PCR products were subjected to agarose gel electrophoresis and eluted using the MinElute™ Gel Extraction Kit (Qiagen, Germany). Purified fragments were ligated into the PCR cloning vector pTZ57R/T (InsTAclone PCR Cloning Kit, Fermentas, W I, USA). A 1:3 molar ratio of vector to insert was ligated at 16°C overnight. Chemically competent *E. coli* (XL1-Blue, Invitrogen, CA, USA) were transformed with the ligation mixes and plated on Luria Bertani ampicillin-, X-gal-, and IPTG-containing agar plates then incubated at 37°C overnight. Colonies positive for an insert (white colonies) were selected for plasmid purification. Plasmids were subjected to restriction enzyme digestion and clones yielding desired results were sequenced (Inqaba Biotechnology, South Africa). Next, the CMV immediate early enhancer promoter sequence within pCI-neo was substituted with the sequences of the liver-specific promoters (Figure 16). The new expression vectors created would therefore run off the liver-specific promoters instead of the constitutively active CMV promoter. The sequences encoding the FVIII, BCP and PreS2 promoters were digested out of their respective plasmids (pTZ-FVIII, pTZ-BCP and pTZ-PreS2) with *Bg*/II and *Hind*III restriction. *Bc*/l is sensitive to methylation, therefore pTZ-A1AT was propagated through the *dcm-* and *dam*-methylase deficient strain of *E. coli*, GM2929. The sequence encoding the A1AT promoter was then restricted from pTZ-A1AT with *Bcl*I and *Hind*III. pCI-neo was digested with *Hind*III and EeoRI to yield a 3815 bp, a 1317 bp and a 340 bp fragment. Secondly, pCI-neo was digested with *Eco*RI and *Bg*/II to yield a 4371 bp and a 1101 bp fragment. The liver-specific promoter sequences were ligated with the 340 bp *Hind*III-*Eco*RI and the 4371 bp *Eco*RI-*Bg*/II fragments to generate the new liver-specific expression vectors (pCI-A1AT, pCI-FVIII, pCI-BCP and pCI-PreS2). *Bc*/l and *Bg*/II generated complementary overhangs thus allowing the A1AT promoter sequence to be ligated to pCI-neo backbone.

### Assessing functionality of liver-specific expression vectors

To assess the functionality of the liver-specific expression vectors the sequence encoding Firefly luciferase was cloned downstream of the promoter sequences. The Firefly luciferase sequence was digested out of pCI-neo FLuc with *Nhe*I and *Sma*I and ligated into the equivalent sites of the liver-specific expression vectors to generate pCI-A1AT FLuc, pCI-FVIII FLuc, pCI-BCP FLuc and pCI-PreS2 FLuc.

### Tissue Culture

The human hepatoma cell line, Huh7 and the Human Embryonic Kidney derivatives, 116 cells were maintained in DMEM growth medium (Sigma, MO, USA) supplemented with 10% foetal calf serum (Gibco BRL, UK). One day prior to transfection cells were seeded at a density of 40% into 24-well dishes (Corning Inc., NY, USA).

Cells were transfected with 100 ng of the different Firefly luciferase expression vectors, 100 ng of phRL-CMV and 100 ng of pCI-neo eGFP. The plasmid DNA was mixed with 50 µl of Opti-MEM (Invitrogen, CA, USA) and incubated at room temperature for 5 minutes. An additional 50 µl of Opti-MEM was mixed with 0. 5 µl of Lipofectamine 2000 (Invitrogen, CA, USA) and also incubated for 5 minutes at room temperature. After the incubation period the DNA:Opti-MEM and Lipofectamine:Opti-MEM mixtures were combined and incubated for an additional 20 minutes at room temperature to allow lipid:DNA complexes to form. Following the second incubation period 100 µl of the transfection mix was added per well to the 24-well dish. Transfections were repeated in triplicate. The cells were incubated for 5 hours at 37°C and 5% CO₂. Thereafter the growth medium was replaced with fresh medium and the cell incubated for 48 hours.

### Luciferase Assay

Fourty eight hours post-transfection cells were assay for *in situ* luciferase activity using the Dual Luciferase Assay System (Promega, WI, USA). Briefly, growth medium was removed and the cells lysed with 100 µl of Passive Lysis Buffer with agitation for 15 minutes. Ten microlitres of the cell lysates were dispensed into a luminometer plate and Firefly luciferase and *Renilla* luciferase activities measured using the Veritas Dual Injection Luminometer (Turner BioSystems, CA, USA).

### Generation of liver-specific miRNA shuttle vectors

To generate liver-specific miRNA shuttles vectors the pri-miR-122 shuttles were digested from pCI-miR-122/5, pCI-miR-122/6 and pCI-miR-122/10 with *Nhe*I and *Sma*I and ligated into the equivalent sites of pCI-A1AT, pCI-FVIII, pCI-BCP and pCI-PreS2.

### Assessing functionality of liver-specific miRNA shuttle vectors

Twenty-four hours before transfection, cells were seeded into 24-well dishes at a density of 40%. Cells were transfected with 80 ng of target plasmid (pCH-FLuc), 800 ng of the different miRNA shuttle vectors, 50 ng of phRL-CMV and 50 ng of pCI-neo eGFP. Plasmid DNA was made up to a total of 1 µg with pCI-neo and diluted with 50 µl of Opti-MEM. One microlitre of Lipofectamine 2000 was diluted in 50 µl of Opti-MEM. Transfections and measurement of Firefly and *Renilla* luciferase activities were carried out as described above.

### Constructed expression vectors are capable of tissue-specific expression of Firefly luciferase

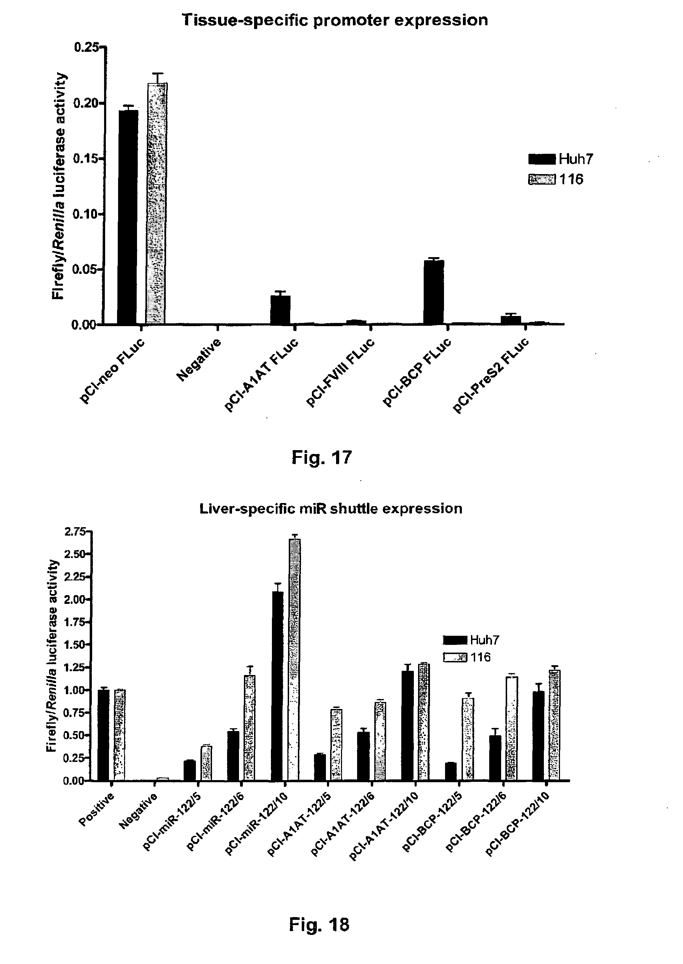

Transfection of cultured mammalian cells with vectors expressing Firefly luciferase allowed for the convenient measurement of luciferase as an indicator of (i) functionality of constructed vectors and (ii) ability of vectors to exhibit tissue-specificity. The promiscuous and constitutively active CMV promoter expressing Firefly luciferase was included as a positive control for expression. Figure 17 illustrates the *in situ* expression of Firefly luciferase activity from the different promoters. The CMV immediate early promoter enhancer is a powerful, constitutively active promoter and as such is expected to strongly express Firefly luciferase in a wide variety of cell types. As expected Firefly luciferase expression in both Huh7 as well as 116 cells transfected with pCI-neo FLuc exhibited high levels of Firefly luciferase activity as compared to cells receiving no Firefly luciferase vector (Negative). None of the liver-specific expression vectors exhibited the same degree of expression in Huh7 cells that was achieved by pCI-neo FLuc. The greatest expression was achieved with the pC-BCP FLuc which was approximately 3-fold less than the expression from the CMV promoter. Expression from pCI-FVIII FLuc, pCI-A1AT FLuc and pCI-PreS2 was approximately 50-, 7-, and 25-fold less than pCI-neo FLuc expression. However, expression of Firefly luciferase from these vectors in 116 cells was significantly decreased.

### Expression vectors are capable of tissue-specific expression of miRNA shuttles

Having demonstrated tissue-specific expression of the vectors the next step was to test whether or not these vectors could silence HBV replication in a tissue-specific manner. The anti HBV pri-miR-122-derived shuttles were cloned downstream of the liver-specific promoters and their ability to inhibit markers of HBV replication selectively in liver-derived cells only was tested. Figure 18 shows knockdown of Firefly luciferase activity (as a measure of HBV replication) by the two vectors which had previously exhibited the best expression levels (*i. e.* pCI-A1AT and pCI-BCP, Fig. 17). In the reporter vector, the HBV target was inserted downstream of the Firefly luciferase open reading frame as described in example 3 and Figure 7. The CMV miRNA shuttles knocked down HBV replication in both Huh7 cells and 116 cells, however silencing of HBV by pCI-A1AT and pCI-BCP miRNA vectors was limited to Huh7 cells. These data demonstrate that the expression vectors are capable of tissue-specific expression of miRNA shuttles. In addition the level of knockdown achieved by the pCI-A1AT and pCI-BCP expression vectors in Huh7 cells was comparable to that achieved by the more powerful CMV expression cassettes.

### Example 9: Design and propagation of multimeric anti HBVpri-miR 31 shuttle plasmids

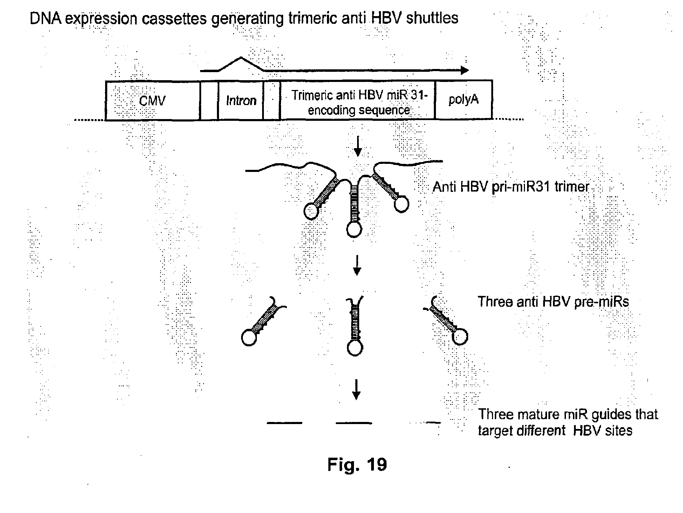

*Rationale.* By generating cassettes that are capable of targeting multiple HBV sites simultaneously, the efficacy of silencing of viral replication should be improved. Moreover, a combination of RNAi effecters that act at different cognate sites of the virus will limit the chances of viral escape and resistance to the inhibitory effects of anti HBV pri-miR shuttles. The schematic illustration of the cassettes that generate the trimeric pri-miR cassettes used here against HBV is shown in Figure 19.

*miR-derived anti HBV sequences.* DNA encoding pre-miR-31 mimics containing the guide sequence targeting HBV coordinates 1775 to 1797 (target 5) (SEQ ID NO: 3 (135)), 1678 to 1700 (target 8) (SEQ ID NO: 7 (137)) and 1574 to 1596 (target 9) (SEQ ID NO: 8 (138)) were generated by primer extension of paired forward and reverse oligonucleotides. Oligodeoxynucleotides encoding the pre-miR sequences were synthesised using phosphoramadite chemistry (Inqaba Biotech, South Africa). Primer extensions were performed as PCR using Promega's PCR Master Mix (Promega, WI, USA). The thermal cycling conditions were as follows: Initial denaturation at 94°C for 5 minutes, followed by 30 cycles of denaturation at 94°C for 10 seconds, annealing at 50°C for 10 seconds and extension at 72°C for 10 seconds and a final extension step at 72°C for 10 minutes. The sequences of the oligonucleotides encoding the anti HBV miR shuttles were:
**Pre-miR-31/5 F**
   5'-GTAACTCGGAACTGGAGAGGCAAGGTCGGTCGTTGACATTGGTTGAACTGGGAA CGACG -3' (SEQ ID NO: 78)
**Pre-miR-31/5 R**
   5'-CTGCTGTCAGACAGGAAAGCCGTGAATCGATGTGCACACGTCGTTCCCAGTTCAA CCCGT -3' (SEQ ID NO: 79)
**Pre-miR-31/8 F**
   5'-GTAACTCGGAACTGGAGAGGCAAGGTCGGTCGTTGACATTGGTTGAACTGGGA ACGAAA -3' (SEQ ID NO: 80)
**Pre-miR-31/8 R** 5'- CTGCTGTCAGACAGGAAAGCTAAGGTTGGTTGTTGACATTTCGTTCCCAGTTCAACCAAT -3' (SEQ ID NO: 81)
**Pre-miR-31/9 F**
   5'- GTAACTCGGAACTGGAGAGGATTTATGCCTACAGCCTCCTAGTTGAACTGGGAA CGAAG -3' (SEQ ID NO: 82)
**Pre-miR-31/9 R**
   5'-CTGCTGTCAGACAGGAAAGCCTTTATTCCTTCAGCCTCCTTCGTTCCCAGTTCAAC TAGG -3' (SEQ ID NO 83)

The primer extended products were subjected to standard agarose gel electrophoresis (1% gel), excised and extracted from the gel slice using Qiagen's MinElute™ Gel Extraction Kit (Qiagen, Germany). Approximately 100 ng of purified amplicons were used as template and amplified with universal forward and reverse pri-miR-31 primers that had the following sequences.
**Pri-miR-31 F** 5'- GCTAGCCATAACAACGAAGAGGGATGGTATTGCTCCTGTAACTCGGAACTGGAGAGG -3' (SEQ ID NO: 84)
**Pri-miR-31 R**
   5'- AAAAAAACTAGTAAGACAAGGAGGAACAGGACGGAGGTAGCCAAGCTGCTGTCAGACAGGAAGC -3' (SEQ ID NO: 85)

The purified DNA fragments were ligated into pTZ-57R/T (InsTAclone™ PCR Cloning Kit, Fermentas, Hanover, MD, USA) to generate pTZ-57R/T pri-miR-31/5 and pTZ-57R/T pri-miR-122/5 respectively. Ligation and selection were carried out according to the manufacturer's instructions. Briefly, the ligation reactions were incubated at 22°C for 2-3 hours. Chemically competent *E. coli* were transformed with the ligation mix, plated on ampicillin, IPTG, X-gal positive LB agar plates and the plates incubated at 37°C overnight. Clones positive for insert and in the reverse orientation (relative to the *ß-galactosidase* gene) were sequenced according to standard dideoxy chain termination protocols (Inqaba Biotechnology, South Afrcia).

Multimeric pri-miR shuttle cassettes expressed from an RNA polymerase II promoter were generated by inserting combinations of pri-miR-31/5, -31/8 and 31/9 sequences downstream of the CMV immediate early promoter enhancer (Figure 19). Cassettes were designed to include single copies all three monomeric pri-miR mimics in all possible combinations of ordering from 5' to 3'. Thus a total of 6 trimeric cassettes was generated (pri-miR-31/5/8/9, -5/9/8, -8/5/9, -8/9/5, -9/5/8 and - 9/8/5). To generate the pri-miR-31/5/8/9 cassette, firstly pri-miR-31/8 was excised from pTZ pri-miR-31/8 with *Nhel* and *Eco*RI and ligated into pTZ pri-miR-31/5 digested with Spel and *Eco*RI to create pTZ pri-miR-31/5/8. Thereafter, the sequence encoding pri-miR-31/9 was excised from pTZ miR-31/9 with *Nhe*I and *Eco*RI and ligated into pTZ pri-miR-31/5/8, which had been digested with *Spe*I and *Eco*RI. Successful ligation generated pTZ pri-miR-31/5/8/9. The other 5 trimeric cassettes were generated by using similar cloning strategies. The trimeric pri-miR-31 cassettes excised with *Nhe*I and *Xba*I were cloned into equivalent sites of pCI-neo (Promega, WI, USA) to generate the CMV pri-miR-31 multimeric plasmid-based cassettes.

### Northern blot analysis

HEK283 cells were harvested 2 days after transfection of a 40% confluent 10 cm diameter culture dish with 10 µg of RNAi effecter plasmid and total RNA was extracted using Tri Reagent (Sigma, MI, USA) according to the manufacturer's instructions. Thirty µg of RNA was resolved on urea denaturing 12. 5% polyacrylamide gels and blotted onto nylon membranes. Blots were hybridised to three DNA oligonucleotides which were complementary to anti HBV guides 5, 8 and 9. Probes were labelled at their 5' ends with [a-³²P]ATP and T4 polynucleotide kinase. After purification using standard procedures, they were hybridized to immobilised RNA, exposed to X-ray film then stripped and reprobed. An oligonucleotide sequence complementary to U6 snRNA was used as a control for equal loading of the cellular RNA. Probe oligonucleotide sequences were:
Guide 5 probe: 5'- CCGTGTGCACTTCGCTTC -3' (SEQ ID NO: 86)
Guide 8 probe: 5'- CAATGTCAACGACCGACC -3' (SEQ ID NO: 87),
Guide 9 probe: 5'- TAGGAGGCTGTAGGCATA -3' (SEQ ID NO: 88); and
U6 snRNA probe: 5' TAGTATATGTGCTGCCGAAGCGAGCA 3' (SEQ ID NO: 89).

### Target Plasmids

To generate dual luciferase targets containing the anti HBV miR 5, anti HBV miR 8 and anti HBV miR 9 targets, primers were used to amplify HBV DNA and introduce a Spe I site at the 3' end of the amplicon. Primer sequences to amplify the targets were as follows:
**Target sequence 5**
   5TS F 5'-CCGTGTGCACTTCGCTTCAC-3' (SEQ ID NO: 90)
   5TS R 5'-ACTAGTCAGAGGTGAAGCGA-3' (SEQ ID NO: 91)
**Target sequence 8**
   8TS F 5'-CAATGTCAACGACCGACCTT-3' (SEQ ID NO: 92)
   8TS R 5'-ACTAGTGCCTCAAGGTCGGT-3' (SEQ ID NO: 93)
**Target sequence 9**
   9TS F 5'-TAGGAGGCTGTAGGCATAAA-3' (SEQ ID NO: 94)
   9TS R 5'-ACTAGTACCAATTTATGCCT-3' (SEQ ID NO: 95)

Purified fragment was initially ligated into the pTZ-57R/T PCR cloning vector and the insert was removed with *Sal* I and *Spe* I and ligated into the *Xho* I and *Spe* I sites of psi-CHECK 2. 2 (Promega, WI, USA) to generate psi-CHECK-5T, psi-CHECK-8T and psi-CHECK-9T with the HBV target site downstream of the Renilla *luciferase* ORF. All plasmid sequences were verified according to standard dideoxy chain termination protocols (Inqaba Biotechnology, South Africa).

The pCH-9/3091 plasmid has been described previously and above [42]. Culture and transfection of Huh7 and HEK293 lines was carried out as has been described [39]. Measurement of HBsAg was carried out using the the Monolisa (ELISA) immunoassay kit (BioRad, CA, USA) as described above. Ratios of 10:1 and 1:1 of U6 shRNA 5 vector to pCH9/3091 were tested, but for all other combinations, the ratio of RNAi expressing vector to pCH9/3091 was 10:1. Using a 12 well culture dish format, cells received 100 ng of pCH9/3091 and 1 µg of RNAi effecter plasmid. A plasmid vector that constitutively produces eGFP [43] was also included in each cotransfection and equivalent transfection efficiencies were verified by fluorescence microscopy.

*Assessment of multimeric miR shuttle-mediated silencing.* To determine the effect of miR-expressing vectors on reporter gene silencing, dual luciferase reporter plasmids (psi-CHECK-5T, psi-CHECK-8T and psi-CHECK-9T) containing independent target sequences were transfected together with pri-miR shuttle-expressing vectors. A plasmid vector that constitutively produces eGFP [43] was also included in each cotransfection to verify equivalent transfection efficiencies using fluorescence microscopy. The activities of *Renilla* and Firefly luciferase were measured with the dual luciferase assay kit (Promega, WI, USA) and using the Veritas dual injection luminometer (Turner BioSystems, CA, USA).

*Statistical Analysis.* Analysis of statistically significant differences was carried out using the student's paired two-tailed t-test. Calculations were made with the GraphPad Prism software package (GraphPad Software Inc., CA, USA).

### Northern Blot analysis of RNA from cells transfected with trimeric miR expression cassettes

Northern blot hybridisation of RNA extracted from cells that had been transfected with multimeric expression cassettes followed by probing for guide sequences complementary to each of the guides of HBV targets 5, 8 and 9 are shown in Figures 20, 21 and 22. Putative guide sequence targeting HBV site 5 was detectable in RNA extracted from cells transfected with each of the trimeric pri-miR expression cassettes (Figure 20). The sizes of the detected bands were approximately 20-22 bases in length, which indicates that there is heterogenous processing of the expressed sequences. Importantly, the concentrations of the guide sequences were approximately equivalent for each of the trimeric cassettes and positioning of the pri-miR 5 sequence did not affect the processing. In addition to the mature processed guide strands, larger molecular weight precursors were also detectable, which indicate incomplete processing of the transcript. Probing for the guide targeting HBV sequence 8 also revealed intended processed guide of approximately 21 nt in length (Figure 21). Unlike with the pri-miR5-derived guide, the size of the mature sequence was homogenous, and incompletely processed precursors were not detectable on the Northern blot analysis. Importantly, the concentrations were equivalent for cells transfected with each of the trimeric cassettes with the exception of plasmids containing the CMV miR 5-9-8 and CMV miR 9-8-5 cassettes. These data suggest that the processing of guide 8 sequence is compromised when positioned immediately downstream of pri-miR 9 RNA. Similar northern blot hybridisation analysis using a probe that was complementary to sequence 9 revealed that the processed guide sequence was present in all the samples analysed (Figure 22). The concentrations were however markedly decreased when compared to the hybridisation signals observed for probes 5 and 8. There were also variations in the concentrations of guide 9 sequence detected, and the amounts detectable in the cells that had been transfected with CMV pri-miR 9, CMV pri-miR 5-9-8 and CMV pri-miR9-8-5 yielded the highest concentrations of guide 9 target. The significance, if any, of these variations is however not clear.

### Assessment of efficacy of muitimeric miR expression cassettes

To assess the efficacy of knockdown, the dual luciferase assay was undertaken on lysates from cells that had been cotransfected with plasmids expressing pri-miR-expressing cassettes together with the psiCHECK-derived vectors that had individual HBV targets inserted downstream of the *Renilla* luciferase open reading frame (Figure 23). Controls (mock treated cells) were cotransfected with the reporter gene plasmid together with a backbone pCl neo vector tat lacked pri-miR shuttles. Calculation of the ratio of *Renilla* to constitutively expressed Firefly luciferase activity was used to determine knockdown efficacy and the value control group was normalised to 1. Figure 23 shows the data that were obtained from this analysis. All 6 trimeric pri-miR shuttle cassettes were capable of significant silencing of *Renilla* luciferase activity when cotransfection was with psi-CHECK-5T. Approximately 85-90% efficiency of knockdown was achieved. When using the psi-CHECK-8T target vector, efficient silencing was achieved with 4 of the 6 trimeric expression plasmids (approx 90%). The CMV miR 5-9-8 and CMV miR 9-8-5 cassettes were however incapable of effecting silencing, and the *Renilla* luciferase activity was equivalent to that of the mock treated cells. Importantly, this observation correlates with the results from Northern blot analysis, which showed that the guide sequence against target 8 was not generated efficiently (Figure 21). Thus, as is expected, poor efficiency of generating the RNAi effecter bears a direct relationship to lack of silencing. When assessing effects of cotransfection on silencing of *Renilla* luciferase activity derived from the psi-CHECK-9T plasmid, the efficacy was lower than that which was achieved with the psi-CHECK-5T and psi-CHECK-8T target reporters. *Renilla* luciferase activity in these cells ranged from approximately 45-75% of the mock treated cells. This again correlates with the data from Northern blot analysis where the amount of processed guide sequence was diminished compared to the sequence 5 and 8 guides (Figure 22). Moreover, the best silencing was achieved with CMV miR 5-9-8 and CMV miR 9-8-5 vectors (approx 75%), which were also the cassettes that generated the highest amount of mature guide 9 sequence (Figure 20).

The effect of the multimeric pri-miR expression cassettes on the secretion of HBsAg from transfected liver cells in culture was also determined (Figure 24). Each of the trimeric cassettes was capable of inhibiting the secretion of HBsAg from the transfected cells and the knockdown that was achieved was > 90%. This highly efficient knockdown was almost equivalent to the background signal of the mock treated cells. Thus, in cultured cells, the trimeric cassettes were capable of efficient silencing of a marker of HBV replication.

### Conclusions

Collectively, these data demonstrate that trimeric pri-miR expression cassettes are capable of generating individual anti HBV guide sequences that are efficient silencers of reporter gene constructs that include HBV targets. The efficiency of guide sequence generation is not uniform, and may be dependent on inherent sequence characteristics of the miR shuttles (e. g. lower processing and silencing achieved by pri-miR 9 shuttles) as well as the surrounding pri-miRs (e. g. diminished pri-miR 8 silencing achieved when located immediately downstream of the pri miR 9 sequence).

### Example 10: The miR31-based hairpin design achieved knockdown of the exogenous viral gene HCV 5' UTR transcript

### Materials and Methods

The DNA sequence for the HCV 5' UTR region cloned into pTAG-A was a gift from Prof Richard M. Elliot, Institute of Virology, University of Glasgow, U. K. pGL3-Basic, Dual-Glo^{™} Luciferase Assay System, and pGEM®-T Easy Vector Kit was from Promega, USA. BenchTop 1kb DNA ladder, InsTAclone^{™} PCR Cloning Kit and PCR Master Mix (2X) was from Fermentas, Lithuania. Oligonucleotide primers were from IDT, USA. Restriction enzymes *Pst*I, *Eco*RI, *Xho*I and *Xba*I were from New England Biolabs, USA. a-D-Galactopyranoside (X-gal), Ampicilin, Lipofectamine 2000^{™} were from Sigma, UK. QIAprep Spin Miniprep Kit and QIAGEN Endofree Maxi-prep kit were from Qiagen, Germany. RPMI, FCS, Penicillin/Streptomycin, and OptiMEM were from Gibco, UK. Nucleospin Kit was from Macllery-Nagel, Germany. The Veritas^{™} Microplate Luminometer was from Turner Biosystems, USA.

### Subcloning of the HCV 5' UTR target sequence and the firefly luciferase coding sequence

The DNA sequence for the HCV 5' UTR region (SEQ ID NO: 34) was amplified using PCR with plasmid construct pTAG-A as template, forward primer F5'UTRXhoI (ATT ACT CGA GTT CAC GCA GAA AGC GTC; SEQ ID NO: 96) and reverse primer R5'UTREcoRI (ATT AGA ATT CGA ACT TGA CGT CCT GTG G; SEQ ID NO: 97). F5'UTRXhoI and R5'UTREcoRI consisted of four spacer 5'-terminal nucleotides, ATTA, the 6-nt recognition sequences for *Xho*I and *Eco*RI restriction endonucleases respectively for future directional cloning and screening purposes, and complementary sequences to the 5' and 3' ends of the HCV 5' UTR region respectively which included between them the first 351 nt of the HCV transcript.

The coding region for firefly luciferase was similarly amplified using pGL-3-Basic as a template, forward primer FLucEcoRI (ATT AGA ATT CAT GGA AGA CGC CAA AAA C; SEQ ID NO: 98) and reverse primer RLucXbal (ATT ATC TAG ATT ACA CGG CGA TCT TTC C; SEQ ID NO: 99). FLucEcoRI and RLucXbal consisted of four spacer 5'-terminal nucleotides, ATTA, the 6-nt recognition sequences for *Eco*RI and *Xba*I restriction endonucleases respectively for future directional cloning and screening purposes, and complementary sequences to the 5' and 3' ends of the firefly luciferase coding sequence respectively which included between them the full length of 1653 nt.

The PCR reactions contained template DNA [2 µl, 100ng]; forward primer [1 µl of 100 µM]; reverse primer [1 µl of 100 µM]; PCR Master Mix (2X) [25 µl] in a final volume of 50 µl. The PCR reaction conditions were 94°C, 2 min; 94°C for 30 s, 55°C for 1 min, 72°C for 2.5 min, 30 cycles; 72°C, 10 min.

The length of the PCR products was confirmed by agarose gel electrophoresis as described in Example 1. BenchTop 1 kb DNA ladder was resolved as a molecular marker. The resolved PCR products were visualized under UV radiation.

PCR product [1 µl] from the HCV 5'UTR and firefly luciferase PCR reactions were ligated into pGEM-T [1 µl] to produce pGEM-T-UTR and pGEM-T-LUC respectively, using the pGEM®-T Easy Vector Kit, in a ligation reaction containing 2x rapid ligation buffer [5 µl]] and T4 ligase enzyme [3U, 1 µl] in a final volume of 10 µl. The ligation reaction was incubated at 15°C overnight before being transformed [5 µl] into competent *Escherichia coli* DH5a [50 µl]. The transformation reaction [55 µl] was incubated on ice for 30 mins, heat pulsed at 42°C for 2 mins, and incubated on ice for 2 mins before being plated out onto 2 x YT broth agar plates containing 0. 1 mg/ml ampicillin, spread with a-D-Galactopyranoside (X-gal) [40 µl, 20 mg/ml in DMSO]. Competent *E*. *coli* DH5a cells were prepared according to standard protocols. The plates were incubated at 37°C for 16 hours.

Putative plasmid DNA was extracted from the *E*. *coli* cultures of transformants exhibiting white colonies for the pGEM-T-UTR and pGEM-T-LUC transformations by a modified standard alkaline lysis method using the QIAprep Spin Miniprep Kit as described in Example 1. The putative plasmid DNA was then screened by restriction with 5 U EcoRI as described in Example 1 in the buffer supplied by the manufacturer. Plasmid DNA exhibiting DNA fragments of the expected length were used for further cloning steps.

### Cloning of the HCV 5' UTR target expression construct (pCineoUTRLUC)

Bulk restriction endonuclease digestions were prepared of pGEM-T-UTR, pGEM-T-LUC, and pCineoHBX plasmid DNA. pGEM-T-UTR was restricted with *Xho*I and *Eco*RI, pGEM-T-LUC with *Eco*RI and *Xba*I*,* and pCineoHBX with *Xho*I and *Xba*I. pCineoHBX was used instead of pCineo in order to better isolate the double-restricted pCineo vector fragment. Bulk digestions consisted of plasmid DNA [60 µl, 200ng/µl], 10X *Eco*RI buffer, first enzyme [20 U, 4 µl], second enzyme [20 U, 4 µl], and water [22 µl]. The bulk digestions were incubated at 37°C for 1.5 hours, resolved by agarose gel electrophoresis as before and gel purified. For gel purification: the appropriate bands were excised out of the gel and extracted using the Nucleospin Kit. Briefly, buffer BT [300 µl] was added to the excised piece of gel and the agarose sample incubated at 50°C for 10 mins with frequent inversion. The melted agarose sample was loaded onto a nucleospin column and centrifuged at 12 000 x g for 30 s. The nucleospin column was then washed twice with buffer NT3 [700 µl] by centrifugation. The bound DNA was eluted by the addition of elution buffer NE [50 µl, 5 mM Tris-HCl, pH 8. 5,] followed by centrifugation at 12 000 x g for 1 min.

Eluted DNA fragments were ligated together in a ratio of 1:3:3 of 5'UTR:LUC:pCineo [1 µl:3 µl:3 µl] in a ligation reaction containing 2x rapid ligation buffer [10 µl] and T4 ligase enzyme [3U, 1 µl] in a final volume of 21 µl. The ligation reaction was incubated at 15°C overnight and transformed into competent *E*. *coli* DH5a as before. The putative plasmid DNA was extracted and screened by restriction as before with 5 U *Sph*I in 1x Buffer Tango [3. 3 mM Tris-acetate, pH 7. 9 at 37°C, 1 mM magnesium acetate, 6. 6 mM potassium acetate, 0.1 mg/ml BSA]. Bulk DNA was prepared as described in Example 1.

### In silico design of sh260 and miR260

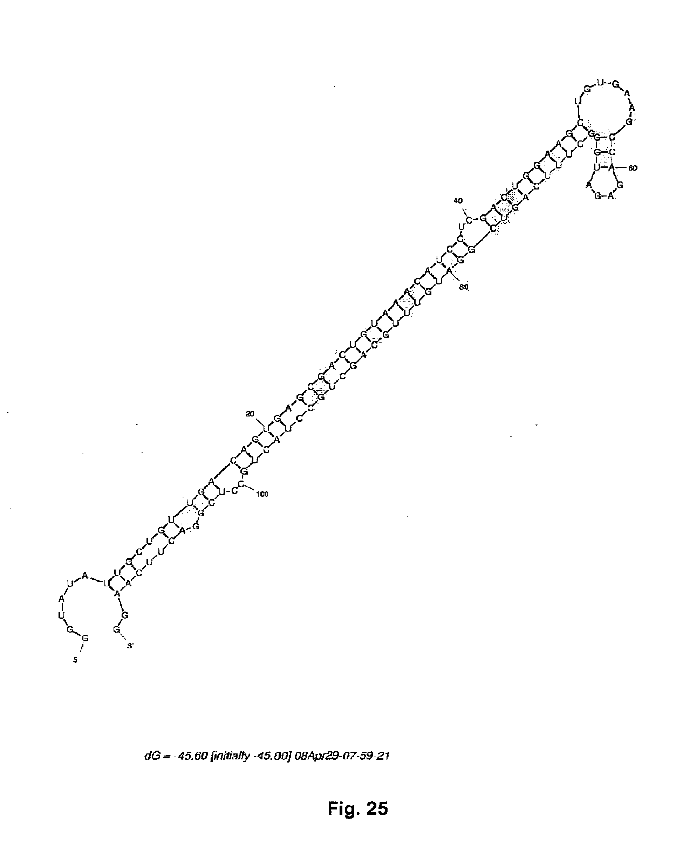

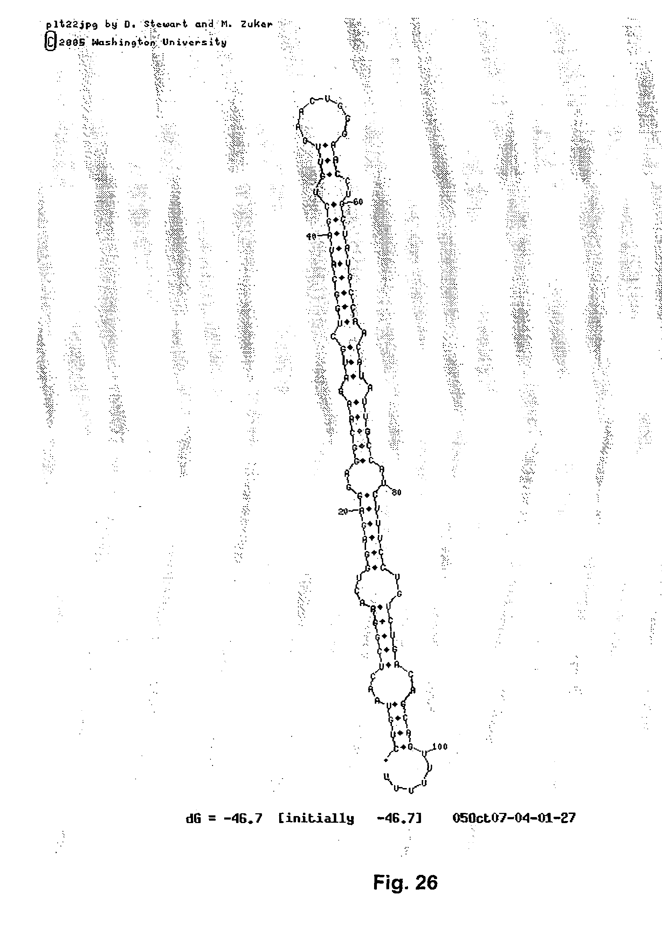

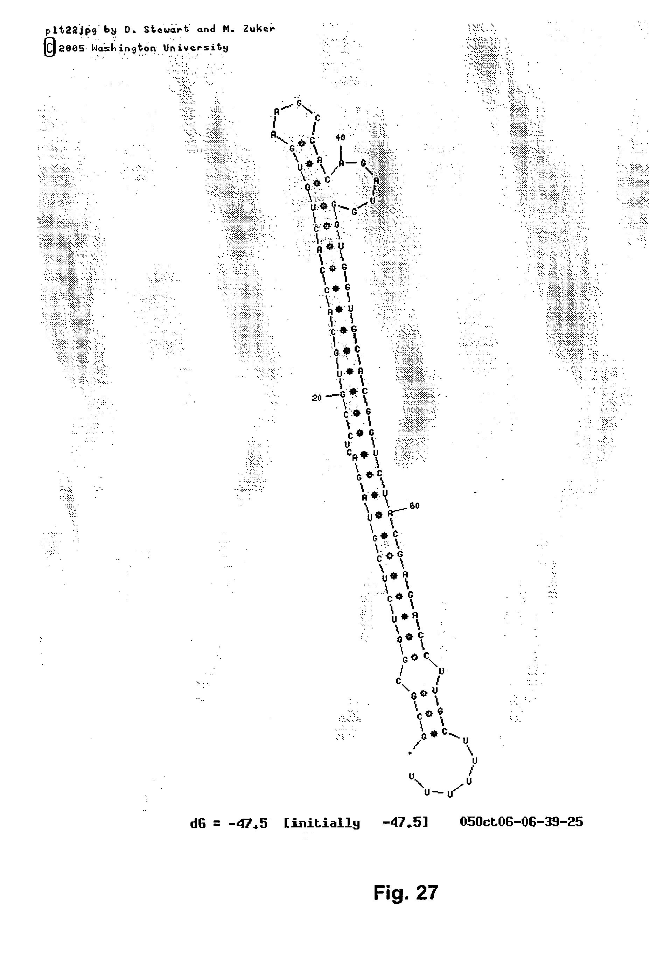

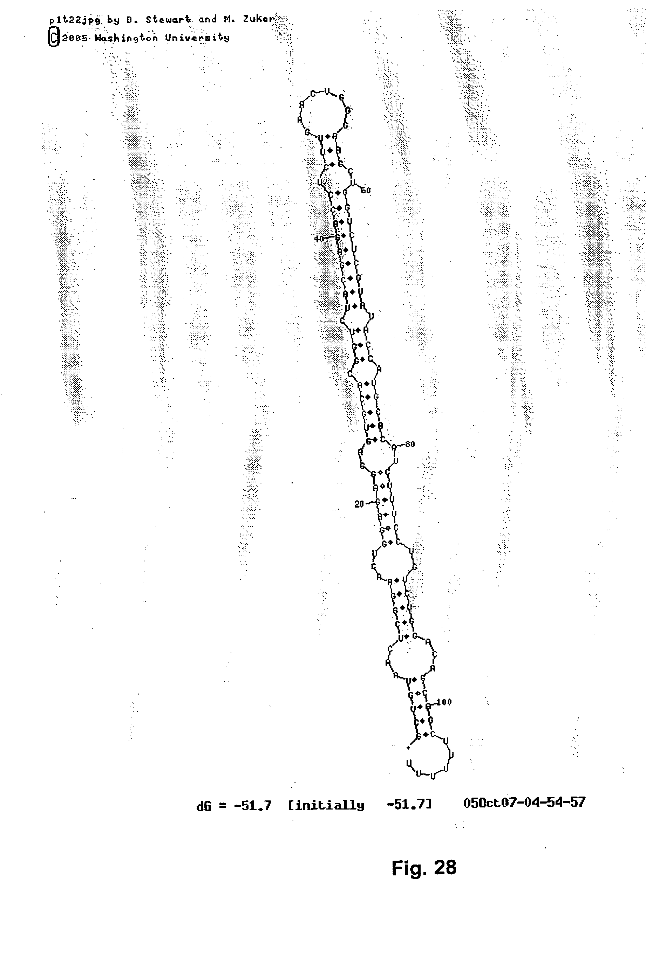

The structure of miR-30 (Figure 25) and miR-31 (Figure 26) [41] was used as a basis to design miR-30-like (SEQ ID NO: 35 (164)) and miR-31-like (SEQ ID NO: 1 (133)) hairpins targeting the HCV 5' UTR region. Briefly, the approach to hairpin design was as follows: the siRNA disclosed in Yokota et al. (2003) [48] as "siRNA 331" was engineered into sh30-260 and miR31-260 designs specific for HCV strain 5a to produce sh260 and miR31-260 respectively, *in silico* using standard bioinformatic software such that mismatches occurred in the sense strand. A U6 promoter initiation sequence of one guanine base was inserted *in silico* at the beginning of the miR-30-like (sh260, Figure 27, SEQ ID NO. 36 (165)) and miR-31-like (miR260, Figure 28, SEQ ID NO. 37 (166)) hairpins and a termination sequence consisting of a run of six uracil bases was inserted at the 3' end. The RNA sequence for sh260 and miR260 was converted to the corresponding DNA sequence and inserted downstream of the DNA sequence for the U6 promoter *in silico* to form the sh260 and miR260 expression cassettes.

### Cloning of the sh260 and miR260 expression constructs (pTz57R-sh260 and pTz57R-miR260)

The DNA sequence for the U6 promoter was PCR amplified in a two-step PCR reaction during which the DNA sequence of either sh260 or miR260 was inserted downstream from the U6 promoter.

For sh260: The forward primer, FU6 (ATT AGA ATT CAA GGT CGG GCA GGA AGA G) (SEQ ID NO: 100), complementary to 24-nt of the 5'-end of the U6 promoter and four spacer 5'-terminal nucleotides, ATTA, was used for all PCR steps. In the first round of PCR, the reverse primer, RU6-sh260-1 (ACC CCC ATC TGT GGC TTC ACA GGG TGC ACG GGA TCT ACG AGA CCT TCG CCG GTG TTT CGT CCT TTC C) (SEQ ID NO: 101) was used. In the second round of PCR, the reverse primer, RU6-sh260-2 (GTC GAC AAA AAA GCA GAG GTC TCG TAG ACC GTG CAC CCC CAT CTG TGG CTT CAC AGG) (SEQ ID NO: 102) was used.

For miR260: The forward primer, FU6 (ATT AGA ATT CAA GGT CGG GCA GGA AGA G) (SEQ ID NO: 103), complementary to 24-nt of the 5'-end of the U6 promoter and four spacer 5'-terminal nucleotides, ATTA, was used for all PCR steps. In the first round of PCR, the reverse primer, RU6-miR31-260-1 (GCT TCC CAG TTC AAG AGG TCT CGT AGA CCG TGC ACT CCT CTC CAG TTC CGA GTT ACA GCG GTG TTT CGT CCT TTC C) (SEQ ID NO: 104) was used. In the second round of PCR, the reverse primer, RU6-miR31-260-2 (GTC GAC AAA AAA GCT GCT GTC CAG ACA GGA AAG ATG TGC ATG GTA TAC GAG ACC AGC TTC CCA GTT CAA GAG GTC TC) (SEQ ID NO: 105) was used.

The first PCR reaction contained pTz57-U6 template [1 µl, 100ng]; FU6 primer [1 µl of 6 mM]; reverse primer [2 µl of 6 mM]; dNTPs [1 µl of 100 mM]; GoTaq® PCR System enzyme [0.5 µl]; and GoTaq® PCR System 10x Buffer [5 µl] in a final volume of 50 µl. The PCR reaction conditions were 94°C, 2 min; 94°C for 30 s, 55°C for 1 min, 72°C for 2.5 min, 30 cycles; 72°C, 10 min. The second PCR reaction contained the same reagents as the first except for the replacement of the template and the reverse primer. One microliter of this first PCR reaction mixture was used as template and re-amplified in the second round of PCR using the same forward primer FU6 and the second reverse primer.

The length of the PCR products from the second round of PCR was confirmed by agarose gel electrophoresis and ligated into pTz57R/T. Briefly, PCR products [5 µl] of the first and second PCR reactions were each added to agarose gel loading buffer [5 µl, 30% glycerol v/v, 0.25% w/v bromophenol blue] and resolved on an agarose gel [50 ml, 0.8% w/v] containing ethidium bromide [0.5 µg/ml], in TBE buffer [45 mM Tris, 45 mM Borate, 1 mM EDTA, pH 8.3] at 100 V for 2 hours. *Pst*I-digested lamda DNA was resolved as a molecular marker. The resolved PCR products were visualized under UV radiation. Putative pTz57R-sh260 transformants and putative pTz57R-miR260 transformants were screened by *Eco*RI restriction enzyme digestion as described in Example 1, and the digested fragments resolved by agarose gel electrophoresis as previously described. Putative pTz57R-sh260 was also confirmed by PCR by PCR amplification of extracted plasmid DNA using FU6 and RU6-sh260-2 under amplification conditions as described above. Purified pTz57R-sh260 and pTz57R-miR260 were prepared using the QIAGEN Endofree Maxi-prep Kit according to manufacturer's instructions.

### Culturing of HuH-7 cells

The human hepatoma cell line, Huh-7, was maintained in RPMI media supplemented with 10% fetal calf serum (FCS), penicillin (100 U mL⁻¹) and streptomycin (100 U mL⁻¹) [complete media] in a humidified atmosphere, at 37°C with 5% CO₂. Cells were typically subcultured every 2 to 3 days and maintained in 6 cm dishes. All solutions were preheated before use. Cells were trypsinized in 1 x Trypsin/EDTA at 37°C for 3-5 mins. The trypsin reaction was stopped by the addition of an equal volume DMEM containing 10% FCS, and the cells collected by centrifugation at 2000 rpm for 2 mins in a desk-top centrifuge. The cell pellet was resuspended in complete media [5 ml] and the suspension used to seed [1 ml] fresh complete media [15 ml] in a 6 cm dish.

### Co-transfection of HuH-7 cells with pCineoUTRLUC, pCMV-Ren, gTz57R-sh260, and pTz57R-miR260 and HCV 5'UTR knockdown analysis by luminescence measurement

HuH-7 cells were transiently co-transfected in triplicate with pCineoUTRLUC, pCMV-Ren, and pTz57R-sh260, or pCineoUTRLUC, pCMV-Ren, and pTz57R-miR260, or pCineoUTRLUC, pCMV-Ren, and pTz57R-miR118 (an expression construct expressing a miR31 hairpin targeting Hepatitis C Virus with poor efficiency and used here as a negative control) using Lipofectamine 2000^{™} in 6 well plates seeded to 60% confluency, according to manufacturer's instructions. pCMV-Ren was used to express Renilla luciferase as an internal efficiency of transfection control. Each transfection contained pCineoUTRLUC [100ng], pCMV-Ren [50 ng], and pTz57R construct [1 µg]. Plasmid DNA: Lipofectamine 2000^{™} complex solutions were prepared per reaction as follows: pre-warmed Lipofectamine 2000^{™} [4 µl per reaction] was added to OptiMEM [150 µl per reaction] and allowed to incubate at room temperature for 15 mins. Plasmid DNA [1. 06 µg total DNA per reaction] was added to OptiMEM [150 µl per reaction] and allowed to incubate at room temperature for 15 mins. The Lipofectamine 2000^{™} and plasmid DNA solutions were then combined, mixed gently by pipetting and allowed to complex at room temperature for 15 mins to produce a transfection reaction.

12 well dishes seeded to 60% confluency were washed with PBS. Each separate transfection reaction was then added to a well and incubated in a humidified atmosphere, at 37°C with 5% CO₂ for 2 hours. The transfection reaction was then removed, complete media [2 ml] added, and the dishes incubated for 48 hours in a humidified atmosphere, at 37°C with 5% CO₂.

To assess HCV 5' UTR knockdown, luciferase expression was assessed using the Dual-Glo^{™} Luciferase Assay System. After the 48 hour incubation, the transfected cells were washed three times with PBS before the addition of 1x PLB buffer [100 µl] directly to the cells followed by gentle rocking at room temperature for 30 mins. Cell lysate [20 µl] was then dispensed from each transfection well into a well of a whiote opaque luminometer microtitre plate. Luminescence measurements were taken using the Veritas^{™} Microplate Luminometer. The P and M injectors were primed with Luciferase Assay Reagent II (LARII) and Stop and Glo reagent respectively. The microtitre plate was placed in the luminometer and LARII [100 µl] injected into each well before the luminescence was read with a 2 s premeasurement delay and a 10 s integration to detect firefly luciferase. Stop and Glo [100 µl] was the injected per well with the M injector and the luminescence read with a 2 s premeasurement delay and a 10 s integration to detect renilla luciferase. The experiment was repeated. The readings were analysed by calculating a firefly/renilla luminescence ratio and normalizing the ratio value obtained for the pTz57R-sh260 and the pTz57R-miR260 transfections against that obtained for the pTz57R-miR118 transfection. Statistical differences were calculated using the Students T-Test.

### Results and Discussion

### Cloning of the HCV 5' UTR target expression construct (pCineoUTRLUC)

The HCV 5'UTR and firefly luciferase PCR amplification successfully yielded a 346 bp and a 1653 bp DNA product (Figure 29 A, lanes 2 and 3). The PCR products were successfully ligated into pGEM-T as shown by restriction endonuclease digestion (Figures 29 B and 29 C). The restriction of pGEM-T-UTR and pGEM-T-LUC with *Eco*RI yielded the expected fragments of 3015 bp and 346 bp (Figure 29 B, lanes 3-12), and 3015 bp and 1653 bp (Figure 29 C, lanes 4, 5, 7-9, and 11) respectively. Clone 2 was selected for pGEM-T-UTR and clone 3 was selected for pGEM-T-LUC for further cloning. The HCV 5'UTR and firefly luciferase DNA was successfully subcloned into pCineo to produce pCineoUTRLUC (Figure 30). *Sph*I-restriction of plasmid DNA extracted from clones 1-5, 8-12,17-19, 21 and 22 yielded the expected fragments of 4593 bp, 2026 bp, and 779 bp (Figure 30, lanes 3-7, 10-14, 19-21, and 23-24). Clone 1 was selected.

### Design of sh260 and miR31-260 and cloning pTz57R-sh260 and pTz57R-miR31260

The predicted structure of the miR-30 [41] (Figure 25) did not exhibit a similar structure to that of the predicted sh260 (Figure 27, SEQ ID NO: 36) as predicted by the online software program mFOLD [49](. However, the predicted structure of the miR-31 (Figure 26) exhibited a similar structure to that of the designed miR260 (Figure 28, SEQ ID NO: 4) showing miR260 to have typical miR31 secondary structure.

### Cloning of the sh260 and miR260 expression constructs (pTz57R-sh260 and pTz57R-miR260)

pTz57R-sh260: The length of the PCR products from the two-step PCR reaction was confirmed by agarose gel electrophoresis to be approximately 347 bp for the products of the first PCR reaction (Figure 31 A, lane 2) and to be approximately 486 bp for the products of the second PCR reaction. (Figure 31 A, lane 3). PCR screening of putative pTz57R-sh260 successfully yielded a PCR product of 486 bp for clones 1-4 and 6 (Figure 31 B, lanes 2-5, and 7). Digestion of putative pTz57R-sh260 from clone 3 with *Sal*I produced the expected fragments of 2886 bp, and 386 bp (Figure 31 C, lane 2). Clone 3 was therefore selected.

pTz57R-miR260: The length of the PCR products from the two-step PCR reaction was confirmed by agarose gel electrophoresis to be approximately 323 bp for the products of the first PCR reaction (Figure 32 A, lane 8) and to be approximately 377 bp for the products of the second PCR reaction. (Figure 32 B, lane 8). Digestion of putative pTz57R-miR260 from clone 1 with *Sal*I produced the expected fragments of 2886 bp, and 377 bp (Figure 32 C, lane 1). Clone 1 was therefore selected.

### Expression of miR260 results in better HCV5'UTR knockdown than sh260

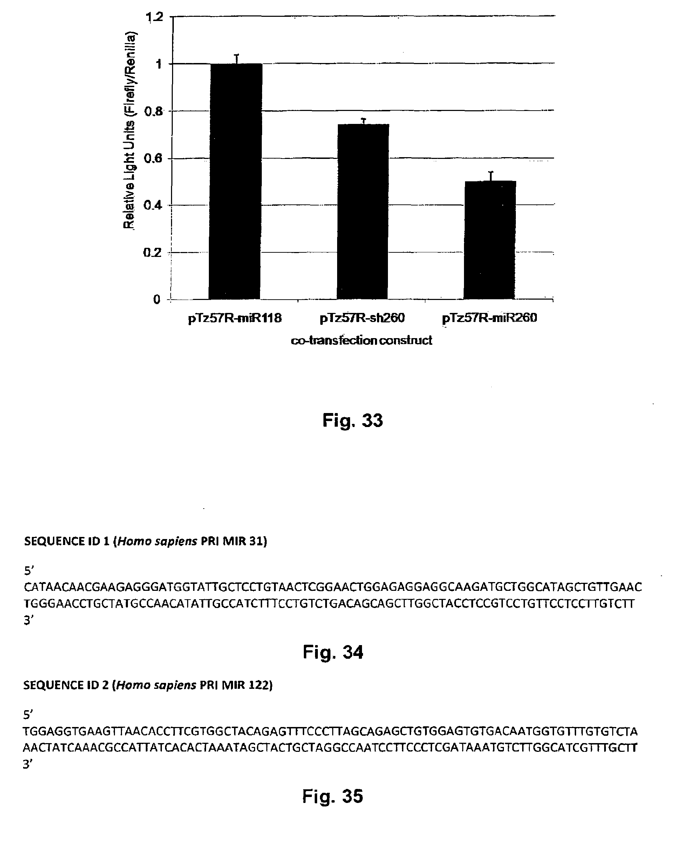

To determine whether sh260 and miR260 knocked down expression of the HCV 5'UTR region, we fused the 5' UTR to the firefly luciferase indicator gene. Both sh260 (p = 0.000562004) and miR260 (p = 9.96473E-05) were able to significantly knock down the expression of the indicator gene when compared to the negative control of miR118 (Figure 33), however miR260 effected a greater knockdown (50%) as compared to sh260 (26%). The miR-31-based design of miR260 was therefore found to be more efficient in targeting the HCV 5'UTR transcript than was sh260.

### References

1. M Wassenegger, T Pelissier (1998): A model for RNA-mediated gene silencing in higher plants. Plant Mol Biol 37:349.
2. H Ngo, C Tschudi, K Gull, E Ullu (1998): Double-stranded RNA induces mRNA degradation in Trypanosoma brucei. Proc Natl Acad Sci U S A 95:14687.
3. L Misquitta, BM Paterson (1999): Targeted disruption of gene function in Drosophila by RNA interference (RNA-i): a role for nautilus in embryonic somatic muscle formation. Proc Natl Acad Sci U S A 96:1451.
4. NJ Caplen, J Fleenor, A Fire, RA Morgan (2000): dsRNA-mediated gene silencing in cultured Drosophila cells: a tissue culture model for the analysis of RNA interference. Gene 252:95.
5. YX Li, MJ Farrell, R Liu, N Mohanty, ML Kirby (2000): Double-stranded RNA injection produces null phenotypes in zebrafish. Dev Biol 217:394.
6. H Akashi, M Miyagishi, T Yokota, T Watanabe, T Hino, K Nishina, M Kohara, T K. (2005): Escape from the interferon response associated with RNA interference using vectors that encode long modified hairpin-RNA. Molecular BioSystems 1:382.
7. EM Maine (2000): A conserved mechanism for post-transcriptional gene silencing? Genome Biol 1:1018.
8. GJ Hannon (2002): RNA interference. Nature 418:244.
9. G Hutvagner, PD Zamore (2002): A microRNA in a multiple-turnover RNAi enzyme complex. Science 297:2056.
10. SM Elbashir, W Lendeckel, T Tuschl (2001): RNA interference is mediated by 21- and 22-nucleotide RNAs. Genes Dev 15:188.
11. M Jiang, J Milner (2002): Selective silencing of viral gene expression in HPV-positive human cervical carcinoma cells treated with siRNA, a primer of RNA interference. Oncogene 21:6041.
12. X Zou, D Ray, A Aziyu, K Christov, AD Boiko, AV Gudkov, H Kiyokawa (2002): Cdk4 disruption renders primary mouse cells resistant to oncogenic transformation, leading to Arf/p53-independent senescence. Genes Dev 16:2923.
13. AJ Hamilton, DC Baulcombe (1999): A species of small antisense RNA in posttranscriptional gene silencing in plants. Science 286:950.
14. E Bernstein, AA Caudy, SM Hammond, GJ Hannon (2001): Role for a bidentate ribonuclease in the initiation step of RNA interference. Nature 409:363.
15. SM Elbashir, J Harborth, W Lendeckel, A Yalcin, K Weber, T Tuschl (2001): Duplexes of 21-nucleotide RNAs mediate RNA interference in cultured mammalian cells. Nature 411:494.
16. T Dalmay, R Horsefield, TH Braunstein, DC Baulcombe (2001): SDE3 encodes an RNA helicase required for post-transcriptional gene silencing in Arabidopsis. Embo J 20:2069.
17. A Nykanen, B Haley, PD Zamore (2001): ATP requirements and small interfering RNA structure in the RNA interference pathway. Cell 107:309.
18. A Smardon, JM Spoerke, SC Stacey, ME Klein, N Mackin, EM Maine (2000): EGO-1 is related to RNA-directed RNA polymerase and functions in germ-line development and RNA interference in C. elegans. Curr Biol 10:169.
19. A Fire, S Xu, MK Montgomery, SA Kostas, SE Driver, CC Mello (1998): Potent and specific genetic interference by double-stranded RNA in Caenorhabditis elegans. Nature 391:806.
20. H Kawasaki, K Taira (2004): Induction of DNA methylation and gene silencing by short interfering RNAs in human cells. Nature*.*
21. J Gil, M Esteban, D Roth (2000): In vivo regulation of the dsRNA-dependent protein kinase PKR by the cellular glycoprotein p67. Biochemistry 39:16016.
22. MR Player, PF Torrence (1998): The 2-5A system: modulation of viral and cellular processes through acceleration of RNA degradation. Pharmacol Ther 78:55.
23. M Kumar, GG Carmichael (1998): Antisense RNA: function and fate of duplex RNA in cells of higher eukaryotes. Microbiol Mol Biol Rev 62:1415.
24. S Agrawal, Q Zhao (1998): Antisense therapeutics. Curr Opin Chem Biol 2:519.
25. M Miyagishi, K Taira (2002): U6 promoter-driven siRNAs with four uridine 3' overhangs efficiently suppress targeted gene expression in mammalian cells. Nat Biotechnol 20:497.
26. NS Lee, T Dohjima, G Bauer, H Li, MJ Li, A Ehsani, P Salvaterra, J Rossi (2002): Expression of small interfering RNAs targeted against HIV-1 rev transcripts in human cells. Nat Biotechnol 20:500.
27. TR Brummelkamp, R Bernards, R Agami (2002): A system for stable expression of short interfering RNAs in mammalian cells. Science 296:550.
28. PJ Paddison, AA Caudy, E Bernstein, GJ Hannon, DS Conklin (2002): Short hairpin RNAs (shRNAs) induce sequence-specific silencing in mammalian cells. Genes Dev 16:948.
29. G Sui, C Soohoo, B Affar el, F Gay, Y Shi, WC Forrester (2002): A DNA vector-based RNAi technology to suppress gene expression in mammalian cells. Proc Natl Acad Sci U S A 99:5515.
30. JY Yu, SL DeRuiter, DL Turner (2002): RNA interference by expression of short-interfering RNAs and hairpin RNAs in mammalian cells. Proc Natl Acad Sci U S A 99:6047.
31. CP Paul, PD Good, I Winer, DR Engelke (2002): Effective expression of small interfering RNA in human cells. Nat Biotechnol 20:505.
32. MT McManus, CP Petersen, BB Haines, J Chen, PA Sharp (2002): Gene silencing using micro-RNA designed hairpins. Rna 8:842.
33. A Grishok, AE Pasquinelli, D Conte, N Li, S Parrish, I Ha, DL Baillie, A Fire, G Ruvkun, CC Mello (2001): Genes and mechanisms related to RNA interference regulate expression of the small temporal RNAs that control C. elegans developmental timing. Cell 106:23.
34. J Beck, M Nassal (1995): Efficient hammerhead ribozyme-mediated cleavage of the structured hepatitis B virus encapsidation signal in vitro and in cell extracts, but not in intact cells. Nucleic Acids Res 23:4954.
35. H Kawasaki, K Taira (2003): Short hairpin type of dsRNAs that are controlled by tRNA(Val) promoter significantly induce RNAi-mediated gene silencing in the cytoplasm of human cells. Nucleic Acids Res 31:700.
36. D Grimm, KL Streetz, CL Jopling, TA Storm, K Pandey, CR Davis, P Marion, F Salazar, MA Kay (2006): Fatality in mice due to oversaturation of cellular microRNA/short hairpin RNA pathways. Nature 441:537.
37. H Xia, Q Mao, HL Paulson, BL Davidson (2002): siRNA-mediated gene silencing in vitro and in vivo. Nat Biotechnol 20:1006.
38. AE Walter, DH Turner, J Kim, MH Lyttle, P Muller, DH Mathews, M Zuker (1994): Coaxial stacking of helixes enhances binding of oligoribonucleotides and improves predictions of RNA folding. Proc Natl Acad Sci U S A 91:9218.
39. S Carmona, A Ely, C Crowther, N Moolla, FH Salazar, PL Marion, N Ferry, MS Weinberg, P Arbuthnot (2006): Effective Inhibition of HBV Replication in Vivo by Anti-HBx Short Hairpin RNAs. Mol Ther 13:411.
40. J Chang, E Nicolas, D Marks, C Sander, A Lerro, MA Buendia, C Xu, WS Mason, T Moloshok, R Bort, KS Zaret, JM Taylor (2004): miR-122, a mammalian liver-specific microRNA, is processed from hcr mRNA and may downregulate the high affinity cationic amino acid transporter CAT-1. RNA Biol 1:106.
41. Y Zeng, BR Cullen (2005): Efficient processing of primary microRNA hairpins by Drosha requires flanking nonstructured RNA sequences. J Biol Chem 280:27595.
42. M Nassal (1992): The arginine-rich domain of the hepatitis B virus core protein is required for pregenome encapsidation and productive viral positive-strand DNA synthesis but not for virus assembly. J Virol 66:4107.
43. M Passman, M Weinberg, M Kew, P Arbuthnot (2000): In situ demonstration of inhibitory effects of hammerhead ribozymes that are targeted to the hepatitis Bx sequence in cultured cells. Biochem Biophys Res Commun 268:728.
44. M Weinberg, M Passman, M Kew, P Arbuthnot (2000): Hammerhead ribozyme-mediated inhibition of hepatitis B virus X gene expression in cultured cells. J Hepatol 33:142.
45. BS Heale, HS Soifer, C Bowers, JJ Rossi (2005): siRNA target site secondary structure predictions using local stable substructures. Nucleic Acids Res 33:e30.
46. KH Heermann, WH Gerlich, M Chudy, S Schaefer, R Thomssen (1999): Quantitative detection of hepatitis B virus DNA in two international reference plasma preparations. Eurohep Pathobiology Group. J Clin Microbiol 37:68.
47. D Moore, D Dowhan (2002): Preparation and analysis of DNA. In: Current Protocols in Molecular Biology. R. Brent, R. Kingston, D. Moore, J. G. Seidman, J. A. Smith and K. Struhl, eds., John Wiley and Sons Inc, Hoboken, NJ, 2.0.1.
48. E Song, P Zhu, SK Lee, D Chowdhury, S Kussman, DM Dykxhoorn, Y Feng, D Palliser, DB Weiner, P Shankar, WA Marasco, J Lieberman (2005): Antibody mediated in vivo delivery of small interfering RNAs via cell-surface receptors. Nat Biotechnol 23:709.
49. T Yokota, N Sakamoto, N Enomoto, Y Tanabe, M Miyagishi, S Maekawa, L Yi, M Kurosaki, K Taira, M Watanabe, H Mizusawa (2003): Inhibition of intracellular hepatitis C virus replication by synthetic and vector-derived small interfering RNAs. EMBO Rep 4:602.
50. M Zuker (2003): Mfold web server for nucleic acid folding and hybridization prediction. Nucleic Acids Res 31:3406.

## Claims

1. An anti-hepatitis virus primary micro RNA (pri-miR) expression cassette, including:
(i) a DNA sequence encoding an artificial pri-miR-31 or pri-miR-122 sequence which mimics a naturally occurring pri-miR-31 or pri-miR-122 sequence, wherein the artificial pri-miR-31 or pri-miR-122 sequence differs from the naturally occurring pri-miR-31 or pri-miR-122 sequence in that the guide sequence of the naturally occurring pri-miR-31 or pri-miR-122 has been replaced with a guide sequence that targets hepatitis virus, and wherein the sequence of the artificial pri-miR-31 or pri-miR-122 has been designed such that the secondary structure of the artificial pri-miR-31 or pri-miR-122 mimics the secondary structure of the naturally occurring pri-miR-31 or pri-miR-122; and
(ii) a Pol II promoter.

2. An expression cassette according to claim 1, wherein the hepatitis virus is hepatitis B or hepatitis C virus.

3. An expression cassette according to either of claims 1 or 2, wherein the Pol II promoter is a constitutive promoter, preferably a cytomegalovirus (CMV) promoter.

4. An expression cassette according to either of claims 1 or 2, wherein the Pol II promoter is a tissue-specific promoter, preferably a liver-specific promoter, more preferably an alpha-1-antitrypsin (A1AT) promoter, Factor VIII (FVIII) promoter, HBV basic core promoter (BCP) or PreS2 promoter.

5. An expression cassette according to any one of claims 1 to 4, wherein the hepatitis target sequence is selected from any one of SEQ ID NOs: 11, 12, 13, 14, 15, 16, 17, 18, 19, 20, 21, 22, 23 , 24, 25, 26, 27, 28, 29, 30, 31, 32, 33, 38, 39 and 106, a sequence which has at least 90% sequence identity thereto; or a homologous sequence of a hepadnavirus.

6. An expression cassette according to claim 5, wherein the hepatitis target sequence is any one of SEQ ID NOs: 11, 12, 13, 14, 106, 109, 110, 111, 112 or 171, or a sequence which has at least 90% sequence identity thereto.

7. An expression cassette according to any one of claims 1 to 6, wherein the artificial pri-miR sequence is any one of SEQ ID NOs: 135, 136, 137, 138, 139, 140, 165 or 166, or a sequence which has at least 90% sequence identity thereto, and the DNA sequence encoding the artificial pri-miR sequence is any one of SEQ ID NOs: 3, 4, 7, 8, 9, 10, 36 or 37.

8. An expression cassette according to any one of claims 1 to 7, which includes a transcription regulatory sequence.

9. An expression cassette according to any one of claims 1 to 8, which is a dimeric or multimeric expression cassette including in any possible combination two or more artificial pri-miR sequences as described in part (i) of claim 1.

10. An expression cassette according to any one of claims 1 to 9, which when expressed in vitro or in vivo is capable of inhibiting or silencing hepatitis virus gene expression.

11. A vector including an expression cassette according to any one of claims 1 to 10.

12. A host cell including an expression cassette or vector as claimed in any one of claims 1 to 11.

13. A composition for treating hepatitis virus infection, the composition including an expression cassette as claimed in any one of claims 1 to 10 or a vector according to claim 11 and a pharmaceutically acceptable adjuvant and/or carrier.

14. The use of an expression cassette as claimed in any one of claims 1 to 10 or a vector as claimed in claim 11 in a method of making a medicament for use in a method of treating hepatitis virus infection.

## Patentansprüche

1. Expressionskassette von Anti-Hepatitisvirus-primary-microRNA (pri-miR), die Folgendes einschließt:
(i) eine DNA-Sequenz, die eine künstliche pri-miR-31- oder pri-miR-122-Sequenz kodiert, die eine natürlich vorkommende pri-miR-31- oder pri-miR-122-Sequenz nachahmt, worin sich die künstliche pri-miR-31- oder pri-miR-122-Sequenz von der natürlich vorkommenden pri-miR-31- oder pri-miR-122-Sequenz darin unterscheidet, dass die Führungssequenz der natürlich vorkommenden pri-miR-31 oder pri-miR-122 durch eine Führungssequenz ersetzt wurde, die auf den Hepatitisvirus abzielt, und worin die Sequenz der künstlichen pri-miR-31 oder pri-miR-122 derart entwickelt wurde, dass die Sekundärstruktur der künstlichen pri-miR-31 oder pri-miR-122 die Sekundärstruktur der natürlich vorkommenden pri-miR-31 oder pri-miR-122 nachahmt; und
(ii) einen Pol II-Promotor.

2. Expressionskassette nach Anspruch 1, worin der Hepatitisvirus Hepatitis B- oder Hepatitis C-Virus ist.

3. Expressionskassette nach einem der Ansprüche 1 oder 2, worin der Pol 11-Promotor ein konstitutiver Promotor ist, bevorzugt ein Cytomegalovirus (CMV)-Promotor.

4. Expressionskassette nach einem der Ansprüche 1 oder 2, worin der Pol 11-Promotor ein gewebespezifischer Promotor ist, bevorzugt ein leberspezifischer Promotor, bevorzugter ein Alpha-1-Antitrypsin (A1AT)-Promotor, Faktor VIII (FVIII)-Promotor, HBV-Basic Core-Promotor (BCP) oder PreS2-Promotor.

5. Expressionskassette nach einem der Ansprüche 1 bis 4, worin die Hepatitis-Zielsequenz Folgendes ist: aus einer der SEQ.-ID-Nr. 11, 12, 13, 14, 15, 16, 17, 18, 19, 20, 21, 22, 23, 24, 25, 26, 27, 28, 29, 30, 31, 32, 33, 38, 39 und 106 ausgewählt ist, eine Sequenz, die mindestens 90 % Sequenzidentität dazu aufweist; oder eine homologe Sequenz eines Hepadnavirus.

6. Expressionskassette nach Anspruch 5, worin die Hepatitis-Zielsequenz Folgendes ist: eine der SEQ.-ID-Nr. 11, 12, 13, 14, 106, 109, 110, 111, 112 oder 171 oder eine Sequenz, die mindestens 90 % Sequenzidentität dazu aufweist.

7. Expressionskassette nach einem der Ansprüche 1 bis 6, worin die künstliche pri-miR-Sequenz Folgendes ist: eine der SEQ.-ID-Nr. 135, 136, 137, 138, 139, 140, 165 oder 166 oder eine Sequenz, die mindestens 90 % Sequenzidentität dazu aufweist, und die DNA-Sequenz, die die künstliche pri-miR-Sequenz kodiert, eine der SEQ.-ID-Nr. 3, 4, 7, 8, 9, 10, 36 oder 37 ist.

8. Expressionskassette nach einem der Ansprüche 1 bis 7, die eine Transkriptionsregulatorische Sequenz einschließt.

9. Expressionskassette nach einem der Ansprüche 1 bis 8, die eine dimere oder multimere Expressionskassette ist, die in jedweder möglichen Kombination zwei oder mehr künstliche pri-miR-Sequenzen, wie im Teil (i) von Anspruch 1 beschrieben, einschließt.

10. Expressionskassette nach einem der Ansprüche 1 bis 9, die bei Expression in vitro oder in vivo die Hepatitisvirus-Genexpression inhibieren oder abschalten kann.

11. Vektor, der eine Expressionskassette nach einem der Ansprüche 1 bis 10 einschließt.

12. Wirtszelle, die eine Expressionskassette oder einen Vektor nach einem der Ansprüche 1 bis 11 einschließt.

13. Zusammensetzung für die Behandlung einer Infektion mit Hepatitisvirus, wobei die Zusammensetzung eine Expressionskassette nach einem der Ansprüche 1 bis 10 oder einen Vektor nach Anspruch 11 und ein pharmazeutisch verträgliches Adjuvans und/oder einen pharmazeutisch verträglichen Träger einschließt.

14. Verwendung einer Expressionskassette nach eineim der Ansprüche 1 bis 10 oder eines Vektors nach Anspruch 11 in einem Verfahren zur Herstellung eines Medikaments für die Verwendung in einem Verfahren zur Behandlung einer Infektion mit Hepatitisvirus.

## Revendications

1. Cassette d'expression d'un microARN primaire (pri-miR) anti-virus de l'hépatite qui comprend :
(i) une séquence d'ADN codant pour une séquence d'un pri-miR-31 ou
d'un pri-miR-122 artificiel qui simule une séquence du pri-miR-31 ou du pri-miR-122 naturel, la séquence du pri-miR-31 ou du pri-miR-122 artificiel différant de la séquence du pri-miR-31 ou du pri-miR-122 naturel en ce que la séquence guide du pri-miR-31 ou du pri-miR-122 naturel a été remplacée par une séquence guide qui cible le virus de l'hépatite et la séquence du pri-miR-31 ou du pri-miR-122 artificiel étant conçue d'une manière telle que la structure secondaire du pri-miR-31 ou du pri-miR-122 artificiel simule la structure secondaire du pri-miR-31 ou du pri-miR-122 naturel ; et
(ii) un promoteur Pol II.

2. Cassette d'expression selon la revendication 1, dans laquelle le virus de l'hépatite est le virus de l'hépatite B ou de l'hépatite C.

3. Cassette d'expression selon l'une ou l'autre des revendications 1 ou 2, dans laquelle le promoteur Pol II est un promoteur constitutif, préférablement un promoteur du cytomégalovirus (CMV).

4. Cassette d'expression selon l'une ou l'autre des revendications 1 ou 2, dans laquelle le promoteur Pol II est un promoteur spécifique d'un tissu, préférablement un promoteur spécifique de foie et plus préférablement un promoteur de l'alpha-1-antitrypsine (A1AT), un promoteur du Facteur VIII (FVIII), un promoteur basal du core (PBC) de l'HBV ou un promoteur de la protéine PreS2.

5. Cassette d'expression selon l'une quelconque des revendications 1 à 4, dans laquelle la séquence cible du virus de l'hépatite est sélectionnée parmi les suivantes : SÉQ ID N° : 11, 12, 13, 14, 15, 16, 17, 18, 19, 20, 21, 22, 23, 24, 25, 26, 27, 28, 29, 30, 31, 32, 33, 38, 39 et 106, une séquence qui est au moins 90 % identique à celles-ci ou une séquence homologue d'un virus à ADN de l'hépatite.

6. Cassette d'expression selon la revendication 5, dans laquelle la séquence cible du virus de l'hépatite est sélectionnée parmi les suivantes : SÉQ ID N° : 11, 12, 13, 14, 106, 109, 110, 111, 112 ou 171 ou une séquence qui est au moins 90 % identique à celles-ci.

7. Cassette d'expression selon l'une quelconque des revendications 1 à 6, dans laquelle la séquence du pri-miR artificiel est l'une quelconque des suivantes : SÉQ ID N° : 135, 136, 137, 138, 139, 140, 165 ou 166 ou une séquence qui est au moins 90 % identique à celles-ci, et la séquence d'ADN codant pour la séquence du pri-miR artificiel est l'une quelconque des suivantes : SÉQ ID N° : 3, 4, 7, 8, 9, 10, 36 ou 37.

8. Cassette d'expression selon l'une quelconque des revendications 1 à 7, qui inclut une séquence régulatrice de la transcription.

9. Cassette d'expression selon l'une quelconque des revendications 1 à 8, qui est une cassette d'expression dimérique ou multimérique incluant, en toute combinaison possible, deux ou plusieurs des séquences de pri-miR artificiels décrites à la partie (i) de la revendication 1.

10. Cassette d'expression selon l'une quelconque des revendications 1 à 9 qui, quand exprimée *in vitro* ou *in vivo,* est capable d'inhiber ou de rendre silencieuse l'expression du gène du virus de l'hépatite.

11. Vecteur incluant une cassette d'expression selon l'une quelconque des revendications 1 à 10.

12. Cellule hôte incluant une cassette d'expression ou un vecteur selon l'une quelconque des revendications 1 à 11.

13. Composition pour le traitement d'une infection par un virus de l'hépatite, la composition incluant une cassette d'expression selon l'une quelconque des revendications 1 à 10 ou un vecteur selon la revendication 11 et un adjuvant et/ou véhicule pharmaceutiquement acceptable.

14. Utilisation d'une cassette d'expression selon l'une quelconque des revendications 1 à 10 ou d'un vecteur selon la revendication 11 dans un processus de fabrication d'un médicament pour une utilisation dans une méthode de traitement d'une infection par un virus de l'hépatite.
